# EUROPEAN PATENT APPLICATION

(11) **EP 4 772 503 A1**
(43) Date of publication of application: **08.07.2026**
(21) Application number: 24879064.4
(22) Date of filing: 17.10.2024
(51) Int. Cl.: C07D 339/04, C07F 5/02, A61P 35/00

(54) **LIPOIC ACID DERIVATIVE, VESICLE COMPOUND AND PREPARATION THEREFOR AND USE THEREOF**

(30) Priority: 20.10.2023 CN 202311370599; 18.01.2024 CN 202410079009; 24.07.2024 CN 202411004416; 12.10.2024 CN 202411423537
(71) Applicant: Neuboron Medtech Research Institute Ltd., Nanjing, Jiangsu 211112 (CN)
(72) Inventor: QIAN, Zhi-yong, Nanjing, Jiangsu 211112 (CN); DAI, Li-qun, Nanjing, Jiangsu 211112 (CN); LIU, Yuan-hao, Nanjing, Jiangsu 211112 (CN)
(74) Representative: Gunzelmann, Rainer
(86) International application number: PCT/CN2024/125417
(87) International publication number: WO 2025/082423

(57) **Abstract**

The present invention relates to a lipoic acid derivative, a vesicle compound, the preparation therefor and the use thereof, particularly to a lipoic acid derivative, or a stereoisomer or pharmaceutically acceptable salt thereof, the structure of the lipoic acid derivative being represented by formula (1). One end of the lipoic acid derivative is a group containing phenylboronic acid, and the other end of the lipoic acid derivative is lipoic acid, with a chemical bond or a linking chain linked therebetween. A drug provided by the present invention is liable to enrich and retain in tumour cells, and the boron drug of the present invention can be conveniently used for boron neutron capture therapy and can realize an excellent anti-tumour effect.

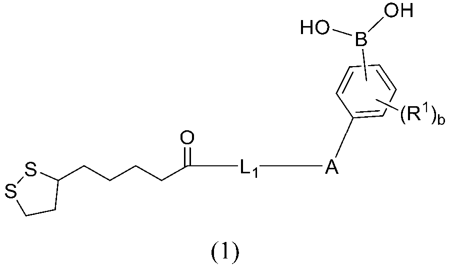

## Description

### TECHNICAL FIELD

The present invention relates to the field of medicine, and specifically, the present invention relates to a lipoic acid derivative, a vesicle compound, and preparation therefor and use thereof.

### BACKGROUND

Boron neutron capture therapy (BNCT), as an emerging and rapidly developing precision tumour radiotherapy technology in the field of tumour treatment, combines biological targeting and heavy ion effect, enabling cell-level precision radiotherapy, and exhibits outstanding clinical advantages for the treatment of recurrent, invasive, and locally metastatic tumours. The principle of BNCT involves first injecting a targeted drug carrying B-10 (a stable, non-radioactive natural isotope) into the patient's body. Utilizing the targeting selectivity of the drug, B-10 is specifically enriched within the tumour tissue. Subsequently, a low-energy epithermal neutron beam is used to directionally irradiate the tumour tissue. After irradiation, B-10 in the tumour tissue is activated, undergoes a boron neutron capture reaction, and releases two heavy ions, alpha particles and ⁷Li, with a range of only about ten micrometres (approximately the size of a cancer cell) and high linear energy transfer, damaging the DNA double strands of cancer cells and achieving eradication of cancer cells. The short-range cytotoxicity of BNCT can induce irreversible damage in cancer cells while preserving the normal cells surrounding the tumour, thereby achieving the objective of site-specific killing of cancer cells at the cellular level without harming normal tissue.

The drug formulations of BNCT widely used in clinical practice are primarily fructose and sorbitol complex formulations of 4-borono-L-phenylalanine (BPA). However, due to certain limiting factors, such as the reverse transport mechanism of amino acids, the retention of BPA in tumours is highly restricted, which is regulated by the amino acid concentration gradient across the cell membrane; In addition, the rapid in vivo metabolism of small molecule compounds is another important factor leading to the short retention time of BPA at the tumour site. Therefore, to maintain a high concentration of B-10 at the tumour site, patients need to receive continuous intravenous infusion during neutron irradiation, and the BPA drug containing B-10 is expensive, which will undoubtedly increase the treatment cost and impose an additional economic burden on patients.

To achieve better tumour treatment efficacy with BNCT, it is necessary not only to effectively deliver a high dose of B-10 to cancer cells but also to ensure its prolonged intracellular retention within cancer cells.

### SUMMARY OF THE INVENTION

To address the aforementioned issues, the present invention has developed a novel phenylboronic acid-based lipoic acid derivative and a vesicle thereof. One end of the lipoic acid derivative is a 4-borono-1-phenylalanine containing a phenylboronic acid group, and the other end of the lipoic acid derivative is lipoic acid, with a chemical bond or a linking group linked therebetween. The present invention can prolong the metabolism and circulation time of boron drugs in vivo, increase the B-10 content at tumour sites, and extend the retention time of drugs within cancer cells, thereby achieving excellent anti-tumour effects in BNCT.

One object of the present invention is to provide a lipoic acid derivative.

Another object of the present invention is to provide a vesicle compound formed by self-assembly of the lipoic acid derivative.

Yet another object of the present invention is to provide a pharmaceutical composition comprising the lipoic acid derivative.

Yet another object of the present invention is to provide a pharmaceutical composition comprising the vesicle compound.

Still another object of the present invention is to provide use of the above-mentioned lipoic acid derivative, vesicle compound and pharmaceutical composition thereof.

Still another object of the present invention is to provide a preparation method of the lipoic acid derivative.

To achieve the above objects, in one aspect, the present invention provides a lipoic acid derivative or a stereoisomer or pharmaceutically acceptable salt thereof, wherein the lipoic acid derivative is represented by formula (1): 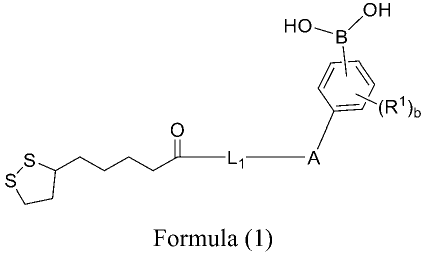
L₁ is selected from a bond, 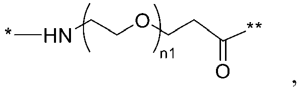 or 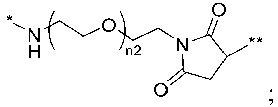
A is selected from 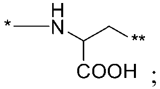
each R¹ is independently selected from H, halogen, a hydrocarbon group having 1 to 4 carbon atoms, or a boronic acid group (-B(OH)₂);
n1 is selected from 0, 1, 2, 3, 4, 5, 6, 7, 8, 9, 10, 11, or 12;
n2 is selected from 0, 1, 2, 3, 4, 5, 6, 7, 8, 9, 10, 11, or 12;
b is selected from 0, 1, 2, 3, or 4;
* and ** are linking sites.

According to some specific embodiments of the present invention, the halogen is selected from F, Cl, Br or I. The hydrocarbon group having 1 to 4 carbon atoms may be selected from one of methyl, ethyl, propyl, or butyl.

According to some specific embodiments of the present invention, each R¹ is independently selected from H, F, Cl, Br, I or boronic acid group (-B(OH)₂).

According to some specific embodiments of the present invention, B (boron) in the lipoic acid derivative is independently selected from natural boron or ¹⁰B. It is known that natural boron contains approximately 19.8% ¹⁰B and 80.2% ¹¹B.

According to some specific embodiments of the present invention, wherein in the structure of the lipoic acid derivative, at least one B (boron) is ¹⁰B.

According to some specific embodiments of the present invention, in the - B(OH)₂ of the lipoic acid derivative, the B (boron) is ¹⁰B.

According to some specific embodiments of the present invention, the lipoic acid derivative has a ¹⁰B abundance of greater than or equal to 90%.

According to some specific embodiments of the present invention, in the lipoic acid derivative, A 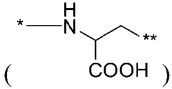 is linked to the phenylboronic acid structure at the ** end and to L₁ at the * end.

According to some specific embodiments of the present invention, wherein the structure of the lipoic acid derivative is represented by formula (1a):

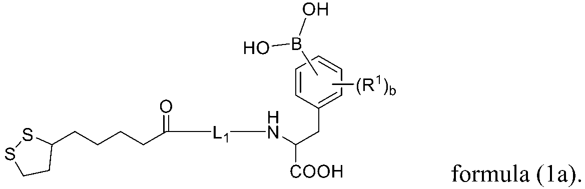

Referring to the lipoic acid derivative of formula (1a), it is conceivable that the lipoic acid derivative can be formed from binding of lipoic acid to the BPA-like compound through L₁.

According to some specific embodiments of the present invention, in the structure represented by formula (1a), B in -B(OH)₂ is ¹⁰B.

According to some specific embodiments of the present invention, in the structure represented by formula (1a), B in at least one -B(OH)₂ is ¹⁰B.

According to some specific embodiments of the present invention, in the structure represented by formula (1a), b is 0.

According to some specific embodiments of the present invention, in the structure represented by formula (1a), b is 1, and R¹ is -B(OH)₂.

According to some specific embodiments of the present invention, in the structure represented by formula (1a), L₁ is

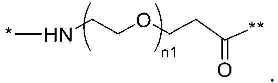

According to some specific embodiments of the present invention, in the structure represented by formula (1a), L₁ is a bond, that is, L₁ is a chemical bond.

According to some specific embodiments of the present invention, L₁ is linked to the lipoic acid structure at the * end. * and ** are linking sites.

According to some specific embodiments of the present invention, the structure of the lipoic acid derivative is selected from one of the following structures:

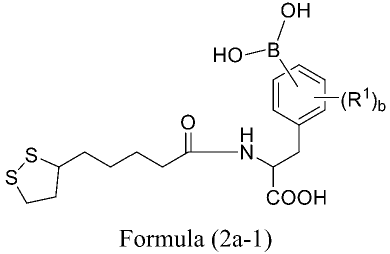

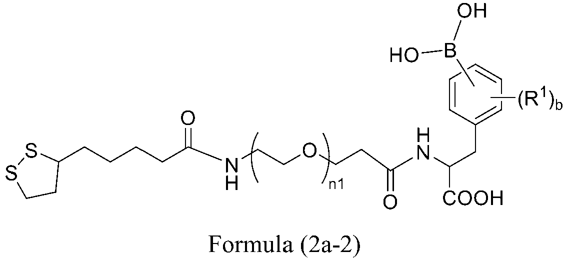

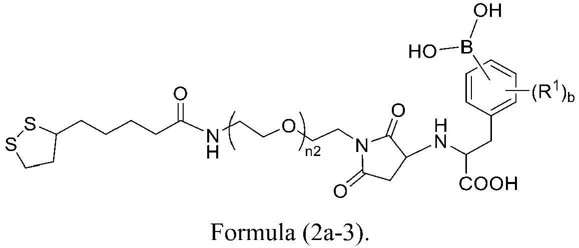

According to some specific embodiments of the present invention, in the structure represented by formula (2a-1), (2a-2) or (2a-3), the B in at least one - B(OH)₂ is ¹⁰B.

According to some specific embodiments of the present invention, in the structure represented by formula (2a-1), (2a-2) or (2a-3), b is 0.

According to some specific embodiments of the present invention, in the structure represented by formula (2a-1), (2a-2) or (2a-3), b is 1, and R¹ is -B(OH)₂.

According to some specific embodiments of the present invention, wherein the lipoic acid derivative is selected from one of the following structures:

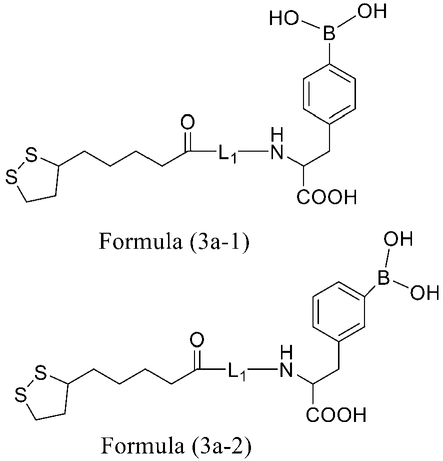

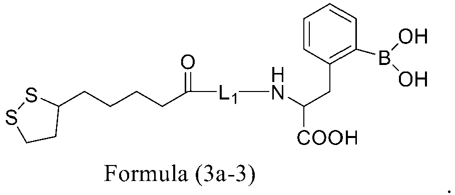

According to some specific embodiments of the present invention, in the structure represented by formula (3a-1), formula (3a-2) or formula (3a-3), the B in - B(OH)₂ is ¹⁰B.

According to some specific embodiments of the present invention, in the structure represented by formula (3a-1), formula (3a-2) or formula (3a-3), L₁ is

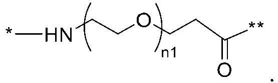

According to some specific embodiments of the present invention, in the structure represented by formula (3a-1), formula (3a-2), or formula (3a-3), L₁ is a bond.

In some embodiments of the present invention, lipoic acid can be linked to p-boronophenylalanine (BPA) through L₁ to form a new boron carrier/boron drug. Similarly, lipoic acid can be linked with m-boronophenylalanine (denoted as 3-BPA) or o-boronophenylalanine (denoted as 2-BPA) to form a new boron carrier/boron drug, namely BPA lipoic acid derivative. In addition, lipoic acid can also combine with diboronophenylalanine to form a new boron carrier/boron drug. For example, any two positions from the 2-position to the 6-position of the benzene ring are boronic acid groups (-B(OH)₂).

According to some specific embodiments of the present invention, the lipoic acid derivative is selected from one of the following structures:

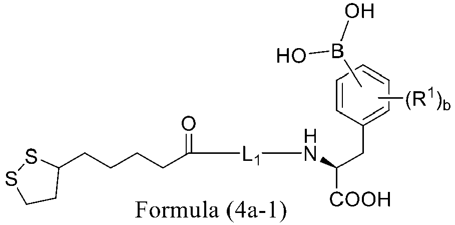

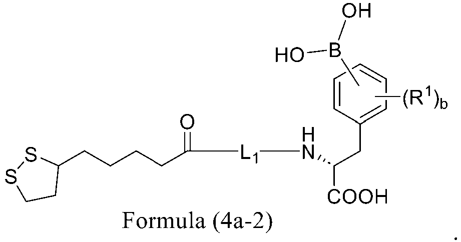

According to some specific embodiments of the present invention, each R¹ is independently selected from H, halogen, a hydrocarbon group having 1 to 4 carbon atoms, or a boronic acid group. In some embodiments, R¹ may be H.

According to some specific embodiments of the present invention, in the structure represented by formula (4a-1) or formula (4a-2), the B in -B(OH)₂ is ¹⁰B. In the structure represented by formula (4a-1) or formula (4a-2), B in at least one - B(OH)₂ is ¹⁰B.

In some embodiments, in the structure represented by formula (4a-1) or formula (4a-2), b is 0.

In some embodiments, in the structure represented by formula (4a-1) or formula (4a-2), b is 1, and R1 is -B(OH)₂.

According to some specific embodiments of the present invention, in the structure represented by formula (4a-1) or formula (4a-2), L₁ is

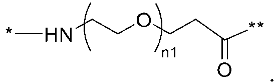

According to some specific embodiments of the present invention, in the structure represented by formula (4a-1) or formula (4a-2), L₁ is a bond.

According to some specific embodiments of the present invention, wherein the lipoic acid derivative is selected from one of the following structures:

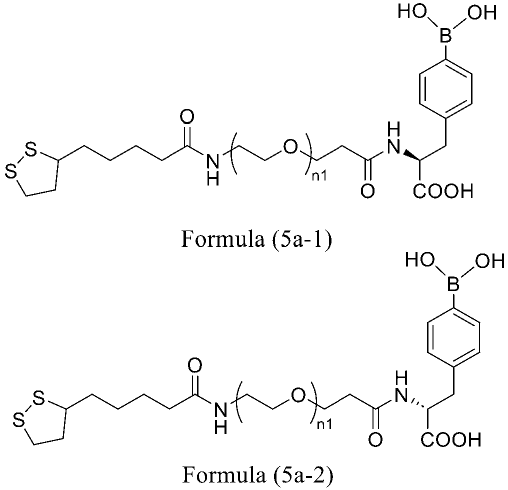

According to some specific embodiments of the present invention, in the structure represented by formula (5a-1) or formula (5a-2), the B in -B(OH)₂ is ¹⁰B.

According to some specific embodiments of the present invention, n1 is independently selected from 0, 1, 2, 3, 4, 5, 6, 7, 8, 9, 10, 11, or 12, respectively. Further, n1 is independently selected from 0, 1, 2, 3, 4, 5, 6, 7, or 8, respectively.

According to some specific embodiments of the present invention, wherein the BPA structure in the lipoic acid derivative may be in L-configuration (L-BPA) or D-configuration (D-BPA).

According to some specific embodiments of the present invention, wherein the B element in the lipoic acid derivative is ¹⁰B.

According to some specific embodiments of the present invention, wherein the lipoic acid derivative is selected from, but not limited to, one of the following structures:

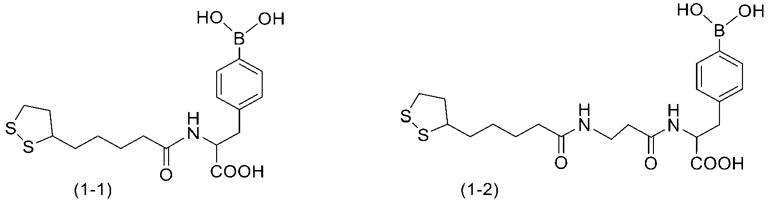

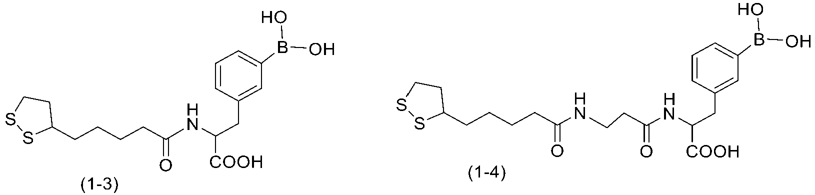

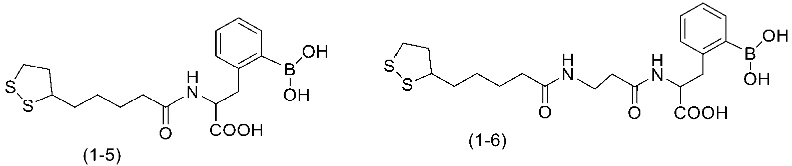

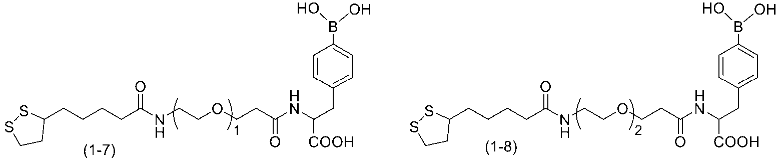

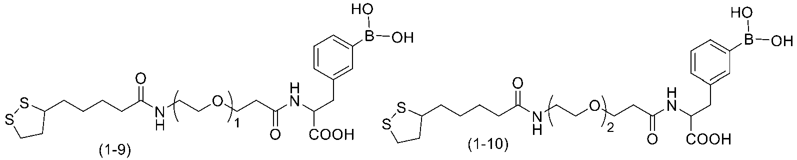

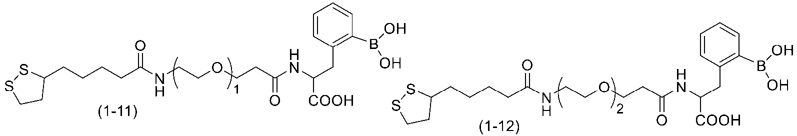

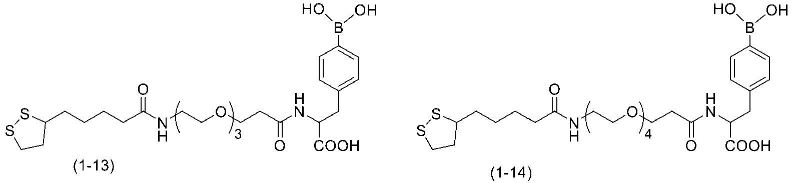

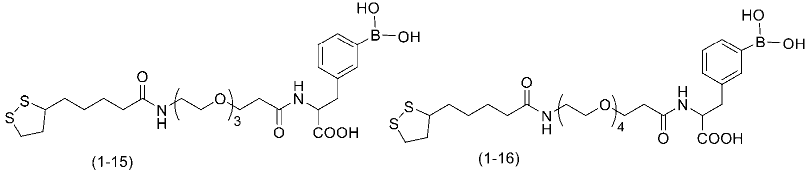

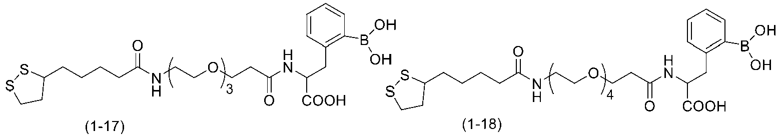

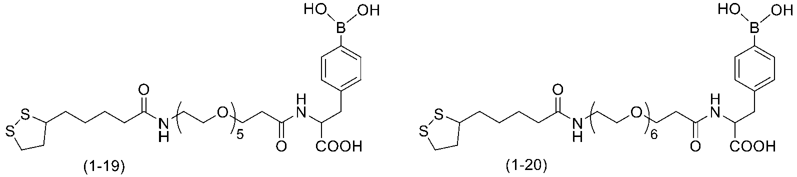

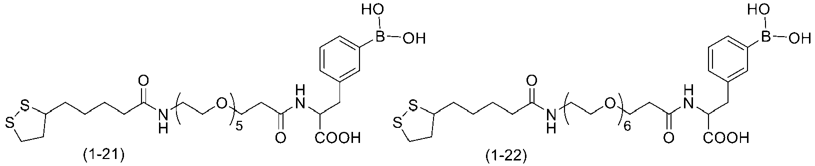

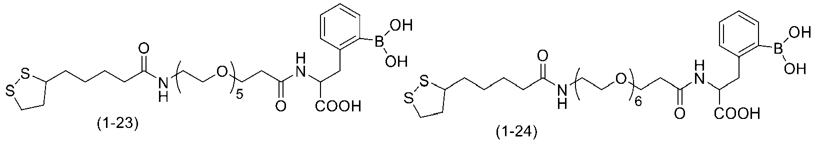

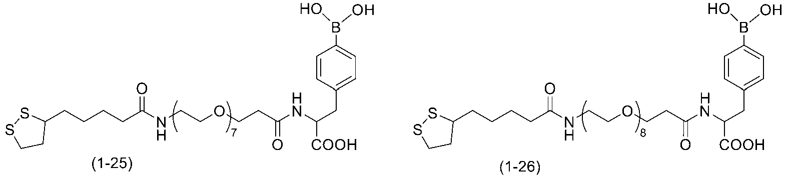

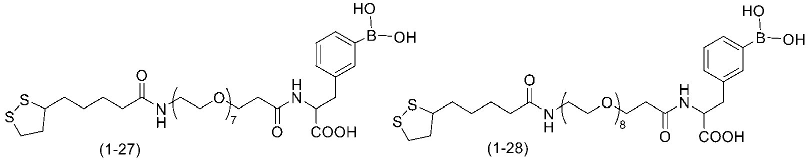

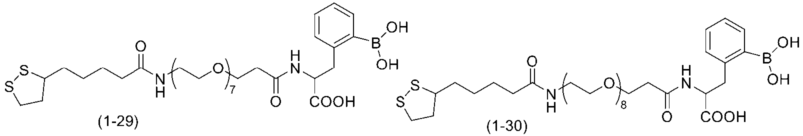

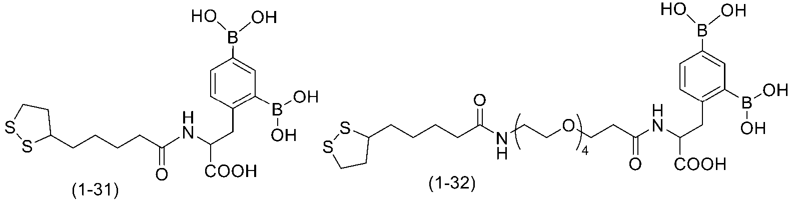

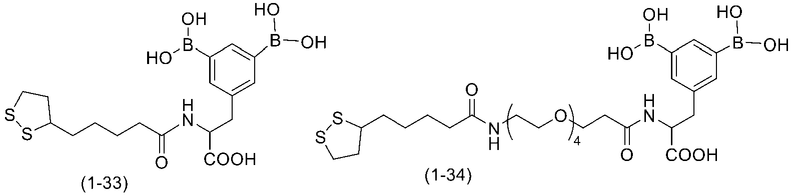

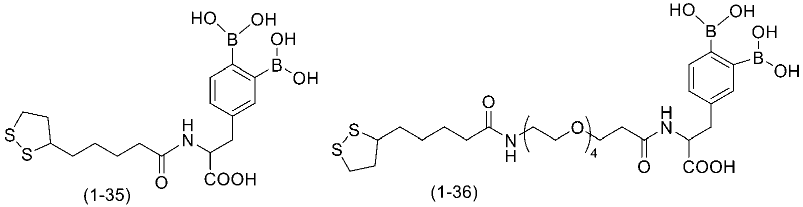

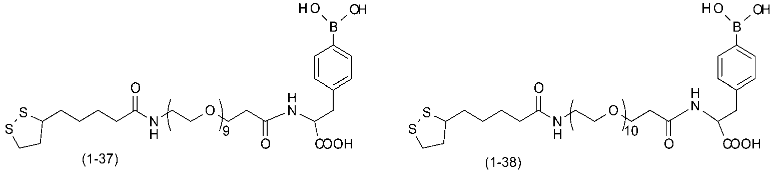

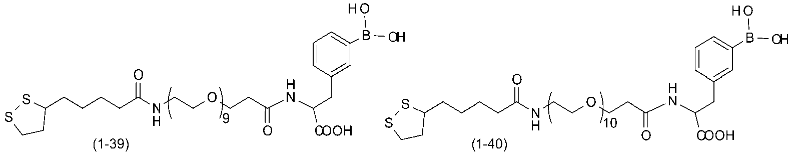

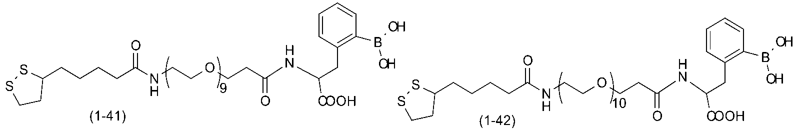

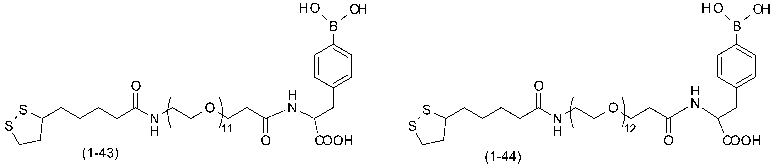

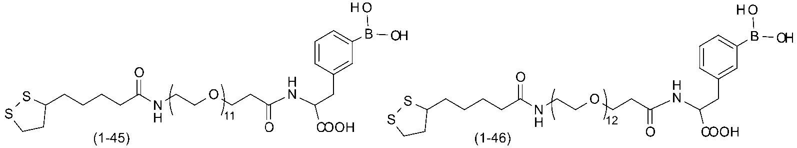

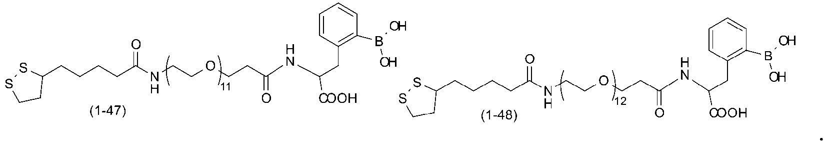

The linking group in the lipoic acid derivative of the present invention not only serves to link 4-borono-L-phenylalanine (or other boronated phenylalanines mentioned above) with lipoic acid, but its hydrophilic property can also regulate the hydrophilicity and hydrophobicity of the small molecules of the lipoic acid derivative. In the lipoic acid derivative of the present invention, this amphiphilic small molecule compound (with phenylboronic acid as the hydrophilic end and the disulfide containing five-membered cycle of lipoic acid as the hydrophobic end) forms a vesicle compound through self-assembly, opening of the five-membered heterocycle, and cross-linking of disulfide bonds. The vesicle compound of the present invention may also be referred to as a vesicle nanoparticle or a vesicle polymer.

In another aspect, the present invention further provides a vesicle compound. The vesicle compound can be formed by a plurality of the above-mentioned lipoic acid derivatives. The ring-opening crosslinking polymerization of disulfide bonds between the lipoic acid derivatives can form the vesicle compound. The vesicle compound may also be referred to as a vesicle nanoparticle or vesicle polymer. It is conceivable that the formation of the vesicle compound requires a plurality of lipoic acid derivatives; among the plurality of lipoic acid derivatives, the lipoic acid derivatives may have the same structure or different structures.

The phenylboronic acid on the surface of the vesicle nanoparticle can confer the biological function of targeting the nano-drug to sialic acid, significantly enhancing the targeted accumulation of the nano-drug at the tumour site. Meanwhile, the present invention can avoid the presence of the reverse transport mechanism of amino acids as seen with BPA, thereby prolonging the retention time of B-10 in cancer cells, which is beneficial for the implementation of BNCT. The present invention may even eliminate the need for administration via intravenous infusion and can achieve the concentration of B-10 within cancer cells via intravenous injection.

According to some specific embodiments of the present invention, wherein the vesicle compound is represented by formula (a): 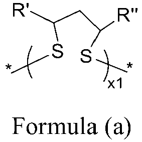
R' and R" are each independently selected from H or 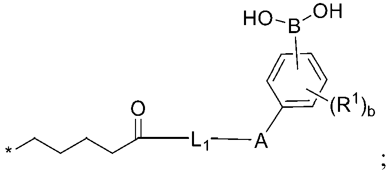 and, in each 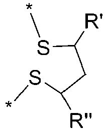 structure, one of R' and R" is 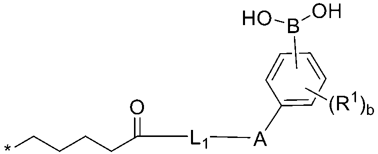 and the other is H;
each L₁ is independently selected from a bond, 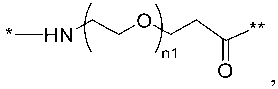 or 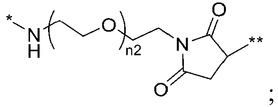
A is selected from 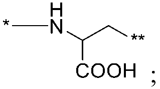
each R¹ is independently selected from H, halogen, a hydrocarbon group having 1 to 4 carbon atoms, or a boronic acid group;
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
x1 is selected from an integer from 1 to 500,000;
* and ** are linking sites.

According to some specific embodiments of the present invention, wherein each n1 is independently selected from 0, 1, 2, 3, 4, 5, 6, 7, or 8.

According to some specific embodiments of the present invention, wherein each n1 is independently selected from 1, 2, 4, or 8.

According to some specific embodiments of the present invention, wherein each n1 is independently 4.

According to some specific embodiments of the present invention, wherein each b is independently selected from 0, or 1.

According to some specific embodiments of the present invention, wherein each b is independently 0.

According to some specific embodiments of the present invention, wherein x1 is not less than 2; or x1 is not less than 3; or x1 is not less than 5; or x1 is not less than 10; or x1 is not less than 20; or x1 is not less than 30; or x1 is not less than 40; or x1 is not less than 50.

According to some specific embodiments of the present invention, x1 is selected from an integer from 1 to 10,003.

According to some specific embodiments of the present invention, wherein x1 is selected from an integer from 1 to 10,003; or x1 is selected from an integer from 10 to 500,000; or x1 is selected from an integer from 10 to 400,000; or x1 is selected from an integer from 10 to 300,000; or x1 is selected from an integer from 10 to 200,000; or x1 is selected from an integer from 10 to 100,000; or x1 is selected from 10,000 to 50,000; or x1 is selected from 10,000 to 100,000; or x1 is selected from 10,000 to 150,000; or x1 is selected from 10,000 to 200,000.

According to some specific embodiments of the present invention, wherein x1 may be 1, 10, 20, 30, 40, 50, 100, 200, 300, 400, 500, 600, 700, 800, 900, 1,000, 1,200, 1,500, 2,000, 2,500, 3,000, 3,500, 4,000, 5,000, 6,000, 7,000, 8,000, 9,000, 10,000, 10,003, 20,000, 25,000, 30,000, 35,000, 40,000, 45,000, 50,000, 55,000, 60,000, 65,000, 70,000, 75,000, 80,000, 85,000, 90,000, 95,000, 100,000, 150,000, 200,000, 250,000, 300,000, 350,000, 400,000, 450,000, or 500,000.

According to some specific embodiments of the present invention, the vesicle compound has a structure represented by formula (b): 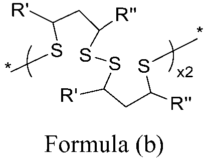
wherein R' and R" are each independently selected from H or 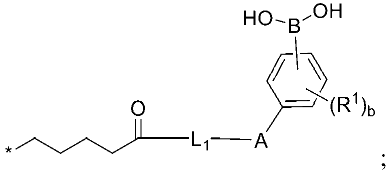 and, in each 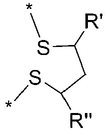 structure, one of R' and R" is 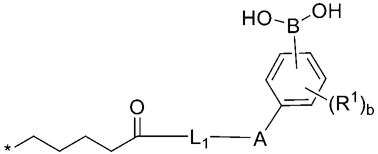 and the other is H;
the definitions of L₁, A, R¹, n1, n2, and b refer to the foregoing description; * is a linking site.

According to some specific embodiments of the present invention, wherein in formula (b), x2 is selected from an integer from 1 to 250,000; or x2 is selected from an integer from 1 to 50,000; or x2 is selected from 10 to 50,000; or x2 is selected from 10 to 100,000; or x2 is selected from an integer from 1 to 5,000.

According to some specific embodiments of the present invention, wherein the vesicle compound has a structure as shown below:

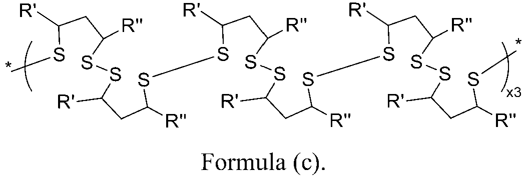

Each 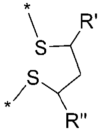 refers to the above description; * is a linking site.

According to some specific embodiments of the present invention, wherein in formula (c), x3 is selected from an integer from 1 to 1,600; or x3 is selected from an integer from 1 to 15,000; x3 is selected from an integer from 1 to 80,000; or x3 is selected from 10 to 20,000; or x3 is selected from 10 to 10,000; or x3 is selected from 1000 to 20,000.

According to some specific embodiments of the present invention, wherein the vesicle compound is represented by formula (2): 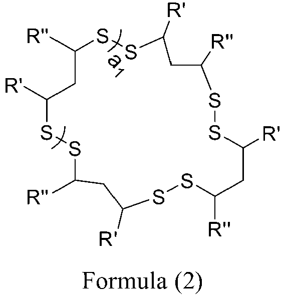
R' and R" are each independently selected from H or 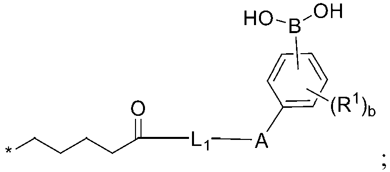 and, in each 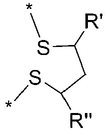 structure, one of R' and R" is 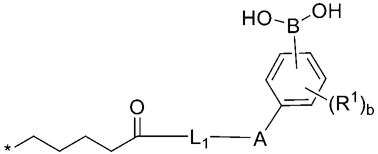 and the other is H;
each L₁ is independently selected from a bond, 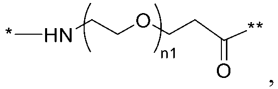 or 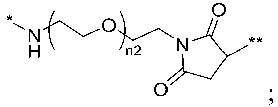
A is selected from 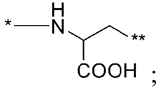
each R¹ is independently selected from H, halogen, a hydrocarbon group having 1 to 4 carbon atoms, or a boronic acid group (-B(OH)₂);
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
a1 is selected from an integer from 0 to 500,000;
* and ** are linking sites.

According to some specific embodiments of the present invention, wherein a1 is selected from an integer from 0 to 500,000; or a1 is selected from an integer from 0 to 400,000; or a1 is selected from an integer from 0 to 200,000; or a1 is selected from an integer from 0 to 100,000; or a1 is selected from an integer from 10 to 500,000; or a1 is selected from an integer from 1,000 to 500,000; or a1 is selected from an integer from 10,000 to 500,000; or a1 is selected from an integer from 10,000 to 200,000; or a1 is selected from an integer from 10,000 to 150,000; or a1 is selected from an integer from 0 to 10,000.

According to some specific embodiments of the present invention, wherein a1 may be 0, 1, 10, 20, 30, 40, 50, 100, 200, 300, 400, 500, 600, 700, 800, 900, 1,000, 1200, 1,500, 2,000, 2,500, 3,000, 3,500, 4,000, 5,000, 6,000, 7,000, 8,000, 9,000, 10,000, 20,000, 25,000, 30,000, 35,000, 40,000, 45,000, 50,000, 55,000, 60,000, 65,000, 70,000, 75,000, 80,000, 85,000, 90,000, 95,000, 100,000, 150,000, 200,000, 250,000, 300,000, 350,000, 400,000, 450,000 or 500,000.

According to some specific embodiments of the present invention, wherein the vesicle compound is represented by formula (2): 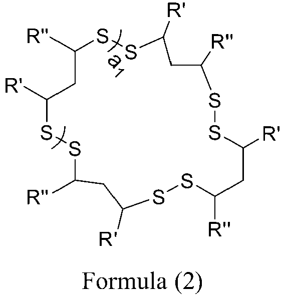
R' and R" are each independently selected from H or 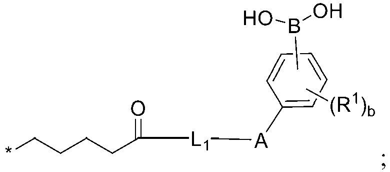 and, in each 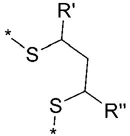 structure, one of R' and R" is 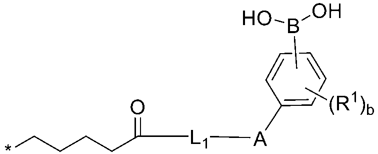 and the other is H;
each L₁ is independently selected from a bond, 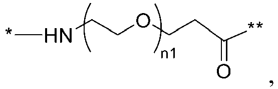 or 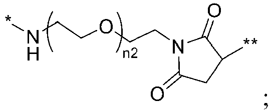
A is selected from 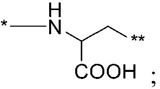
each R¹ is independently selected from H, halogen, a hydrocarbon group having 1 to 4 carbon atoms, or a boronic acid group (-B(OH)₂);
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
a1 is selected from an integer from 0 to 10,000;
* and ** are linking sites.

According to some specific embodiments of the present invention, wherein each L₁ is independently selected from a bond, 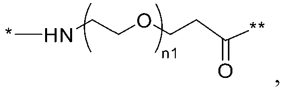 or 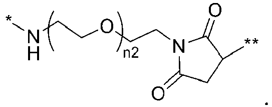 In some embodiments, L₁ is independently selected from a bond or 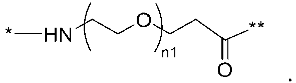 In some embodiments, L₁ is

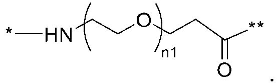

According to some specific embodiments of the present invention, wherein each R¹ is independently selected from H, halogen, a hydrocarbon group having 1 to 4 carbon atoms, or a boronic acid group; in some embodiments, R¹ is H.

In some embodiments, each R' or R" in the vesicle compound may be located on the surface of the vesicle. In particular, the phenylboronic acid end of R' or R" is located on the surface of the vesicle. Further, the surface of the vesicle includes the inner and outer surfaces of the vesicle. Each R' or R" in the vesicle compound may be located on the surface of the vesicle, indicating that R' or R" may extend outward from the vesicle or inward into the vesicle.

That is, the hydrophilic group of the lipoic acid derivative monomer is located on the surface of the vesicle, and the hydrophobic group is located inside the vesicle. The phenylboronic acid end of the lipoic acid derivative monomer is a hydrophilic group, and the five-membered heterocycle end of the lipoic acid derivative monomer is a hydrophobic group.

It is conceivable that the nanoparticle formed by crosslinking polymerization of lipoic acid derivatives may have a vesicle structure with a cavity. The phenylboronic acid end as a hydrophilic group is located on the surface of the vesicle, including the outer surface of the vesicle and the inner surface of the vesicle cavity, while the five-membered heterocycle end of lipoic acid as a hydrophobic group is located inside the vesicle.

According to some specific embodiments of the present invention, the structure of the vesicle compound may be represented by formula (3): 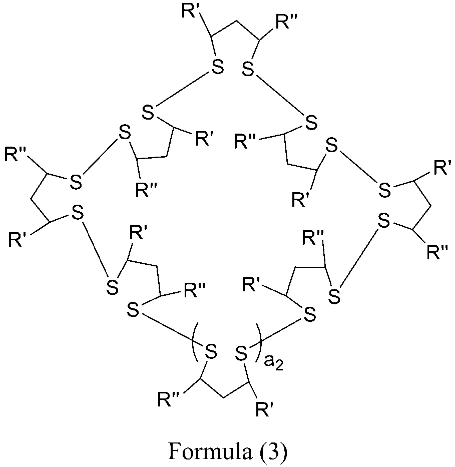 wherein the definition of each 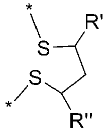 refers to the foregoing description. a2 may be selected from an integer from 0 to 10,000. a2 may be selected from an integer from 0 to 100,000. a2 may be selected from an integer from 0 to 500,000. a2 may be selected from an integer from 10 to 500,000. a2 may be selected from an integer from 0 to 100,000. a2 may be selected from an integer from 10 to 500,000. a2 may be selected from an integer from 1,000 to 500,000. a2 may be selected from an integer from 10,000 to 500,000.

As shown in the structure of formula (3), the portions R' and R" containing hydrophilic groups extend toward the outer surface of the vesicle, the portions R' and R" containing hydrophilic groups extend toward the inner surface of the vesicle, and the disulfide bond portion as a hydrophobic group is located inside the vesicle.

Both formula (2) and formula (3) are merely planar structures; in reality, the chemical bond in formula (2) can also rotate to obtain a vesicle structure similar to formula (3), where R' and R" are located on the surface of the vesicle.

According to some specific embodiments of the present invention, wherein each n1 is independently selected from 1, 2, 3, 4, 5, 6, 7, or 8.

According to some specific embodiments of the present invention, wherein each n2 is independently selected from 1, 2, 3, 4, 5, 6, 7, or 8.

According to some specific embodiments of the present invention, each R¹ is independently selected from H, halogen or boronic acid group.

According to some specific embodiments of the present invention, the definitions of L₁, A, R¹, n1, and b in the vesicle compound can all refer to the descriptions of L₁, A, R¹, n1, and b in the aforementioned lipoic acid derivative, which will not be reiterated here.

According to some specific embodiments of the present invention, wherein in the vesicle compound, each B is independently selected from natural boron or ¹⁰B. It is conceivable that the B (boron) of the present invention may be either natural boron or ¹⁰B.

According to some specific embodiments of the present invention, wherein at least one B in the vesicle compound is ¹⁰B.

According to some specific embodiments of the present invention, wherein in the vesicle compound, B has a ¹⁰B abundance of greater than or equal to 90%. For example, the ¹⁰B abundance in boron (B) in the vesicle compound may be 90%, 91%, 92%, 93%, 94%, 95%, 96%, 97%, 98%, 99%, or 100%.

According to some specific embodiments of the present invention, the vesicle compound has a ¹⁰B abundance of greater than or equal to 95%.

The vesicle compound of the present invention facilitates the delivery of boron-10 (¹⁰B) into tumour cells and enrichment within the tumour cells, thereby enabling its use in BNCT therapy.

In some embodiments, in the structure represented by formula (1), formula (1a), formula (2a-1), formula (2a-2), formula (2a-3), formula (3a-1), formula (3a-2), formula (3a-3), formula (4a-1), formula (4a-2), formula (5a-1), formula (5a-2), formula (a), formula (b), formula (2), or formula (3) of the present invention, L₁ is

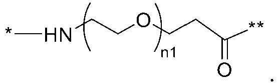

In some embodiments, in the structure represented by formula (1), formula (1a), formula (2a-1), formula (2a-2), formula (2a-3), formula (3a-1), formula (3a-2), formula (3a-3), formula (4a-1), formula (4a-2), formula (5a-1), formula (5a-2), formula (a), formula (b), formula (2), or formula (3) of the present invention, b is 0.

In some embodiments, in the structure represented by formula (1), formula (1a), formula (2a-1), formula (2a-2), formula (2a-3), formula (3a-1), formula (3a-2), formula (3a-3), formula (4a-1), formula (4a-2), formula (5a-1), formula (5a-2), formula (a), formula (b), formula (2), or formula (3) of the present invention, n1 is selected from 0, 1, 2, 3, 4, 5, 6, 7, 8, 9, 10, 11 or 12; preferably 0, 1, 2, 3, 4, 5, 6, 7 or 8; more preferably 1, 2, 4, or 8; further preferably 4.

In some embodiments, in the structure represented by formula (1), formula (1a), formula (2a-1), formula (2a-2), formula (2a-3), formula (3a-1), formula (3a-2), formula (3a-3), formula (4a-1), formula (4a-2), formula (5a-1), formula (5a-2), formula (a), formula (b), formula (2), or formula (3) of the present invention, the B in -B(OH)₂ is natural B or ¹⁰B; preferably ¹⁰B.

According to some specific embodiments of the present invention, wherein the grain size of the vesicle compound is 50-300 nm.

For example, the grain size of the vesicle compound may be 50 nm, 60 nm, 70 nm, 80 nm, 90 nm, 100 nm, 110 nm, 120 nm, 130 nm, 140 nm, 150 nm, 160 nm, 170 nm, 180 nm, 190 nm, 200 nm, 210 nm, 220 nm, 230 nm, 240 nm, 250 nm, 260 nm, 270 nm, 280 nm, 290 nm or 300 nm.

According to some specific embodiments of the present invention, the grain size of the vesicle compound is 50-250 nm. Further, the grain size of the vesicle compound may also be 50-200 nm. the grain size of the vesicle compound may also be 100-200 nm.

According to some specific embodiments of the present invention, wherein the grain size of the vesicle compound is 140 nm to 250 nm, 140 nm to 240 nm, or 140 nm to 150 nm.

Unless otherwise specified in the embodiments, those skilled in the art will understand that the value of grain size (hereinafter referred to as GS) includes GS value ±30%. In one embodiment, the value is GS value ±25%. In one embodiment, the value is GS value ±20%. In one embodiment, the value is GS value ±15%. In one embodiment, the value is GS value ±10%. In one embodiment, the value is GS value ±5%. For example, grain size 10 ± 20% means a range of 8-12.

In another aspect, the present invention further provides a pharmaceutical composition comprising the lipoic acid derivative and/or the vesicle compound as described in the present invention.

In yet another aspect, the present invention further provides use of the lipoic acid derivative, the vesicle compound or the pharmaceutical composition as described in the present invention in the preparation of an antitumour drug.

According to some specific embodiments of the present invention, the tumour is a tumour with high sialic acid expression.

According to some specific embodiments of the present invention, the tumour is selected from prostate cancer, ovarian cancer, liver cancer, glioma, pancreatic cancer, or breast cancer.

According to some specific embodiments of the present invention, the tumour may be melanoma.

According to some specific embodiments of the present invention, the tumour may be melanoma, glioblastoma, renal neuroblastoma, astrocytoma, brain tumour, head and neck cancer, sarcoma, rhabdomyosarcoma, mastocarcinoma, bladder cancer, colon cancer, ovarian cancer, colorectal cancer, prostate cancer, gastric cancer, pancreatic cancer, extrahepatic cholangiocarcinoma, cervical cancer, adenocarcinoma, liver cancer, lung cancer, breast cancer, cysteine cancer, renal cancer, childhood leukaemia, acute myelocytic leukaemia, chronic myeloid leukaemia, multiple myeloma, squamous cell carcinoma, cervical cancer, or epithelial cell carcinoma. The tumour may also be other tumours with high sialic acid expression. In addition, the tumour types with high sialic acid expression may refer to the records in the literature Exploration of sialic acid receptors as a potential target for cancer treatment: A comprehensive review.

In yet another aspect, the present invention further provides the preparation method of the lipoic acid derivative, wherein,
when A is selected from 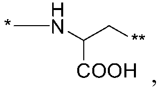 and L₁ is selected from a bond or 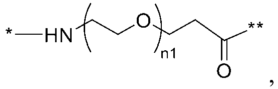
the method comprises preparing a lipoic acid derivative using a compound of formula (11) and a phenylboronic acid derivative of formula (12) as starting materials:

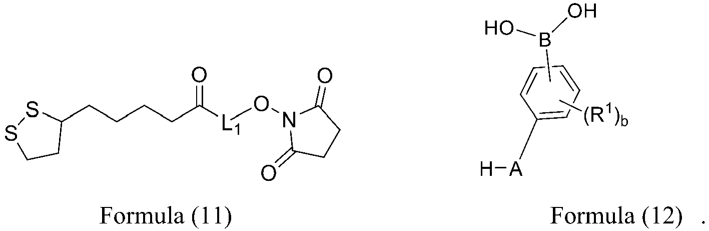

According to some specific embodiments of the present invention, wherein the method further comprises preparing the compound of formula (11) using a lipoic acid compound of formula (13) as a starting material:

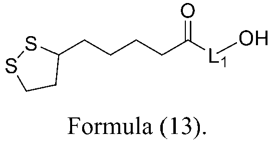

According to some specific embodiments of the present invention, wherein the method further comprises preparing the compound of formula (11) using a lipoic acid compound of formula (13) and N-hydroxysuccinimide as starting materials:

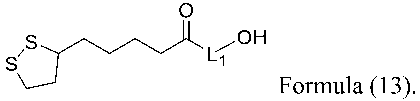

According to some specific embodiments of the present invention, wherein,
when L1 is 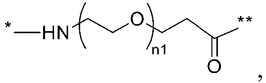 the method further comprises:
preparing the compound of formula (13) using a compound of formula (14) as a starting material: 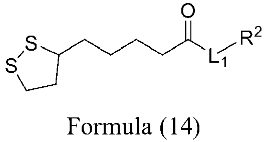
R² is a carboxyl protecting group.

According to some specific embodiments of the present invention, wherein the method further comprises preparing the compound of formula (14) using a compound of formula (15) and a compound of formula (16) as starting materials: 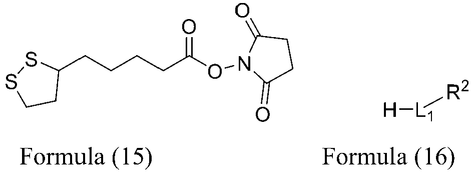 in the compound of Formula (16), R² is a carboxyl protecting group.

In some embodiments of the present invention, the method prepares formula (14) via formula (15) and formula (16), and then prepares the compound of formula (13) via formula (14). In other embodiments of the present invention, the method can prepare the compound of formula (13) via formula (15) and formula (18).

According to some specific embodiments of the present invention, wherein,
when L₁ is 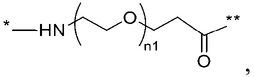 the method further comprises:
preparing the compound of formula (13) using a compound of formula (15) and a compound of formula (18) as starting materials:

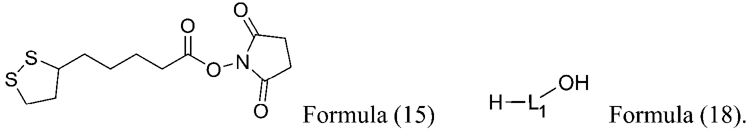

That is to say, when L1 in the compound of formula (11) is a bond, the compound of formula (11) is the same as the compound of formula (15). It can be seen that the preparation of the compound of formula (15) can refer to the preparation of formula (11).

According to some specific embodiments of the present invention, wherein the method further comprises preparing the compound of formula (15) using lipoic acid of formula (17) and N-hydroxysuccinimide as starting materials:

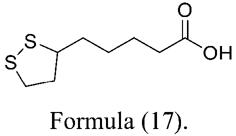

N-hydroxysuccinimide, also known as N-hydroxysuccimide (NHS), has the structure

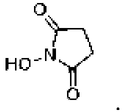

According to some specific embodiments of the present invention, the compound of formula (16) is 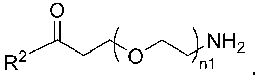 In the compound of Formula (16), R² is a carboxyl protecting group. Further, R² is selected from -OR³ and -NR⁴R⁵.

According to some specific embodiments of the present invention, wherein the compound of formula (16) has the following structure: 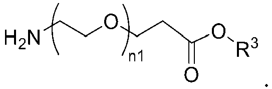 wherein n1 is selected from 0, 1, 2, 3, 4, 5, 6, 7, 8, 9, 10, 11, or 12.

According to some specific embodiments of the present invention, wherein R³ is selected from alkyl having 1 to 20 carbon atoms and heteroalkyl having 1 to 20 carbon atoms. The heteroalkyl contains one or more heteroatoms, such as nitrogen, oxygen, or sulphur.

According to some specific embodiments of the present invention, R³ is selected from, but not limited to, methyl, dimethyl, ethyl, tert-butyl, benzyl, diphenylmethyl, p-nitrobenzyl, p-methoxybenzyl, 4-pyridylmethyl, β,β,β-trichloroethyl, β-methylthioethyl, β-p-toluenesulfonylethyl or β-p-nitrophenylthioethyl.

According to some specific embodiments of the present invention, when R² is selected from -OR³, and R³ is selected from tert-butyl, the compound of formula (16) is 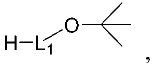 that is, 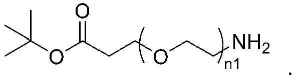 Correspondingly, the compound of formula (14) is

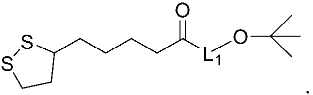

According to some specific embodiments of the present invention, wherein the compound of formula (16) has the following structure: 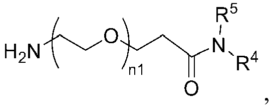 wherein n1 may be 0, 1, 2, 3, 4, 5, 6, 7, 8, 9, 10, 11, or 12.

According to some specific embodiments of the present invention, R⁴ is selected from, but not limited to, hydrogen, alkyl having 1 to 20 carbon atoms and heteroalkyl having 1 to 20 carbon atoms. The heteroalkyl contains one or more heteroatoms, such as nitrogen, oxygen, or sulphur.

According to some specific embodiments of the present invention, R⁵ is selected from, but not limited to, alkyl having 1 to 20 carbon atoms and heteroalkyl having 1 to 20 carbon atoms. The heteroalkyl contains one or more heteroatoms, such as nitrogen, oxygen, or sulphur.

According to some specific embodiments of the present invention, wherein when L₁ is a bond, the preparation method of the lipoic acid derivative comprises:

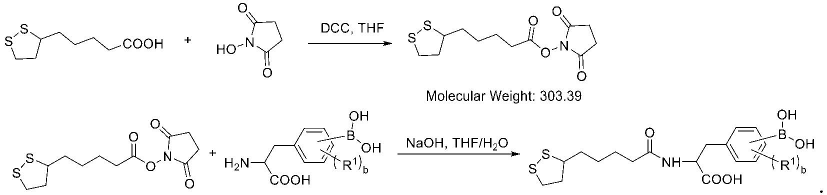

According to some specific embodiments of the present invention, wherein when L₁ is a bond, the preparation method of the lipoic acid derivative comprises:

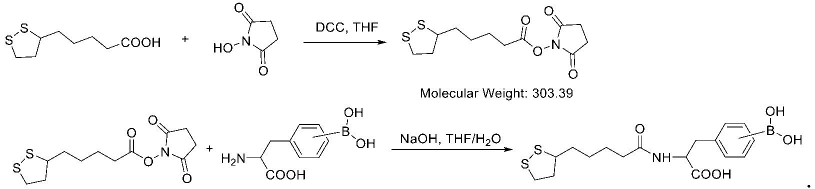

According to some specific embodiments of the present invention, wherein when L₁ is 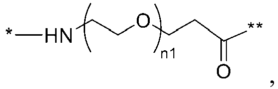 the preparation method of the lipoic acid derivative comprises:

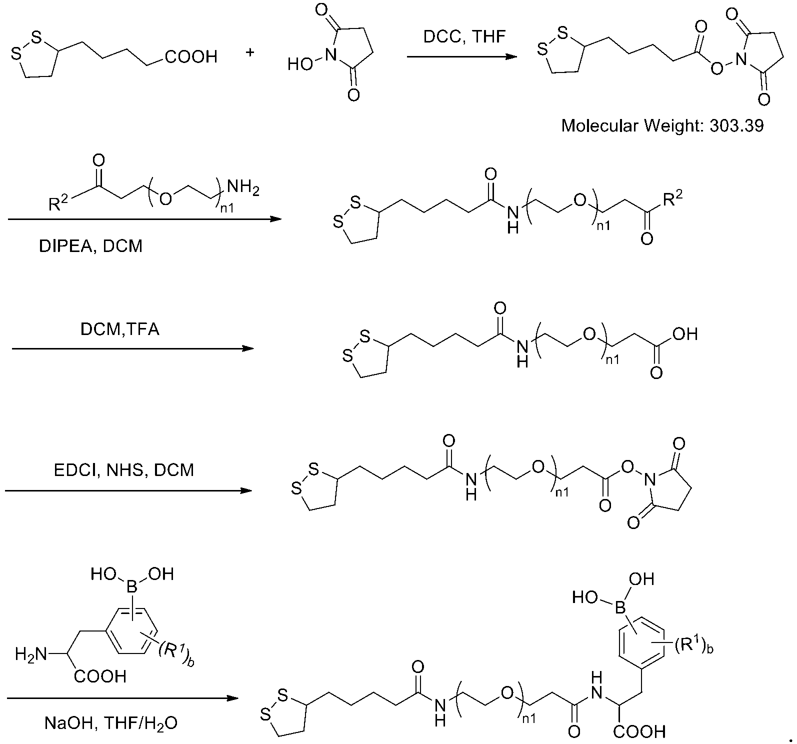

According to some specific embodiments of the present invention, wherein when L₁ is 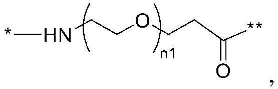 the preparation method of the lipoic acid derivative comprises:

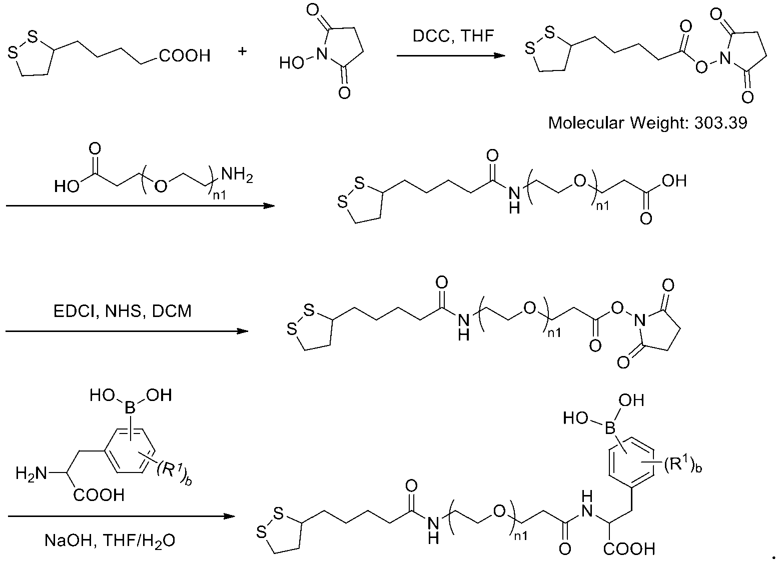

According to some specific embodiments of the present invention, wherein the preparation method of the lipoic acid derivative comprises:

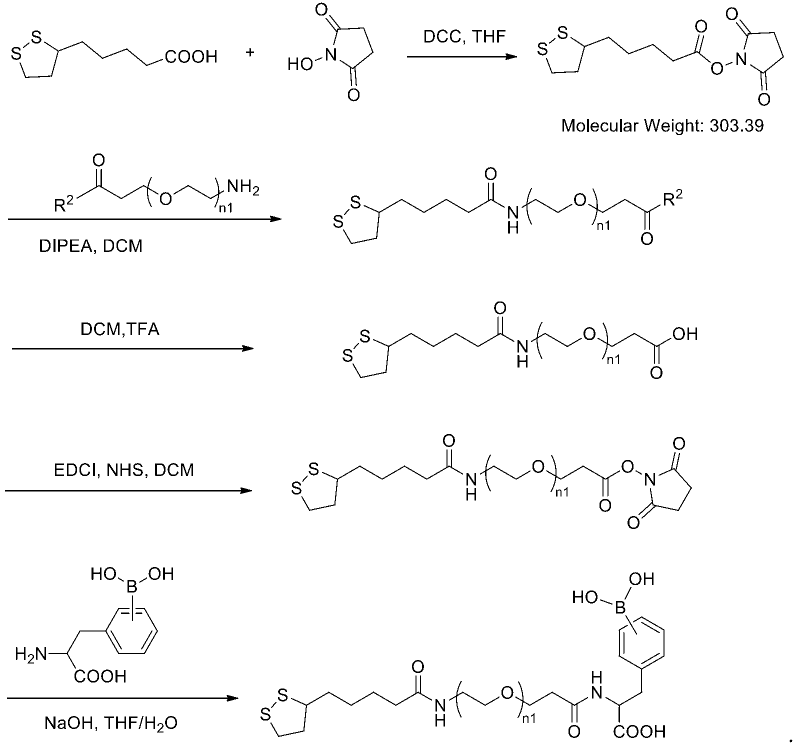

According to some specific embodiments of the present invention, wherein the preparation method of the lipoic acid derivative comprises:

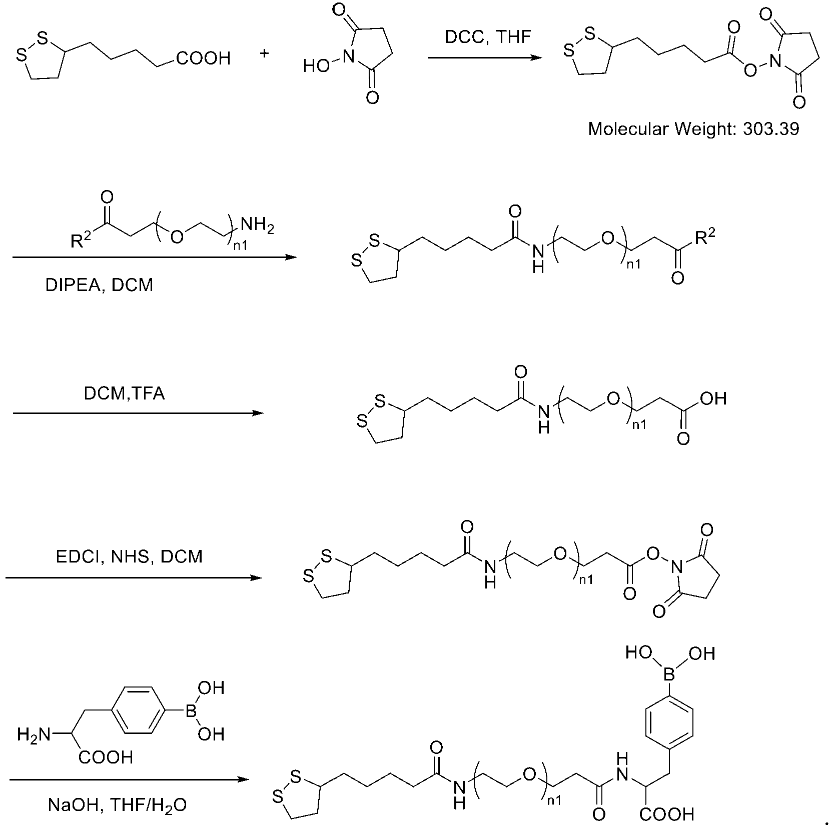

According to some specific embodiments of the present invention, wherein when L₁ is 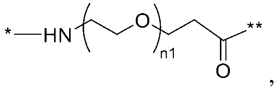 the preparation method of the lipoic acid derivative comprises:

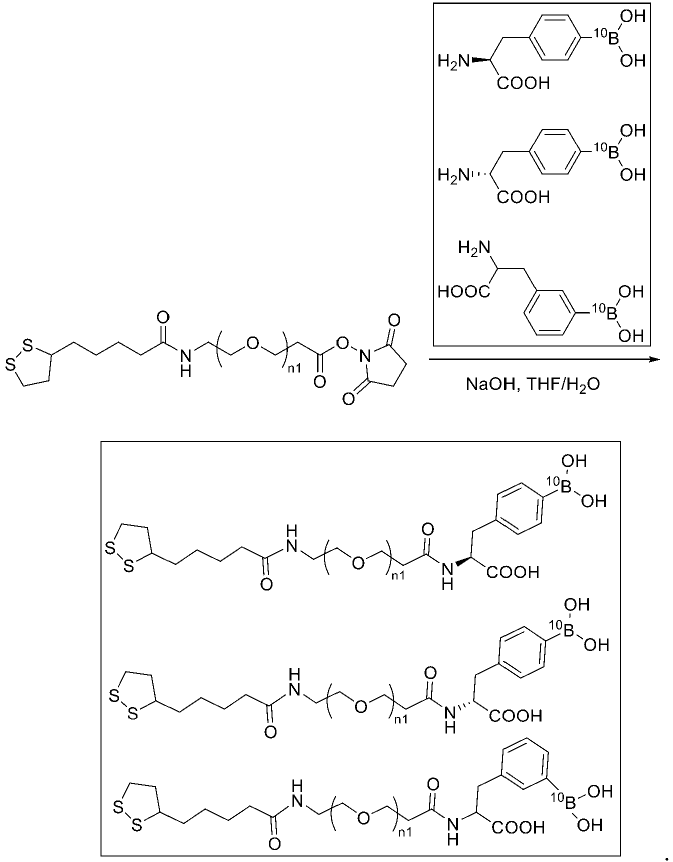

It is conceivable that in each of the aforementioned preparation methods, when a starting material containing ¹⁰B (boron-10) is employed, a lipoic acid derivative containing ¹⁰B can be obtained.

The B of the present invention may be ¹¹B or ¹⁰B, unless otherwise specified in the present invention.

In yet another aspect, the present invention further provides a preparation method of the vesicle compound, wherein the preparation method comprises preparing the vesicle compound using the above-mentioned lipoic acid derivative as the starting material.

According to some specific embodiments of the present invention, the preparation method comprises: reacting the lipoic acid derivative under a condition of pH less than or equal to 7.2 to form a vesicle compound; subjecting the five-membered heterocycle in the lipoic acid derivative to ring opening and disulfide bond cross-linking to form a nanovesicle.

According to some specific embodiments of the present invention, the preparation method comprises: adding a base to the reaction system of the lipoic acid derivative to dissolve the compound, followed by adding an acid to adjust the pH to less than or equal to 7.2, followed by reaction to form vesicle nanoparticles.

According to some specific embodiments of the present invention, the preparation method comprises: reacting a lipoic acid derivative under a condition of pH 5 to 7.2 to form a vesicle compound. Further, the pH may be 5, 5.1, 5.2, 5.4, 5.5, 5.6, 5.7, 5.8, 6, 6.5, 6.8, 7, 7.1, or 7.2.

According to some specific embodiments of the present invention, the reaction solvent in the reaction system may be an aqueous solvent. For example, the aqueous solvent includes, but is not limited to, at least one of water, sodium chloride solution, and glucose solution.

According to some specific embodiments of the present invention, the preparation method comprises: adding a base to the lipoic acid derivative such that the pH value of the system is greater than or equal to 8 to dissolve the compound.

According to some specific embodiments of the present invention, wherein the lipoic acid derivative assembles to form a vesicle nanoparticle, which is reversible in alternating acidic and alkaline environments.

According to some specific embodiments of the present invention, the preparation method of the vesicle nanoparticle comprises: adding a base to dissolve the lipoic acid derivative, followed by adding an acid to adjust the pH to form vesicle nanoparticles by polymerization.

According to some specific embodiments of the present invention, the pH after adding the base is greater than or equal to 8, such as but not limited to 8, 8.5, 9, 9.5, 10, 10.5, 11, 11.5, 12, 12.5, 13, or 13.5.

According to some specific embodiments of the present invention, the pH after adding the acid is less than or equal to 7.2, such as but not limited to 5, 5.1, 5.2, 5.4, 5.5, 5.6, 5.7, 5.8, 6, 6.5, 6.8, 7, 7.1, or 7.2.

It is conceivable that the aforementioned formed vesicle nanoparticles can be depolymerized by adding base again to obtain the lipoic acid derivative. Further, the lipoic acid derivative obtained by depolymerization can be polymerized by adding acid again to form vesicle nanoparticles. This process can be repeated.

According to some specific embodiments of the present invention, wherein the preparation method of the vesicle further comprises, after the reaction to form the vesicle nanoparticle, repeating the steps of adding a base to the reaction system for reaction and then adding an acid for reaction; wherein when adding the base, a pH value of the reaction system is adjusted to be not less than 8; when adding the acid, a pH value of the reaction system is adjusted to be not greater than 7.2.

According to some specific embodiments of the present invention, wherein the method further comprises: after the reaction to form vesicle nanoparticle, adding a base to the reaction system to adjust the pH value of the system to not less than 8, then adding an acid to adjust the pH value of the system to not greater than 7.2, then adding a base to adjust the pH value to not less than 8, then adding an acid to adjust the pH value to not greater than 7.2, repeating multiple times, and finally adding an acid to adjust the pH value of the system to not greater than 7.2 to form vesicle nanoparticle. That is, the vesicles are assembled repeatedly multiple times.

According to some specific embodiments of the present invention, wherein the repetition of the step of repeatedly adding a base to the reaction system for reaction and then adding an acid for reaction can be determined according to actual needs. The repetition of this step can make the vesicle grain size more uniform. That is to say, during the preparation of vesicles, repeating the steps of depolymerization and polymerization helps to improve the uniformity of the formed vesicle size. For example, the number of repetitions may be 1, 2, 3, 4, 5, 6, 7, 8, 9, 10, 15, 20, 25, 30, 40, 50, 60 times or more.

According to some specific embodiments of the present invention, the grain size of the vesicle can be regulated by repeated assembly and/or PEG chain.

According to some specific embodiments of the present invention, wherein the base may be a conventional inorganic base or organic base in the art.

According to some specific embodiments of the present invention, wherein the base is selected from, but not limited to, sodium hydroxide, potassium hydroxide, sodium carbonate, potassium carbonate, sodium bicarbonate, and potassium bicarbonate.

According to some specific embodiments of the present invention, wherein the base is selected from, but not limited to, amine compounds, nitrogen-containing heterocyclic compounds, organometallic compounds, and quaternary ammonium salts, and may also be selected from triethanolamine, monoethanolamine, trimethylamine, triethylamine, and diethylenetriamine.

According to some specific embodiments of the present invention, wherein the acid may be conventional inorganic acids or organic acids in the art.

According to some specific embodiments of the present invention, wherein the acid is selected from, but not limited to, hydrochloric acid, formic acid, acetic acid, trifluoroacetic acid, and sulfuric acid.

According to some specific embodiments of the present invention, the preparation method comprises: reacting a lipoic acid derivative under a condition of pH 5 to 7.2 to form a vesicle compound.

According to some specific embodiments of the present invention, wherein in the preparation method of the vesicle compound, the concentration of the lipoic acid derivative in the reaction system is greater than a critical aggregation concentration.

According to some specific embodiments of the present invention, the critical aggregation concentration is 230 uM to 300 uM.

Further, the critical aggregation concentration may be 230 uM, 240 uM, 250 uM, 260 uM, 270 uM, 280 uM, 290 uM, or 300 uM.

According to some specific embodiments of the present invention, the detailed structure of the lipoic acid derivative is as described above. A plurality of lipoic acid derivatives polymerize through the breaking and cross-linking of disulfide bonds, thereby forming the vesicle nanoparticles of the present invention.

In summary, the present invention provides a lipoic acid derivative, a vesicle compound, and preparation therefor and use thereof. The technical solution of the present invention has the following advantages:
the lipoic acid derivative developed in the present invention is a novel phenylboronic acid-based boron drug. One end of the boron drug is phenylboronic acid and the other end of the boron drug is lipoic acid, with a bond or a linking group linked therebetween. This amphiphilic small molecule compound (with phenylboronic acid as the hydrophilic end and the five-membered heterocycle of lipoic acid as the hydrophobic end) forms a nanovesicle through self-assembly, opening of the five-membered heterocycle, and cross-linking of disulfide bonds. The nano-drug uses the phenylboronic acid end as the hydrophilic end of the nanovesicle, and phenylboronic acid as the hydrophilic-end compound can confer the biological function of targeting the nano-drug to sialic acid, significantly enhancing the effective enrichment of the nano-drug at the tumour site; meanwhile, it can also achieve the purpose of inhibiting the reverse transport mechanism of amino acids, prolonging the retention time of the boron drug in cancer cells, which is beneficial for the implementation of BNCT.

In addition, this vesicle can serve as a delivery system for small-molecule theranostic agents, enabling in vivo visualization of nano-drugs, and can also be used in combination with other anti-tumour active drugs to further enhance the anti-tumour effect of BNCT.

### BRIEF DESCRIPTION OF THE DRAWINGS

FIG. 1 shows the critical aggregation concentration diagram of the product in vesicle example 1;
FIG. 2 shows the transmission scanning electron microscopy image of the product in vesicle example 1;
FIG. 3 shows the hydrated grain size diagram of the product in vesicle example 1 detected by an exosome grain size tracking analyser;
FIG. 4 shows the transmission electron microscopy (TEM) image of test example 1;
FIG. 5 shows the vesicle grain size diagram A of test example 2;
FIG. 6 shows the vesicle grain size diagram B of test example 2;
FIG. 7 shows the grain size change diagram of vesicle nanoparticles as a function of pH in test example 2;
FIG. 8 shows the zeta potential change trend diagram of vesicles under different pH conditions in test example 2;
FIG. 9 shows the schematic diagram of reversible assembly of vesicles in test example 2;
FIG. 10 shows the ultraviolet absorption value change diagram during the depolymerization into monomers-reassembly into vesicles cycle in test example 2;
FIG. 11 shows the nuclear magnetic resonance spectrum of the recovered depolymerized monomers in test example 2;
FIG. 12 shows the grain size distribution diagram of vesicle nanoparticles during three cycles of polymerization-depolymerization in test example 2;
FIG. 13 shows the schematic diagram of lipoic acid derivative self-assembly to form vesicles in test example 2;
FIG. 14 shows the confocal imaging diagram of fluorescently labelled vesicle nanoparticles in test example 3;
FIG. 15 shows the change trend diagram of cell proliferation activity as a function of increasing drug concentration in test example 4;
FIG. 16 shows the confocal microscope imaging diagram of cancer cell uptake in test example 5;
FIG. 17 shows the in vivo distribution diagram in tumour-bearing nude mice in test example 6;
FIG. 18 shows the tumour volume statistics of each experimental group in test example 6;
FIG. 19 shows the body weight statistics of mice in each experimental group in test example 6;
FIG. 20 shows the statistical results of the survival time of mice in each experimental group in test example 6;
FIG. 21 shows the tumour and body weight changes in mice after neutron irradiation in the PEG1, PEG2, PEG4, and PEG8 vesicle groups in test example 6;
In FIG. 22, panel a shows the B-10 concentration in test example 7, and panel b shows the tumour cell viability results of test example 7;
FIG. 23 shows the flow cytometer detection results of cell apoptosis in each experimental group in test example 7;
FIG. 24 shows the confocal imaging results of live/dead cell staining in each experimental group in test example 7;
FIG. 25 shows the γ-H2AX staining imaging results in each experimental group in test example 7;
FIG. 26 shows the in vivo distribution diagram of fluorescent probe-loaded vesicles versus free probe in test example 8;
FIG. 27 shows the distribution results diagram of vesicles in various organ tissues in test example 8;
FIG. 28 shows the distribution results diagram of boron-10 in various organ tissues in test example 8;
FIG. 29 shows the HE pathological images of major organs (excluding the liver) in the experimental group in test example 9;
FIG. 30 shows the HE pathological image of the liver in the experimental group in test example 9;
FIG. 31 shows the HE pathological image of the tumour in the experimental group in test example 9;
FIG. 32 shows the fluorescence imaging results of the Ki-67 proliferation assay and TUNEL apoptosis assay in each experimental group in test example 9;
FIG. 33 shows the cell uptake results (c) and statistics (d) of PANC-1 cells untreated and treated with neuraminidase in test example 10;
FIG. 34 shows the schematic diagram of the sialic acid-targeting mechanism of vesicles;
FIG. 35 shows the uptake of vesicles in cancer cells with high sialic acid expression in each experimental group in test example 10;
FIG. 36 shows the in vivo fluorescence distribution diagram in test example 11;
FIG. 37 shows the fluorescence distribution diagram in various tissues in test example 11;
FIG. 38 shows the distribution of boron-10 in various organ tissues in test example 11;
FIG. 39 shows the tumour volume statistics in each experimental group in test example 12;
FIG. 40 shows a comparison diagram of tumour volumes among the experimental groups in test example 12;
FIG. 41 shows the body weights diagram of mice in each experimental group in test example 12;
FIG. 42 shows the statistical results of the survival time in each experimental group in test example 12;
FIG. 43 shows the tumour HE pathological images in each experimental group in test example 12;
FIG. 44 shows the organ HE pathological images in each experimental group in test example 12.

### DETAILED DESCRIPTION OF THE INVENTION

The following describes the implementation process of the present invention and the beneficial effects produced through specific examples in detail, aiming to help readers better understand the essence and characteristics of the present invention, and is not intended to limit the scope of implementation of the present case.

### Lipoic acid derivative example 1

This example provides a lipoic acid derivative (LA-BPA). The synthesis steps of LA-BPA are shown below:

### Step 1: Preparation of LA-NHS

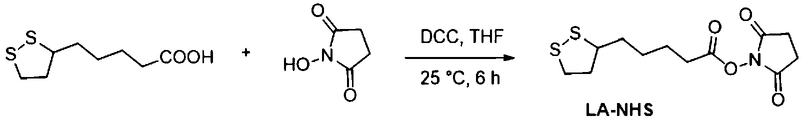

LA-NHS: Lipoic acid (4.12 g, 19.97 mmol) and NHS (N-hydroxysuccinimide, 2.76 g, 23.98 mmol) were weighed and placed into a reaction vessel, and dissolved in 100 mL of anhydrous THF (tetrahydrofuran); DCC (N,N-dicyclohexylcarbodiimide, 2.96 g, 14.35 mmol) was dissolved in 50 mL of anhydrous THF, then added dropwise to the reaction vessel under ice-water bath conditions, and the mixture was stirred at room temperature for 6 h; 200 mL of ethyl acetate was added to the reaction solution, the DCC byproduct was precipitated, removed by vacuum filtration, and the solvent was removed under vacuum using a rotary evaporator. 200 mL of ethyl acetate was added again, the byproduct was removed under vacuum again, and the solvent was removed under vacuum using a rotary evaporator. The product was dissolved in ethyl acetate/n-hexane (V/V = 1 : 1) under heating, and after complete dissolution, the excess byproduct was removed again by hot filtration under vacuum. After cooling to room temperature, the mixture was stored in a 4°C refrigerator overnight to allow product recrystallization, yielding 5.60 g of pale yellow crystalline product with a yield of approximately 92%.

### Step 2: Preparation of LA-BPA

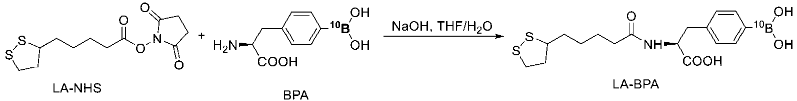

LA-BPA: LA-NHS (100 mg, 0.3296 mmol) was dissolved in 3.5 mL of THF; BPA (55 mg, 0.2642 mmol) was weighed and placed into a reaction vessel, 3.5 mL of double-distilled water was added and mixed by vortexing, followed by addition of 0.4 mL of 2.5 M NaOH aqueous solution. After dissolution of BPA, LA-NHS was added dropwise to the reaction vessel, and the mixture was reacted under stirring at room temperature. The organic solvent was removed under vacuum using a rotary evaporator. Purification by C18 silica gel column chromatography and lyophilization yielded 82 mg of LA-BPA as a yellow solid product with a yield of approximately 80%.

MS(m/z, ACN/H⁺):396.9[M+H⁺].

¹H NMR (400 MHz, DMSO-d₆) δ 7.72 - 7.59 (m, 2H), 7.18 (d, J = 7.7 Hz, 2H), 4.43 (dd, J = 9.8, 4.8 Hz, 1H), 3.48 (dt, J = 14.3, 6.1 Hz, 1H), 3.13 - 2.99 (m, 3H), 2.82 (dd, J = 13.9, 10.0 Hz, 1H), 2.34 (tq, J = 9.6, 3.2 Hz, 1H), 2.03 (t, J = 7.3 Hz, 2H), 1.80 (dqd, J = 13.2, 6.6, 6.1, 2.5 Hz, 1H), 1.67 - 1.30 (m, 4H), 1.22 - 1.10 (m, 2H) .

¹³C NMR (101 MHz, DMSO) δ 173.61, 173.17, 140.10, 134.45, 132.01, 128.65, 56.52, 53.56, 40.34, 40.00, 39.80, 39.59, 39.38, 39.17, 38.96, 38.74, 38.47, 37.11, 35.25, 34.42, 28.35, 25.36, 25.32.

### Lipoic acid derivative example 2:

This example provides a lipoic acid derivative (LA-PEG₄-BPA, denoted as L^{p}B-3), the synthesis route is as follows: LA-PEG₄-BPA:

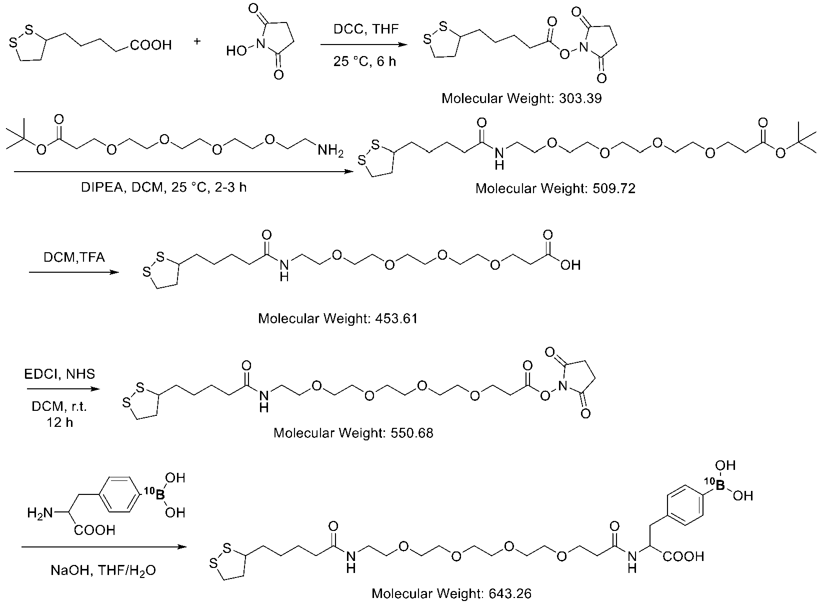

The synthesis steps of the lipoic acid derivative (LA-PEG₄-BPA) in this example are as follows:

### Step 1: Preparation of LA-NHS

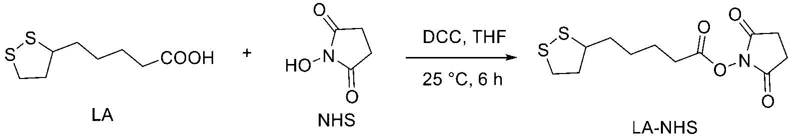

LA-NHS: Lipoic acid (4.12 g, 19.97 mmol) and NHS (N-hydroxysuccinimide, 2.76 g, 23.98 mmol) were weighed and placed into a reaction vessel, and dissolved in 100 mL of anhydrous THF (tetrahydrofuran); DCC (N,N-dicyclohexylcarbodiimide, 2.96 g, 14.35 mmol) was dissolved in 50 mL of anhydrous THF, then added dropwise to the reaction vessel under ice-water bath conditions, and the mixture was stirred at room temperature for 6 h; 200 mL of ethyl acetate was added to the reaction solution, the byproduct was precipitated, removed by vacuum filtration, and the solvent was removed under vacuum using a rotary evaporator. 200 mL of ethyl acetate was added again, the byproduct was removed under vacuum again, and the solvent was removed under vacuum using a rotary evaporator. The product was dissolved in ethyl acetate/n-hexane (V/V = 1 : 1) under heating, and after complete dissolution, the excess byproduct was removed again by hot filtration under vacuum. After cooling to room temperature, the mixture was stored in a 4°C refrigerator overnight to allow product recrystallization, yielding 5.60 g of pale yellow crystalline product with a yield of approximately 92%.

¹H NMR (400 MHz, Chloroform-d) δ 3.58 (dq, J = 8.2, 6.4 Hz, 1H), 3.25 - 3.07 (m, 2H), 2.92 - 2.77(m, 4H), 2.63 (t, J = 7.4 Hz, 2H), 2.54 - 2.41 (m, 1H), 1.93 (dq, J = 13.6, 6.9 Hz, 1H), 1.85 - 1.68 (m, 4H), 1.62 - 1.51 (m, 2H).

¹³C NMR (101 MHz, CDCl₃) δ 169.25, 168.54, 56.21, 40.28, 38.64, 34.54, 30.91, 28.44, 25.72, 24.48.

### Step 2: Preparation of LA-PEG4-COOtBu

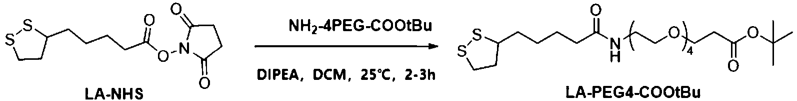

LA-NHS (3.03 g, 9.98 mmol) and NH₂-PEG₄-COOtBu (2.65 g, 8.25 mmol) were weighed and placed into a reaction vessel, 60 mL of anhydrous DCM (dichloromethane) was added under argon protection, stirred for dissolution, then DIPEA (2.50 g, 19.34 mmol) was added dropwise. The reaction was stirred at room temperature for 2-3 h, the solvent was removed under vacuum using a rotary evaporator, and purification by silica gel column chromatography yielded 4.10 g of the product as a pale yellow oil, with a yield of approximately 97.50%.

¹H NMR (400 MHz, Chloroform-d) δ 6.20 (t, J = 5.7 Hz, 1H), 3.71 (t, J = 6.6 Hz, 2H), 3.68 - 3.58 (m, 12H), 3.56 (dd, J = 5.7, 4.5 Hz, 2H), 3.44 (q, J = 5.3 Hz, 2H), 3.22 - 3.08 (m, 2H), 2.54 - 2.40 (m, 3H), 2.19 (t, J = 7.5 Hz, 2H), 1.91 (dq, J = 13.6, 6.9 Hz, 1H), 1.78 - 1.60 (m, 4H), 1.54 - 1.47 (m, 1H), 1.45 (s, 9H).

¹³C NMR (101 MHz, CDCl3) δ 172.72, 170.81, 80.49, 70.57, 70.51, 70.49, 70.46, 70.34, 70.21, 69.85, 66.86, 56.39, 40.20, 39.14, 38.44, 36.30, 36.23, 34.65, 28.89, 28.08, 25.36.

HRMS (ESI) m/z calcd for C₂₃H₄₄NO₇S₂⁺ (M + H)⁺ 510.2554, found 510.2561.

### Step 3: LA-PEG₄-COOH

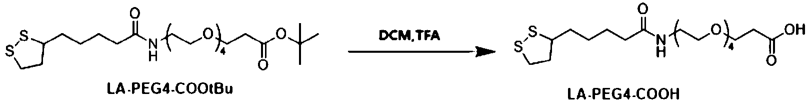

LA-PEG₄-COOtBu (3.03 g, 5.94 mmol) was weighed and placed into a reaction vessel, 40 mL DCM was added, after complete dissolution, 20 mL TFA (trifluoroacetic acid) was added, the reaction was stirred at room temperature for 1 h, the solvent was removed under vacuum using a rotary evaporator, and purification by silica gel column chromatography yielded 2.40 g of the product as a pale yellow oil, with a yield of approximately 89%.

### Step 4: Preparation of LA-PEG₄-NHS ester

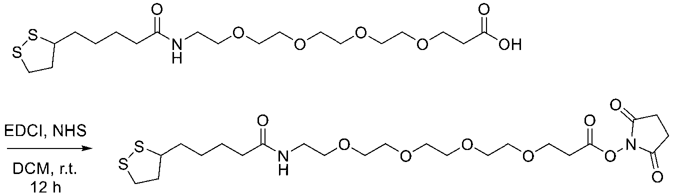

LA-PEG₄-COOH (2.70 g, 5.95 mmol), NHS (0.828 g, 7.19 mmol), and EDCI (1-(3-dimethylpropyl)-3-ethylcarbodiimide hydrochloride, 1.38 g, 7.20 mmol) were weighed and placed into a reaction vessel, 80 mL of anhydrous DCM (dichloromethane) was added, and the reaction was stirred at room temperature for 12 h. The solvent was removed under vacuum using a rotary evaporator, and purification by silica gel column chromatography yielded 2.90 g of the product as a pale yellow oil, with a yield of approximately 88.57%.

¹H NMR (400 MHz, Chloroform-d) δ 6.57 (s, 1H), 3.78 (t, J = 6.4 Hz, 2H), 3.66 - 3.53 (m, 11H), 3.48 (t, J = 5.1 Hz, 2H), 3.37 (q, J = 5.2 Hz, 2H), 3.14 - 3.01 (m, 2H), 2.90 - 2.68 (m, 8H), 2.43 - 2.35 (m, 1H), 2.13 (t, J = 7.5 Hz, 2H), 1.88 - 1.80 (m, 1H), 1.70 - 1.51 (m, 4H), 1.47 - 1.32 (m, 2H).

¹³C NMR (101 MHz, CDCl3) δ 173.16, 169.05, 166.73, 70.67, 70.54, 70.47, 70.44, 70.35, 70.12, 70.01, 65.70, 56.47, 40.23, 39.18, 38.46, 36.23, 34.65, 32.09, 28.91, 25.60, 25.39.

HRMS (ESI) m/z calcd for C₂₃H₃₈N₂O₉S₂⁺ (M + H)⁺ 550.2019, found 551.2102.

### Step 5: Preparation of LA-PEG₄-BPA

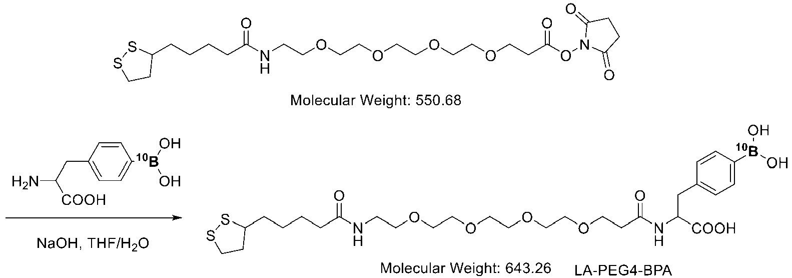

LA-PEG₄-NHS ester (0.66 g, 1.20 mmol) was dissolved in 10 mL of THF; BPA (0.25 g, 1.20 mmol) was weighed and placed into a reaction vessel, 7 mL of double-distilled water was added and mixed by vortexing, followed by addition of 2 mL of 2.5 M NaOH aqueous solution. After dissolution of BPA, LA-PEG₄-NHS was added dropwise to the reaction vessel, and the reaction was stirred at room temperature for 6-8 h. The organic solvent was removed under vacuum using a rotary evaporator. Purification by C18 silica gel column chromatography and lyophilization yielded 0.68 g of LA-PEG₄-BPA as a yellow solid product with a yield of approximately 87.5%.

¹H NMR (400 MHz, Deuterium Oxide) δ 7.63 (d, J = 6.7 Hz, 2H), 7.32 - 7.11 (m, 2H), 4.43 (dd, J = 8.8, 4.7 Hz, 1H), 3.62 - 3.48 (m, 12H), 3.45 - 3.34 (m, 4H), 3.29 (t, J = 5.3 Hz, 2H), 3.18 - 3.06 (m, 2H), 2.88 (dd, J = 14.0, 8.8 Hz, 1H), 2.46 - 2.28 (m, 3H), 2.15 (t, J = 7.3 Hz, 2H), 1.94 - 1.83 (m, 1H), 1.68 - 1.57 (m, 1H), 1.56 - 1.47 (m, 2H), 1.39 - 1.22 (m, 2H) .

¹³C NMR (101 MHz, D₂O) δ 177.95, 176.86, 173.13, 160.33, 133.73, 128.84, 69.62, 69.57, 69.50, 69.42, 69.34, 68.88, 66.67, 56.49, 55.97, 40.24, 38.91, 38.06, 37.65, 35.94, 35.46, 33.71, 27.83, 25.01.

HRMS (ESI) m/z calcd for C₂₈H₄₆¹⁰BN₂O₁₀S₂⁺ (M + H)⁺ 644.2718, found 644.2723.

### Lipoic acid derivative example 3:

This example provides a lipoic acid derivative (LA-PEG₂-BPA, denoted as L^{p}B-2). The synthesis route is as follows:

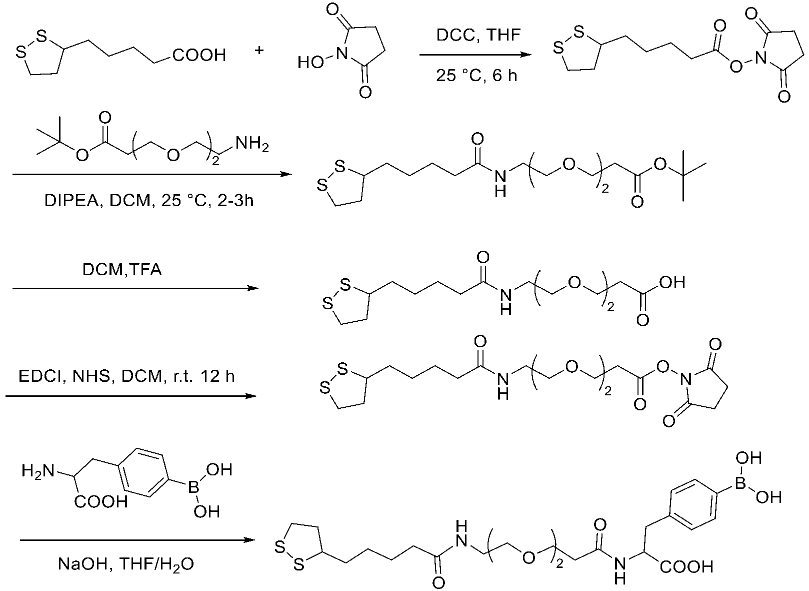

The synthesis steps of LA-PEG₂-BPA in this example refer to the lipoic acid derivative example 2, with the difference being that PEG₄ is replaced with PEG₂ in this example.

¹H NMR (400 MHz, D₂O) δ 8.36 (s, 2H), 7.67 - 7.63 (m, 2H), 7.28 - 7.22 (m, 2H), 4.46 (dd, J = 8.8, 4.7 Hz, 1H), 3.61 - 3.31 (m, 12H), 3.27 (t, J = 5.3 Hz, 2H), 3.19 - 3.03 (m, 3H), 2.90 (dd, J = 14.0, 8.8 Hz, 1H), 2.44 - 2.33 (m, 3H), 2.14 (t, J = 7.3 Hz, 2H), 1.87 (dq, J = 13.7, 6.9 Hz, 1H), 1.57 - 1.44 (m, 4H), 1.33 - 1.25 (m, 2H).

¹³C NMR (101 MHz, D₂O) δ 177.66, 176.84, 173.21, 170.50, 140.71, 133.91, 128.91, 69.40, 68.89, 66.67, 56.51, 55.76, 40.25, 38.94, 38.08, 37.60, 35.94, 35.46, 33.71, 27.84, 25.03.

### Lipoic acid derivative example 4:

This example provides a lipoic acid derivative (LA-PEG₈-BPA, denoted as L^{p}B-4). The synthesis route is as follows:

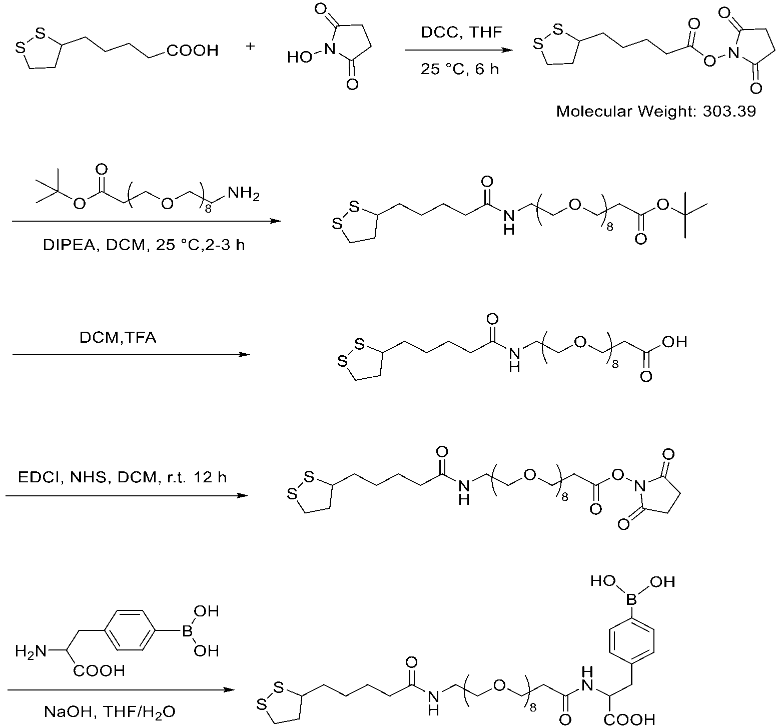

The synthesis steps of LA-PEG₈-BPA in this example refer to the lipoic acid derivative example 2, with the difference being that PEG₄ is replaced with PEG₈ in this example.

¹H NMR (400 MHz, CD₃OD) δ 7.66 (s, 1H), 7.56 (s, 1H), 7.29 - 7.15 (m, 2H), 4.68 (dd, J = 8.4, 5.0 Hz, 1H), 3.65 - 3.58 (m, 25H), 3.53 (tq, J = 4.8, 2.2 Hz, 6H), 3.35 (t, J = 5.5 Hz, 2H), 3.25 - 3.14 (m, 2H), 3.14 - 3.06 (m, 1H), 3.00 (dd, J = 13.9, 8.5 Hz, 1H), 2.50 - 2.38 (m, 3H), 2.20 (t, J = 7.4 Hz, 2H), 1.88 (dq, J = 13.5, 6.9 Hz, 1H), 1.76 - 1.58 (m, 4H), 1.52 - 1.40 (m, 2H).

¹³C NMR (101 MHz, CD₃OD) δ 174.67, 173.23, 172.36, 133.57, 128.27, 70.18, 70.16, 70.13, 70.11, 69.97, 69.89, 69.88, 69.20, 66.71, 56.20, 53.46, 39.93, 38.99, 37.96, 37.07, 35.98, 35.42, 34.36, 28.48, 25.33.

### Lipoic acid derivative example 5:

This example provides a lipoic acid derivative (LA-PEG₄-3-BPA, denoted as L^{m}B-3). The synthesis route is as follows:

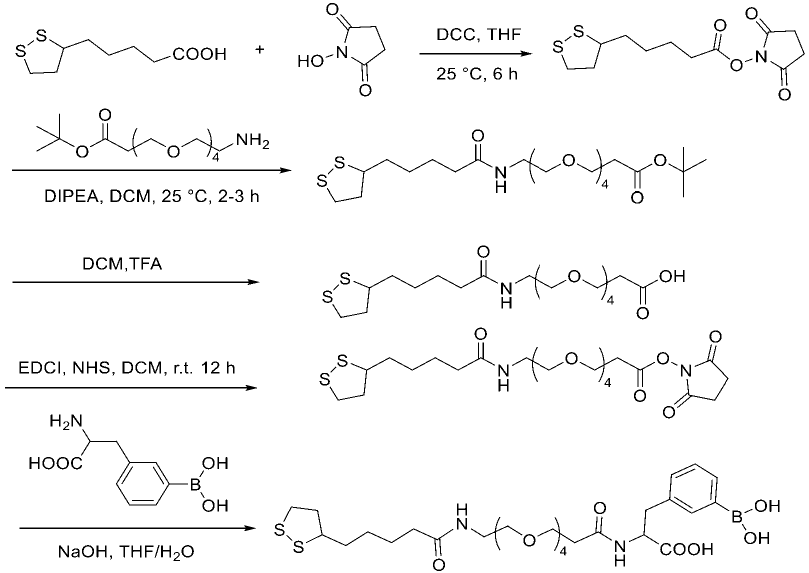

The synthesis steps of LA-PEG₄-3-BPA in this example refer to the lipoic acid derivative example 2, with the difference being that BPA is replaced with 3-BPA of this example in the final step.

¹H NMR (400 MHz, D₂O) δ 8.36 (s, 2H), 7.60 - 7.50 (m, 2H), 7.34 - 7.21 (m, 2H), 4.43 (dt, J = 8.8, 5.0 Hz, 1H), 3.61 - 3.39 (m, 20H), 3.28 (t, J = 5.4 Hz, 3H), 3.21 - 2.98 (m, 2H), 2.88 (dd, J = 14.0, 8.7 Hz, 1H), 2.38 (dddd, J = 24.1, 15.0, 11.4, 6.2 Hz, 3H), 2.15 (q, J = 6.2 Hz, 2H), 1.86 (tt, J = 13.5, 6.8 Hz, 1H), 1.57 - 1.43 (m, 4H), 1.30 (td, J = 16.3, 14.2, 8.9 Hz, 2H).

¹³C NMR (101 MHz, D₂O) δ 177.74, 176.62, 173.11, 173.00, 170.77, 137.12, 137.00, 134.62, 134.47, 132.12, 131.92, 128.16, 69.51, 69.46, 69.38, 69.09, 68.92, 66.72, 56.52, 55.98, 40.27, 38.93, 38.16, 37.64, 35.98, 35.51, 33.84, 27.98.

### Lipoic acid derivative example 6:

This example provides a lipoic acid derivative (LA-PEG₄-2-BPA). The synthesis route is as follows:

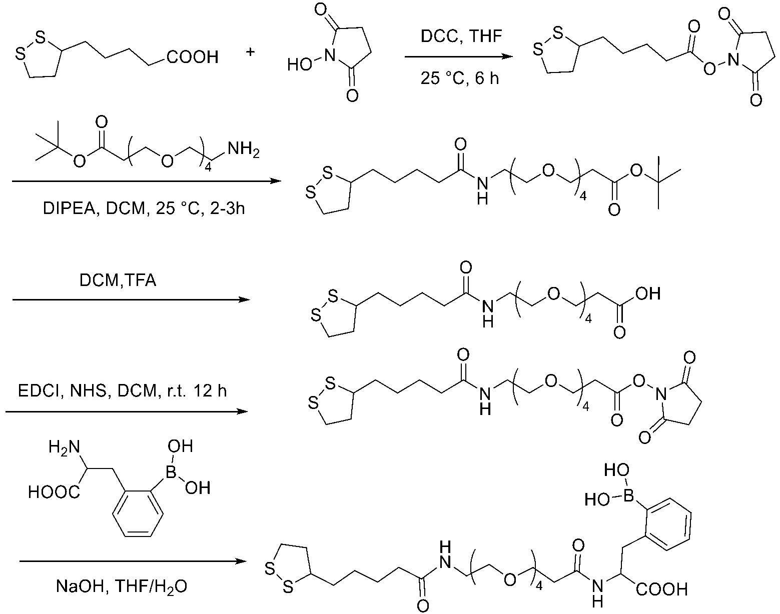

The synthesis steps of LA-PEG₄-2-BPA in this example refer to the lipoic acid derivative example 2, with the difference being that BPA is replaced with 2-BPA of this example in the final step.

¹H NMR (500 MHz, Chloroform-d) δ 9.86 (s, 1H), 8.06 (d, *J =* 11.9 Hz, 1H), 7.38 (t, *J* = 6.8 Hz, 1H), 7.36 - 7.23 (m, 4H), 5.80 (s, 2H), 4.42 (dt, *J* = 11.9, 7.0 Hz, 1H), 3.85 - 3.77 (m, 1H), 3.80 - 3.73 (m, 1H), 3.76 - 3.51 (m, 16H), 3.51 - 3.43 (m, 1H), 3.45 - 3.32 (m, 2H), 3.24 (dt, *J =* 12.4, 7.1 Hz, 1H), 3.20 - 3.10 (m, 2H), 3.07 (ddd, *J* = 12.4, 7.0, 0.9 Hz, 1H), 2.51 (t, *J* = 7.1 Hz, 2H), 2.51 - 2.42 (m, 1H), 2.29 (dq, *J* = 12.3, 7.0 Hz, 1H), 2.24 - 2.15 (m, 2H), 1.92 (dq, *J* = 12.4, 7.0 Hz, 1H), 1.74 (dq, *J* = 12.4, 7.1 Hz, 1H), 1.68 - 1.54 (m, 3H), 1.52 - 1.40 (m, 1H).

¹³C NMR (125 MHz, D₂O) δ 177.30, 174.07, 172.99, 141.32, 135.28, 133.23, 131.10, 128.61, 127.81, 127.80, 127.80, 70.74, 70.67, 70.59, 69.60, 69.45, 66.59, 58.30, 54.23, 41.28, 38.57, 37.78, 37.63, 37.25, 36.51, 34.13, 28.34, 25.32.

### Lipoic acid derivative example 7:

This example provides a lipoic acid derivative (LA-PEG₁-BPA, denoted as L^{p}B-1). The synthesis route is as follows:

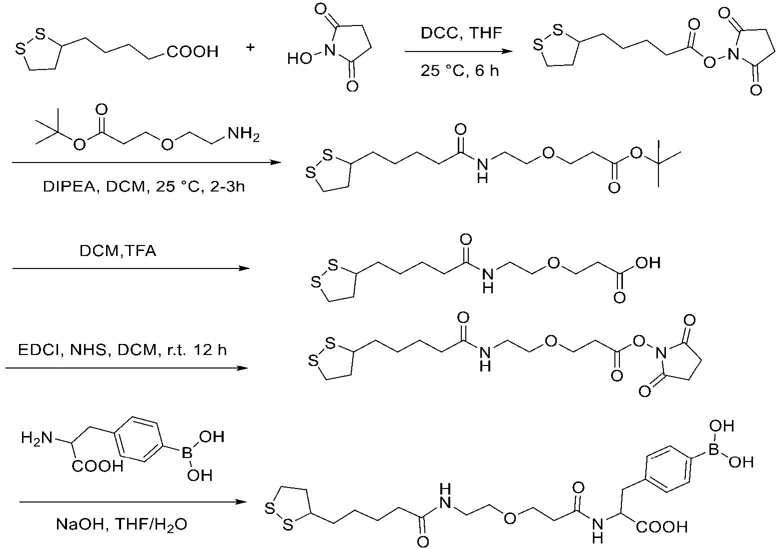

The synthesis steps of LA-PEG₁-BPA in this example refer to the lipoic acid derivative example 2, with the difference being that PEG₄ is replaced with PEG₁ in this example.

¹H NMR (400 MHz, D₂O) δ 8.49 (s, 1H), 7.55 - 7.47 (m, 2H), 7.21 - 7.11 (m, 2H), 4.49 (dd, J = 7.5, 5.0 Hz, 1H), 3.76 - 3.65 (m, 3H), 3.57 (t, J = 5.3 Hz, 2H), 3.42 - 3.06 (m, 6H), 2.98 (dd, J = 13.9, 7.6 Hz, 1H), 2.57 - 2.46 (m, 2H), 2.28 (t, J = 7.3 Hz, 2H), 2.01 (dq, J = 13.5, 6.9 Hz, 1H), 1.77 (dtd, J = 13.7, 8.0, 5.6 Hz, 1H), 1.70 - 1.57 (m, 3H), 1.43 (p, J = 7.4 Hz, 2H).

¹³C NMR (101 MHz, D₂O) δ 178.18, 176.95, 173.14, 171.07, 134.38, 131.27, 128.08, 68.66, 66.48, 66.44, 56.49, 56.28, 40.24, 38.93, 38.06, 37.44, 35.46, 33.67, 27.80, 25.00.

### Lipoic acid derivative example 8:

This example provides a lipoic acid derivative (LA-PEG₁-3-BPA, denoted as L^{m}B-1). The synthesis route is as follows:

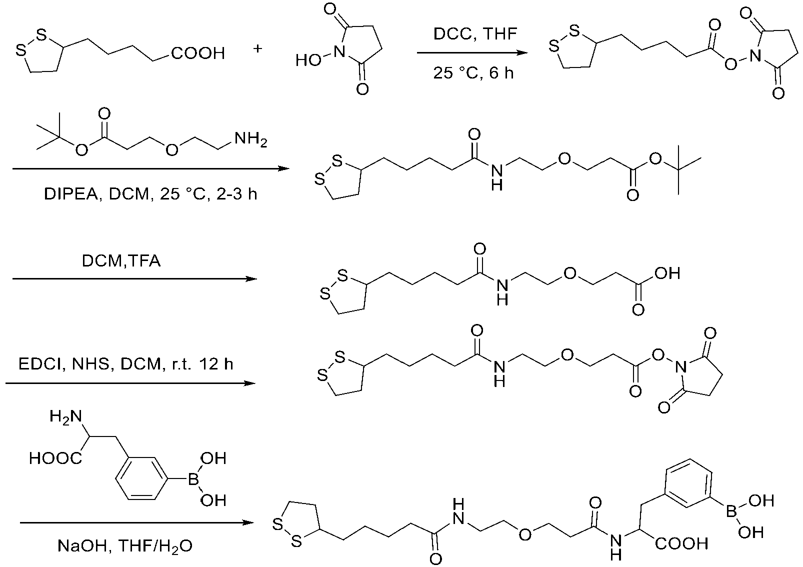

The synthesis steps of LA-PEG₁-3-BPA in this example refer to the lipoic acid derivative example 5, with the difference being that PEG₄ is replaced with PEG₁ in this example.

¹H NMR (400 MHz, D₂O) δ 7.46 - 7.35 (m, 2H), 7.21 (t, J = 7.4 Hz, 1H), 7.02 (d, J = 7.5 Hz, 1H), 4.47 (dd, J = 8.2, 5.0 Hz, 1H), 3.78 (td, J = 6.7, 1.6 Hz, 2H), 3.71 - 3.60 (m, 3H), 3.49 (t, J = 5.3 Hz, 2H), 3.17 (ddd, J = 27.7, 13.1, 5.5 Hz, 3H), 2.92 (dd, J = 14.0, 8.2 Hz, 1H), 2.42 (td, J = 6.7, 1.6 Hz, 2H), 2.23 (t, J = 7.2 Hz, 2H), 1.96 (dt, J = 13.7, 6.8 Hz, 1H), 1.73 (dq, J = 14.0, 7.5 Hz, 1H), 1.61 (dp, J = 12.6, 7.5 Hz, 4H), 1.39 (p, J = 7.6 Hz, 2H).

¹³C NMR (101 MHz, D₂O) δ 178.39, 176.99, 173.20, 171.05, 135.81, 132.10, 129.52, 127.22, 126.35, 68.63, 58.87, 56.47, 56.41, 40.23, 40.00, 38.93, 38.03, 37.95, 35.43, 33.66, 27.77, 24.99.

### Lipoic acid derivative example 9:

This example provides a lipoic acid derivative (LA-PEG₂-3-BPA, denoted as L^{m}B-2). The synthesis route is as follows:

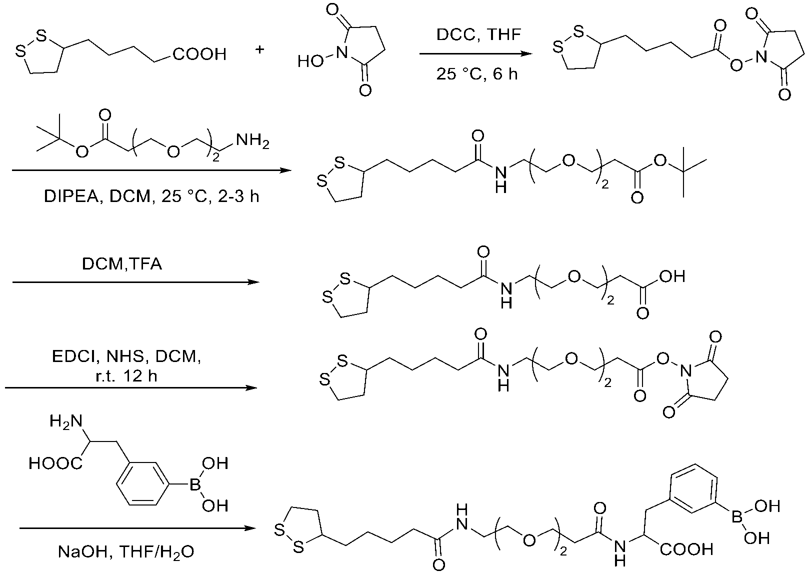

The synthesis steps of LA-PEG₂-3-BPA in this example refer to the lipoic acid derivative example 5, with the difference being that PEG₄ is replaced with PEG₂ in this example.

¹H NMR (400 MHz, D₂O) δ 7.41 - 7.31 (m, 2H), 7.17 (t, J = 7.4 Hz, 1H), 7.03 - 6.95 (m, 1H), 4.42 (dd, J = 8.3, 4.9 Hz, 1H), 3.66 - 3.60 (m, 3H), 3.57 - 3.45 (m, 6H), 3.36 - 3.27 (m, 2H), 3.23 - 3.05 (m, 3H), 2.87 (dd, J = 13.9, 8.4 Hz, 1H), 2.49 - 2.38 (m, 3H), 2.20 (t, J = 7.2 Hz, 2H), 1.93 (dq, J = 13.6, 6.9 Hz, 1H), 1.75 - 1.64 (m, 1H), 1.58 (dtt, J = 14.4, 10.7, 5.3 Hz, 3H), 1.36 (p, J = 7.6 Hz, 2H).

¹³C NMR (101 MHz, D₂O) δ 178.48, 177.00, 173.09, 171.05, 135.85, 132.04, 129.53, 127.23, 126.35, 69.43, 69.38, 68.85, 66.65, 56.49, 40.24, 38.91, 38.04, 37.96, 35.82, 35.43, 33.67, 27.76, 24.99.

### Lipoic acid derivative example 10:

This example provides a lipoic acid derivative (LA-PEG₈-3-BPA, denoted as L^{m}B-4). The synthesis route is as follows:

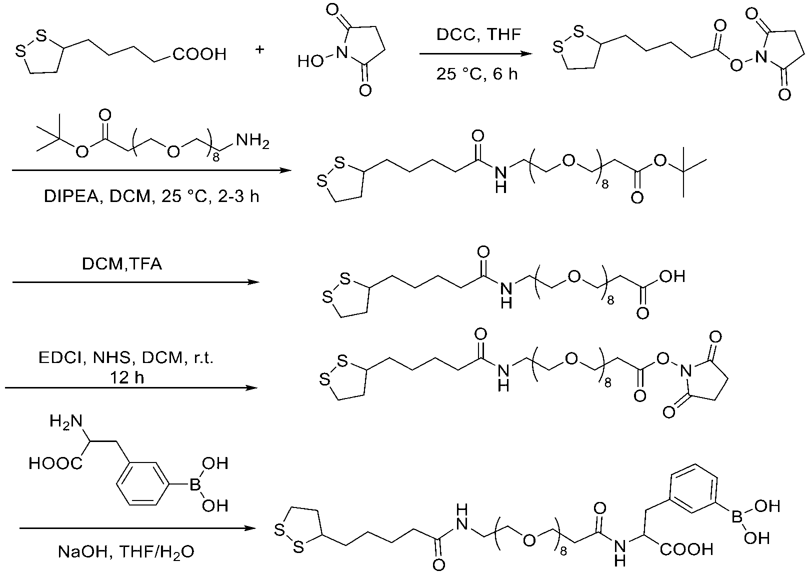

The synthesis steps of LA-PEG₈-3-BPA in this example refer to the lipoic acid derivative example 5, with the difference being that PEG₄ is replaced with PEG₈ in this example.

¹H NMR (400 MHz, D₂O) δ 7.49 - 7.41 (m, 2H), 7.26 (t, J = 7.4 Hz, 1H), 7.07 (dt, J = 7.5, 1.6 Hz, 1H), 4.50 (dd, J = 8.3, 5.0 Hz, 1H), 3.75 - 3.69 (m, 27H), 3.66 (t, J = 4.4 Hz, 4H), 3.61 (dt, J = 6.7, 2.6 Hz, 2H), 3.42 (t, J = 5.3 Hz, 2H), 3.32 - 3.13 (m, 3H), 2.96 (dd, J = 13.9, 8.3 Hz, 1H), 2.53 (tq, J = 8.2, 3.1, 2.0 Hz, 3H), 2.30 (t, J = 7.2 Hz, 2H), 2.02 (dq, J = 13.5, 6.9 Hz, 1H), 1.79 (dtd, J = 13.6, 7.9, 5.6 Hz, 1H), 1.73 - 1.60 (m, 3H), 1.46 (q, J = 7.7 Hz, 2H).

¹³C NMR (101 MHz, D₂O) δ 178.47, 176.92, 173.08, 135.85, 132.04, 129.54, 127.22, 126.36, 69.65, 69.59, 69.56, 69.51, 69.43, 69.39, 68.88, 66.66, 56.52, 56.50, 48.84, 45.32, 40.27, 38.90, 38.07, 37.98, 35.83, 35.47, 33.71, 27.82, 25.02.

### Lipoic acid derivative example 11:

This example provides a lipoic acid derivative (LA-3-BPA).

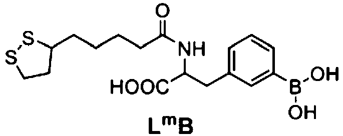

The synthetic steps of this example refer to the lipoic acid derivative example 1, with the difference being that BPA is replaced with 3-BPA of this example.

¹H NMR (400 MHz, D₂O) δ 7.34 (dt, J = 9.8, 1.8 Hz, 2H), 7.16 (td, J = 7.4, 1.4 Hz, 1H), 6.97 (dt, J = 7.6, 1.6 Hz, 1H), 4.39 (dd, J = 8.9, 4.8 Hz, 1H), 3.56 (dq, J = 8.5, 6.2 Hz, 1H), 3.12 (dddd, J = 20.8, 13.9, 10.4, 5.5 Hz, 3H), 2.82 (dd, J = 13.9, 8.9 Hz, 1H), 2.39 (dq, J = 12.2, 6.0 Hz, 1H), 2.12 (t, J = 7.3 Hz, 2H), 1.88 (dqd, J = 13.7, 6.9, 2.7 Hz, 1H), 1.60 (dtd, J = 13.7, 7.9, 5.9 Hz, 1H), 1.53 - 1.37 (m, 3H), 1.19 (qd, J = 8.1, 5.7 Hz, 2H).

¹³C NMR (101 MHz, D₂O) δ 178.79, 176.07, 176.06, 135.97, 131.91, 129.52, 127.28, 126.34, 56.45, 56.43, 56.39, 40.25, 38.05, 35.47, 33.68, 27.78, 24.90.

B in the present invention may represent ¹¹B or ¹⁰B, unless otherwise specified in the present invention. It is noteworthy that the preparation method of the natural boron-containing and boron-10-containing lipoic acid derivative in the present invention is substantially the same, with the difference being that the boron-containing starting materials used are natural boron and boron-10, respectively. The preparation methods of LA-PEG₁-¹⁰BPA, LA-PEG₂-¹⁰BPA, LA-PEG₄-¹⁰BPA, LA-PEG₈-¹⁰BPA, LA-PEG₁-3-¹⁰BPA, LA-PEG₂-3-¹⁰BPA, LA-PEG₄-3-¹⁰BPA, LA-PEG₈-3-¹⁰BPA, and other lipoic acid derivatives containing ¹⁰B are similar to those described in the above examples.

| Compound | Structural formula |
|---|---|
| LA-PEG₁-¹⁰BPA | 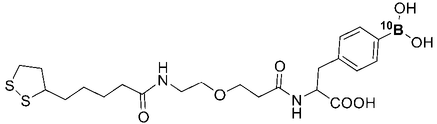 |
| LA-PEG₂-¹⁰BPA | 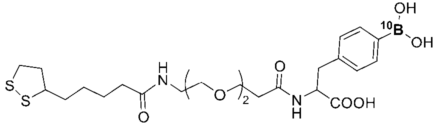 |
| LA-PEG₄-¹⁰BPA | 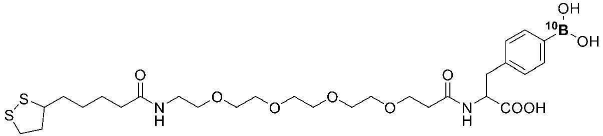 |
| LA-PEG₈-¹⁰BPA | 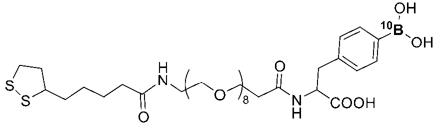 |
| LA-PEG₁-3-¹⁰BPA | 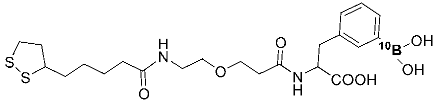 |
| LA-PEG₂-3-¹⁰BPA | 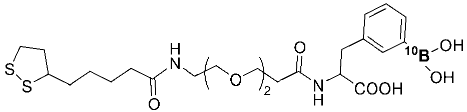 |
| LA-PEG₄-3-¹⁰BPA | 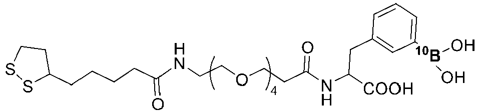 |
| LA-PEG₈-3-¹⁰BPA | 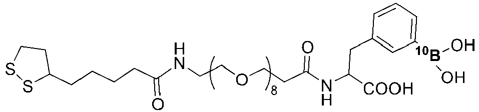 |
| LA-PEG₉-¹⁰BPA | 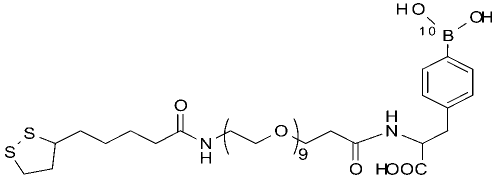 |
| LA-PEG₁₀-¹⁰BPA | 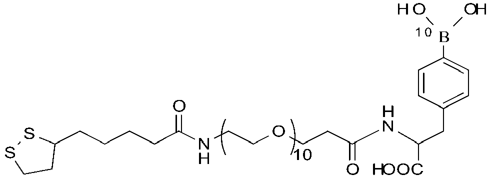 |

### Vesicle example 1

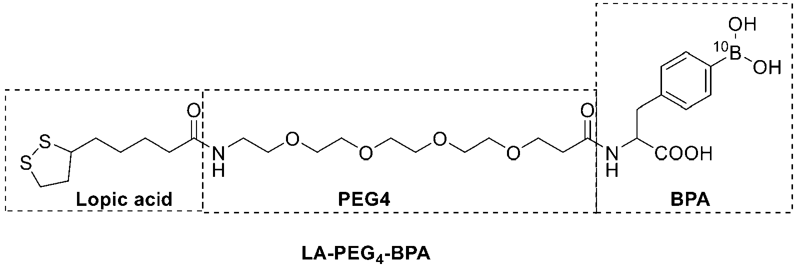

The lipoic acid derivative (LA-PEG₄-BPA, L^{P}B-3) prepared by the present invention was used for vesicle preparation:
3.4 mg of LA-PEG4-BPA was weighed and placed into a 2 mL EP tube, 1 mL of double-distilled water was added, followed by the addition of 10 µL of NaOH aqueous solution (2.5 M), with the pH between 10 and 11. After complete dissolution of LA-PEG4-BPA, 20 µL of HCl (1 M) was added, at which point the pH was around 7.0. The solution rapidly changed from a clear and transparent state to a white emulsion, accompanied by a distinct Tyndall effect, indicating the completion of vesicle self-assembly.

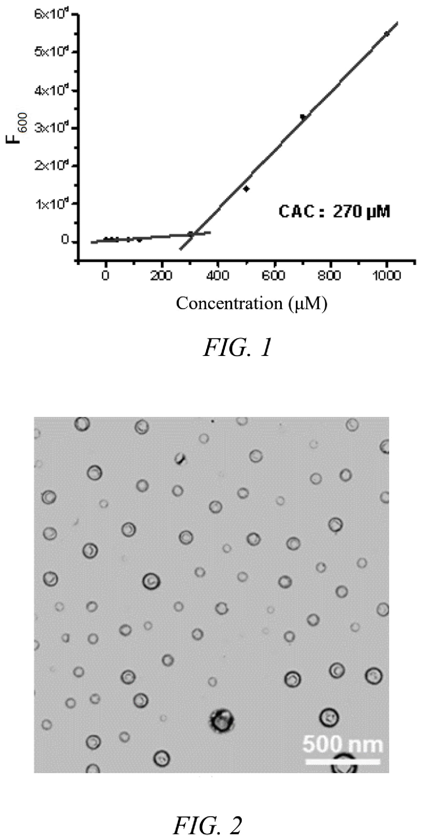

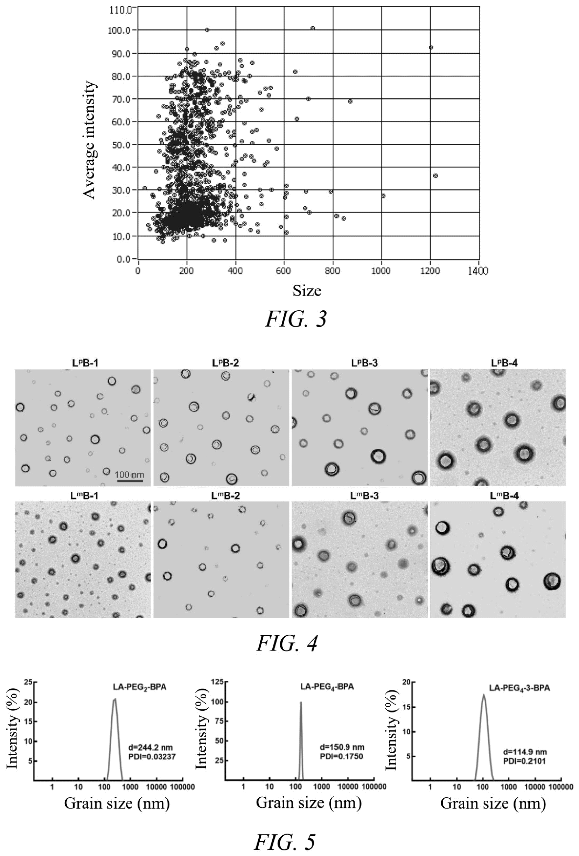

Refer to FIG. 1, the critical aggregation concentration was determined to be 270 uM by the rhodamine dye method. Refer to FIG. 2, the transmission electron microscopy detection results of the vesicle. The hydrated grain size of the vesicle was determined by DLS, and the hydrated grain size was about 150 nm. Refer to FIG. 3, the hydrated grain size diagram of the vesicle was determined by exosome grain size tracking analyser.

### Vesicle example 2

This example differs from the aforementioned vesicle example 1 in that the lipoic acid derivative (LA-PEG₂-BPA, L^{P}B-2) prepared in the aforementioned lipoic acid derivative example 3 was used for vesicle preparation in this example, while other conditions were substantially the same as those in vesicle example 1.

### Vesicle example 3

This example differs from the aforementioned vesicle example 1 in that the lipoic acid derivative (LA-PEG₄-3-BPA, L^{m}B-3) prepared in the aforementioned lipoic acid derivative example 5 was used for vesicle preparation in this example, while other conditions were substantially the same as those in vesicle example 1.

### Vesicle example 4

This example differs from the aforementioned vesicle example 1 in that the lipoic acid derivative (LA-PEG₈-BPA, L^{P}B-4) prepared in the aforementioned lipoic acid derivative example 4 was used for vesicle preparation in this example, while other conditions were substantially the same as those in vesicle example 1.

### Vesicle example 5

This example differs from the aforementioned vesicle example 1 in that the lipoic acid derivative (LA-PEG₁-BPA, L^{P}B-1) prepared in the aforementioned lipoic acid derivative example 7 was used for vesicle preparation in this example, while other conditions were substantially the same as those in vesicle example 1.

### Vesicle example 6

This example differs from the aforementioned vesicle example 1 in that the lipoic acid derivative (LA-PEG₁-3-BPA, L^{m}B-1) prepared in the aforementioned lipoic acid derivative example 8 was used for vesicle preparation in this example, while other conditions were substantially the same as those in vesicle example 1.

### Vesicle example 7

This example differs from the aforementioned vesicle example 1 in that the lipoic acid derivative (LA-PEG₂-3-BPA, L^{m}B-2) prepared in the aforementioned lipoic acid derivative example 9 was used for vesicle preparation in this example, while other conditions were substantially the same as those in vesicle example 1.

### Vesicle example 8

This example differs from the aforementioned vesicle example 1 in that the lipoic acid derivative (LA-PEG₈-3-BPA, L^{m}B-4) prepared in the aforementioned lipoic acid derivative example 10 was used for vesicle preparation in this example, while other conditions were substantially the same as those in vesicle example 1.

### Test example 1

In this test example, transmission electron microscopy test was performed on vesicles formed from L^{p}B-1, L^{p}B-2, L^{p}B-3, L^{p}B-4, L^{m}B-1, L^{m}B-2, L^{m}B-3, and L^{m}B-4 lipoic acid derivatives, respectively.

Preparation of the vesicle in this test example: An appropriate amount of the corresponding compound was weighed and placed into a sample vial, double-distilled water was added, an appropriate amount of NaOH aqueous solution was added to adjust the pH to between 10 and 11 until the starting material was completely dissolved, followed by addition of an appropriate amount of hydrochloric acid to adjust the pH to below 7.2, thereby completing vesicle assembly.

Representative transmission electron microscopy images of the vesicles are shown in FIG. 4. The transmission electron microscopy (TEM) images confirmed that the vesicle compound assembled from the lipoic acid derivative exhibited a spherical vesicular nanostructure with high monodispersity. Additionally, it could be observed that the grain size of the formed vesicles falls within the range of nano-drug grain sizes, which facilitated their use as nano-drugs.

### Test example 2

### (1) Grain size detection

① In this test example, the grain sizes of vesicle compounds formed by three compounds, LA-PEG₂-BPA, LA-PEG₄-BPA, and LA-PEG₄-3-BPA, were detected. The DLS was performed to measure the grain size, and the experimental results are shown in FIG. 5.

Preparation of the vesicle nanoparticles to be detected for grain size in this test example includes: An appropriate amount of the corresponding compound was weighed and placed into a sample vial, double-distilled water was added, an appropriate amount of NaOH aqueous solution was added to adjust the pH to between 10 and 11 until the starting material was completely dissolved, followed by addition of an appropriate amount of hydrochloric acid to adjust the pH to below 7.2, thereby completing vesicle assembly.

As shown in FIG. 5: the grain size of the vesicles obtained from LA-PEG₂-BPA was approximately 244 nm, the grain size of the vesicles obtained from LA-PEG₄-BPA was approximately 150 nm, and the grain size of the vesicles obtained from LA-PEG₄-3-BPA was approximately 114 nm.

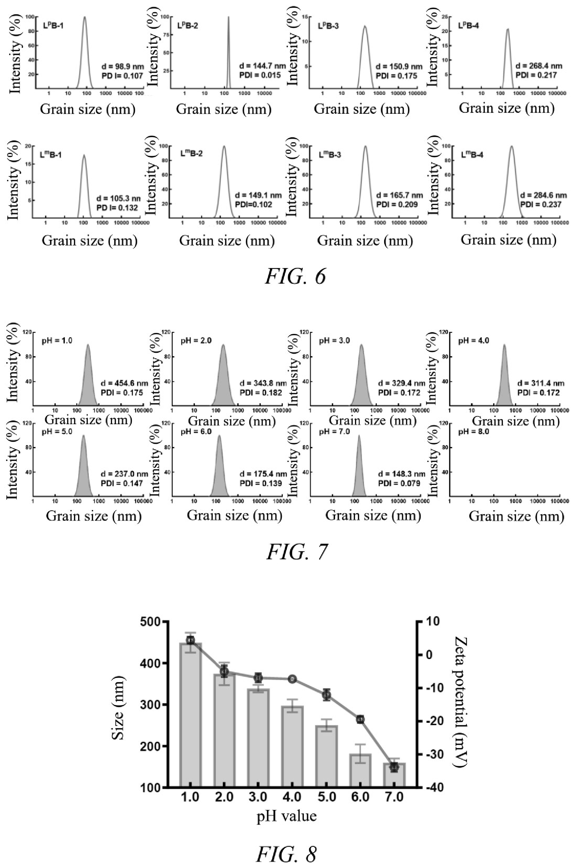

② The lipoic acid derivatives were separately assembled into vesicles, including LA-PEG₁-BPA (denoted as L^{p}B-1), LA-PEG₂-BPA (denoted as L^{p}B-2), LA-PEG₄-BPA (denoted as L^{p}B-3), LA-PEG₈-BPA (denoted as L^{p}B-4), LA-PEG₁-3-BPA (denoted as L^{m}B-1), LA-PEG₂-3-BPA (denoted as L^{m}B-2), LA-PEG₄-3-BPA (denoted as L^{m}B-3), and LA-PEG₈-3-BPA (denoted as L^{m}B-4); the vesicle assembly method in this test example was based on the preparation method of the vesicle examples and depolymerization and assembly were repeatedly three times; the physicochemical properties of the self-assembled vesicles were detected, and the grain size, polymer dispersancy index (PDI), and zeta potential of the vesicles were detected using DLS (dynamic light scattering) technology. The detection results are shown in Table 1 and FIG. 6 below.

**Table 1**

| Item | L^{p}B-1 | L^{p}B-2 | L^{p}B-3 | L^{p}B-4 | L^{m}B-1 | L^{m}B-2 | L^{m}B-3 | L^{m}B-4 |
|---|---|---|---|---|---|---|---|---|
| d(nm) | 98.9 | 144.7 | 150.9 | 268.4 | 105.3 | 149.1 | 165.7 | 284.6 |
| PDI | 0.107 | 0.015 | 0.175 | 0.217 | 0.132 | 0.102 | 0.209 | 0.237 |
| zeta potential (mV) | -32.5 | -30.9 | -35.2 | -29.35 | -30.7 | -28.61 | -35.20 | -35.2 |

As shown in Table 1 and FIG. 6, the lipoic acid derivative readily self-assembles into nanoparticles, with all samples exhibiting negative zeta potential values and polymer dispersancy indices below 0.3. For vesicles formed through repeated self-assembly of the lipoic acid derivative, the grain size distribution shifts toward larger average sizes as the PEG chain length increases. Moreover, the vesicle nanoparticles have a spherical core-shell nanostructure with high monodispersity.

In summary, based on the grain size detection results, the grain size range of the vesicles assembled from the lipoic acid derivative of the present invention falls within the grain size range of nano-drugs, and the vesicles exhibit a significant size effect, and can be effectively utilized in the field of drugs.

### (2) Detection of the critical aggregation concentration of vesicles assembled from lipoic acid derivatives.

A mixture of Nile Red (at a concentration of 1.0 µM) and the corresponding BPA lipoic acid derivative (at a concentration of 0-20 mM) in distilled water was oscillated overnight under constant temperature conditions. The fluorescence emission intensity of Nile Red at 525 nm wavelength (λex = 485 nm) was measured using a FluoroMax-4 fluorometer. The critical aggregation concentration of the BPA lipoic acid derivative in aqueous medium was calculated from the intersection point of two linear regression equations.

The critical aggregation concentration (CAC) refers to the minimum concentration at which a substance forms aggregates in an aqueous solution. When the concentration of a surfactant or colloidal particle in an aqueous solution exceeds the critical aggregation concentration, they begin to self-assemble into aggregates due to intermolecular attractive forces. This process is referred to as aggregation or self-assembly.

The results of the critical aggregation concentration detection in this test example are shown in Table 2 below.

**Table 2**

| Item | L^{p}B-1 | L^{p}B-2 | L^{p}B-3 | L^{p}B-4 | L^{m}B-1 | L^{m}B-2 | L^{m}B-3 | L^{m}B-4 |
|---|---|---|---|---|---|---|---|---|
| CAC(uM) | 243 | 248 | 270 | 277 | 256 | 254 | 274 | 280 |

### (3) Study on pH conditions for assembly of lipoic acid derivatives into vesicles.

1) When the lipoic acid derivative assembled into vesicles, the pH value of the solution was adjusted to 1.0, 2.0, 3.0, 4.0, 5.0, 6.0, 7.0, and 8.0 using 1 M HCl, respectively, for vesicle assembly. The DLS grain size, polymer dispersancy index (PDI), and zeta potential of the vesicle nanoparticles (LA-PEG₄-BPA vesicles) under different pH conditions were detected, and the detection results are shown in Table 3, FIG. 7 and FIG. 8 below.

**Table 3**

| pH | 1.0 | 2.0 | 3.0 | 4.0 | 5.0 | 6.0 | 7.0 | 8.0 |
|---|---|---|---|---|---|---|---|---|
| d(nm) | 454.6 | 343.8 | 329.4 | 311.4 | 237.0 | 175.4 | 148.4 | / |
| PDI | 0.175 | 0.182 | 0.172 | 0.172 | 0.147 | 0.139 | 0.079 | / |

According to the results, it can be seen that the grain size of vesicles prepared by self-assembly of the lipoic acid derivative prepared in the present invention increases with decreasing pH within the range of pH 1 to 7; when the pH is 8.0, vesicle nanoparticles cannot be formed.

The grain size of the vesicles decreases with increasing pH levels, while the zeta potential of the vesicles increases with increasing pH levels.

2) Vesicle assembly under different acid conditions. Common acids can catalyse the formation of vesicle nanoparticles, including but not limited to hydrochloric acid, formic acid, acetic acid, trifluoroacetic acid, sulfuric acid, and nitric acid.

Under conditions of formic acid, acetic acid, trifluoroacetic acid, and hydrochloric acid (HCl) respectively, the pH of the LA-PEG₄-BPA solution was adjusted to 6.8, and the LA-PEG₄-BPA monomer could self-assemble into vesicles. The DLS grain sizes of the vesicles were approximately 148.3 nm, 152.1 nm, 153.7 nm, and 150.9 nm, respectively, and the PDI values of the vesicles were 0.104, 0.168, 0.153, and 0.175, respectively.

It can be seen that the lipoic acid derivative can effectively self-assemble into vesicles under different acid conditions, and the size of the vesicles is also very uniform.

### (4) Study on the reversible assembly of LA-PEG₄-BPA (denoted as L^{p}B-3) and its vesicles.

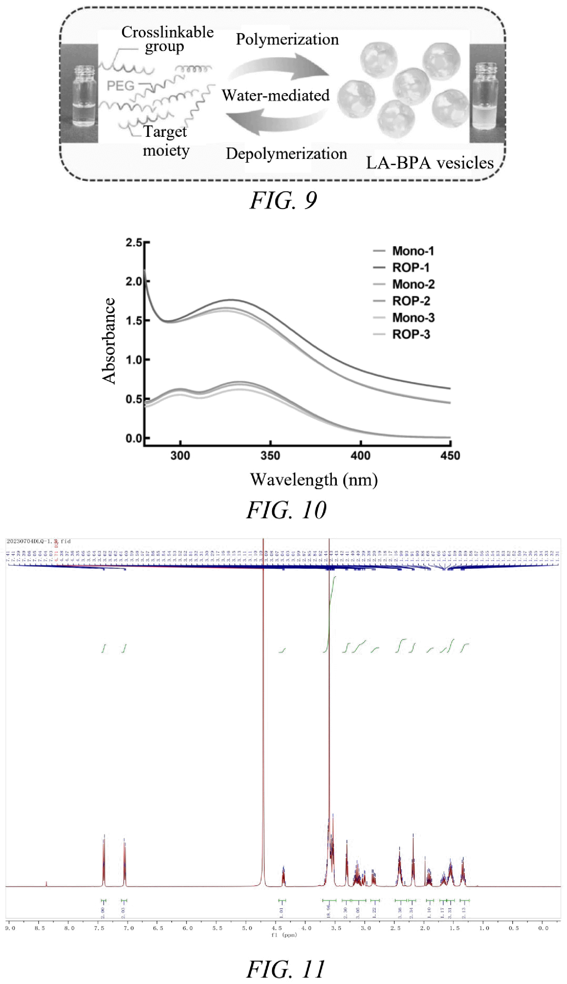

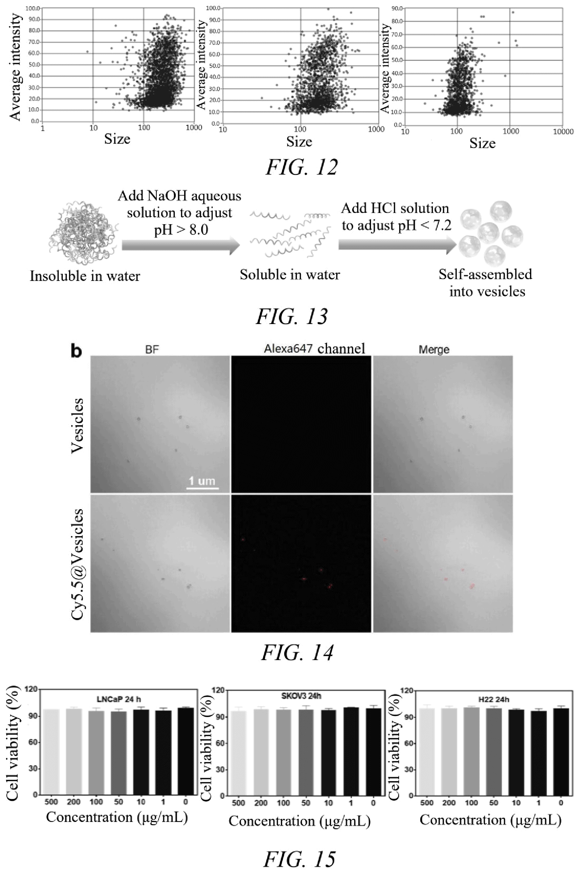

1) ① Alternately adding base (NaOH aqueous solution) and acid (HCl solution) to the reaction system enables reversible assembly of the vesicles. Throughout the process, as the pH increased from 1.0 to 8.5, the solution gradually changed from white turbidity to clear (see the left reaction vessel in FIG. 9), indicating vesicle depolymerization. Then, hydrochloric acid was added dropwise to the above solution for acidification. When the solution pH dropped below 7.2, the clear solution turned into a white suspension (see the right reaction vessel in FIG. 9), indicating vesicle formation. This result indicates that the morphological changes of vesicles can be repeatedly reversed through the polymerization-depolymerization cycle.
   ② The lipoic acid derivative LA-PEG₄-BPA was placed in water, and the pH was adjusted to greater than 8 to dissolve the lipoic acid derivative; the pH was readjusted to within 7.2, and the lipoic acid derivative self-assembled to form vesicles, as shown in FIG. 13.
2) The LA-PEG₄-BPA monomer and its vesicles can undergo multiple assembly and depolymerization by pH adjustment, and the multiple assembly and depolymerization do not affect their properties.

By alternately introducing NaOH aqueous solution and HCl in a water-mediated system, the aim is to create reversible compound vesicles. L^{p}B-3 underwent three additional cycles of polymerization and depolymerization, with the cycles of depolymerization into monomers and reassembly into vesicles continuously monitored by UV-Vis spectroscopy. The ultraviolet absorbance values were recorded after each polymerization and depolymerization during the experimental process.

The ultraviolet absorption value change diagram is shown in FIG. 10: when assembled into vesicles, there is an ultraviolet absorption curve (blue curve, ROP), and when depolymerized into monomers, there is an ultraviolet absorption curve (yellow curve, Mono). As reassembly occurs again, the ultraviolet absorption curve changes to the blue curve, and as depolymerization occurs again, the ultraviolet absorption changes to the yellow curve. In FIG. 10, the curves from top to bottom are ROP-1, ROP-2, ROP-3, Mono-1, Mono-2, and Mono-3, respectively. Among them, the ROP-1, ROP-2, and ROP-3 curves are essentially identical, while the Mono-1, Mono-2, and Mono-3 curves are essentially identical. It can be seen that the nanoparticles can undergo multiple assembly and depolymerization by pH adjustment without affecting their properties. During vesicle depolymerization, an absorption band was observed at 330 nm, which was characteristic of the L^{p}B-3 monomer. This indicates an ultrafast transition between the polymerized and depolymerized states. The vesicles underwent base-induced depolymerization without any impact on the molecular structure, and it was found that this polymerization-depolymerization cycle is reversible under various conditions. The recovered depolymerized monomers were identified by nuclear magnetic resonance as LA-PEG₄-BPA (L^{p}B-3) monomers, as shown in the nuclear magnetic resonance spectrum in FIG. 11.

Meanwhile, the changes in nanograin size during the depolymerization into monomers and reassembly into vesicles cycle were studied. As shown in FIG. 12, the grain size distribution diagram of vesicles after different cycles of assembly measured by nanoparticle tracking analyser is presented.

FIG. 12 shows that with multiple assemblies, the grain size of vesicles after multiple assemblies tends to be distributed around 100 nm, with higher uniformity (see the trend of grain size distribution from left to right in FIG. 12 for details). This result indicates that repeated assembly can form smaller vesicles with a narrower and more uniform grain size distribution.

In summary, the nanoparticles achieve efficient assembly under acid addition condition and rapid depolymerization into monomers under base addition condition; the assembly and depolymerization processes can be cycled multiple times, and repeated assembly processes can make the vesicle grain size more uniform.

### Test example 3

Fluorescent labelling (Fluorescent probe@Vesicles) was performed on the vesicles prepared from LA-PEG₄-BPA (the product prepared in vesicle example 1).

To the aqueous solution of vesicles (5 mL) at a concentration of 1 mg/mL, 5 µL (1 mM) of Cy5.5 fluorescent dye was added, mixed well by pipetting, and dialyzed for 12 h, followed by imaging using a confocal microscope. As shown in FIG. 14, vesicles loaded with the Cy5.5 fluorescent probe exhibited distinct vesicle-like structures detectable in the bright field (BF), and red fluorescence signals were detected in the fluorescence channel (Alexa647 Tunnel). The Merge imaging demonstrated complete overlap between the bright field and fluorescence channel, confirming that vesicles can indeed be labelled by fluorescent probes such as Cy5.5; no obvious fluorescence signal was detected in the fluorescence channel of the experimental group not labelled with the Cy5.5 fluorescent probe. BF is the bright field image. Merge refers to combining images from different colour channels. As can be seen from the imaging diagram of vesicles loaded with fluorescent materials, the vesicle morphology after labelled with free small-molecule probes shows no significant changes, and the fluorescent materials are adsorbed onto the vesicle surface. Evidently, the present invention enables monitoring of cell uptake and in vivo distribution in animals of vesicles by loading the fluorescent probe Cy5.5.

### Test example 4

### In vitro safety evaluation

Human prostate cancer LNCaP cells were seeded in a 96-well cell culture plate (approximately 10⁵ cells/well) and incubated for 24 h for adherence; the original culture medium was discarded, 150 µL of LA-PEG₄-BPA assembled vesicles (the product prepared in vesicle example 1) with different concentration gradients was added, returned to the cell culture incubator, and continued to culture for 24 h; the original culture medium was aspirated, washed with PBS 3 times, 100 µL of CCK8 working solution (1 mL of CCK8 reagent added to 9 mL of cell culture medium) was added, the well plate was transferred to the cell culture incubator and incubated for 60 min, the absorbance value was measured at 450 nm wavelength, the relative cell viability was calculated, the change trend diagram of cell proliferation activity as a function of increasing drug concentration was plotted (see FIG. 15), and the in vitro safety of the vesicles was evaluated; the same method was used to examine the effects on the cell viability of human ovarian cancer cells (SKOV3) and mouse hepatocellular carcinoma cells (H22). The CCK8 detection results indicate that after treatment with vesicles prepared from LA-PEG₄-BPA for 24 h, the cell viability of all three cell types remained above 90% compared to normally cultured cells, demonstrating that this vesicle exhibits favourable biosafety.

### Test example 5

### PANC-1 cell uptake assay in PANC-1 cell line

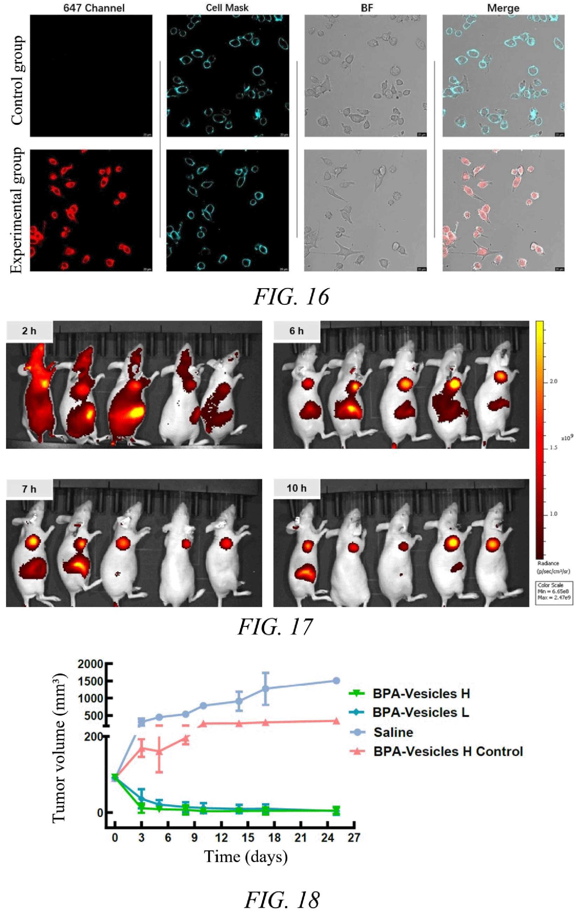

PANC-1 cells were seeded in 6-well plates at a density of 3 × 10⁷ cells per well and incubated for 24 h for adherence; the original culture medium was discarded, with the experimental group receiving 1.5 mL (1 mg/mL) of Cy5.5-loaded LA-PEG₄-BPA vesicle working solution, and the control group receiving the same dose of free Cy5.5 working solution; the plate was placed in a cell culture incubator and incubated for an additional 2 h; the cells were subjected to digestion and detachment, collected, centrifuged to discard the supernatant, washed three times with PBS as the solvent by centrifugation, and the cell membrane was labelled with Cell Mask. The experimental results were detected by imaging using a confocal microscope, as shown in FIG. 16. The cell membranes of the experimental group and the control group were labelled with Cell Mask to display blue fluorescence signal. In the fluorescence channel (Alex647 channel), distinct red fluorescence signals were detected intracellularly in the vesicle experimental group, and all were within the cell (as evidenced by the Merge fluorescence results, where the Cy5.5-loaded vesicles indicated by red fluorescence were within the cell membrane shown by blue fluorescence), demonstrating intracellular uptake of the vesicle compound by the experimental group; no significant intracellular fluorescence signal was detected in the control group with the addition of free Cy5.5 probe. BF is the bright field image. Merge refers to combining images from different colour channels. It can be seen that the vesicle compound of the present invention can be taken up by cells.

### Test example 6

(1) Mouse animal model: A subcutaneous pancreatic cancer model was used to evaluate the role of vesicles in tumour therapy. Nude mice were inoculated with subcutaneous xenografts of PANC-1 cells (pancreatic cancer cells) (approximately 1 × 10^⁶ cells per mouse), which were suspended in a solution of PBS and Matrigel (PBS : Matrigel = 1 : 1, V/V). When the tumour volume reached approximately 120 mm³, the nude mice were randomly grouped. Tumour volume (mm³) was calculated according to the following formula: Tumour volume = 0.5 × length × width².

Vesicle preparation: An appropriate amount of the corresponding lipoic acid derivative was weighed and placed into a sample vial, double-distilled water was added, an appropriate amount of NaOH aqueous solution was added to adjust the pH to approximately 10-11, mixed well, and an appropriate amount of hydrochloric acid was added to adjust the pH to approximately 7.0, thereby completing vesicle assembly.
1) In vivo distribution detection in tumour-bearing nude mice: 5 tumour-bearing (PANC-1) nude mice were respectively injected via the tail vein with Cy5.5 fluorescent probe-loaded LA-PEG₄-BPA vesicles (i.e., vesicles prepared using the lipoic acid derivative LA-PEG₄-BPA as the starting material, the same applies below) (10 mg/kg), and the in vivo distribution of the vesicles in the mice is shown in FIG. 17.

FIG. 17 shows the in vivo distribution of mice at 2 h, 6 h, 7 h, and 10 h after tail vein injection. Among them, the fluorescence signal intensity at the tumour site was very high at 7 h after tail vein injection, it can be seen that the LA-PEG₄-BPA vesicles can achieve high tumour enrichment after approximately 7 h of in vivo circulation.

### 2) BNCT efficacy evaluation

Approximately 6-8 h prior to neutron irradiation, the drug or physiological saline was injected into the tumour-bearing mouse via tail vein. The types and doses of injected drugs are detailed in the experimental groups below. Prior to irradiation, the experimental mice were anesthetized by intraperitoneal injection of sodium pentobarbital. After deep anesthesia, the mice were fixed in the corresponding animal neutron irradiation fixator, and the holder was installed at the irradiation window for neutron irradiation for approximately 9-14 minutes. After the irradiation experiment was completed, the mice were returned to their respective cages according to the grouping for continued housing upon regaining consciousness.

During the treatment period, the tumour volume and body weight of PANC-1 tumour-bearing mice were measured every 1-3 days to monitor changes in tumour volume and body weight of the experimental mice. The survival time of experimental mice in each group was recorded simultaneously.

Approximately 2 weeks after neutron irradiation, tissue samples were collected from the animals for efficacy evaluation. The experimental animals were euthanized under anesthesia, and major organs (heart, liver, spleen, lung, kidney, tumour) were harvested. After dehydration treatment, paraffin embedding was performed, followed by sectioning and H&E (hematoxylin & eosin) staining. Finally, the experimental results were observed under an optical microscope and recorded.

2.1) The experimental groups were as follows: high-dose (LA-PEG₄-¹⁰BPA vesicle 350 mg/kg) treatment group, low-dose treatment group (LA-PEG₄-¹⁰BPA vesicle 250 mg/kg), high-dose control group (LA-PEG₄-¹⁰BPA vesicle 350 mg/kg), and physiological saline group. The treatment group underwent neutron irradiation, the high-dose control group did not undergo neutron irradiation, and the physiological saline group was injected with the same volume of physiological saline only. Referring to Table 4, the radiation dose of neutron irradiation for nude mice in the high-dose group was approximately 1.5 Gy; the radiation dose of neutron irradiation for mice in the low-dose group was approximately 1.24 Gy.

The statistics of BNCT radiation dose in PANC-1 subcutaneous tumour model mice are shown in Table 4.

**Table 4. Statistics of BNCT radiation dose of BPA-Vesicles in subcutaneous tumour (PANC-1)**

| Item | Boron concentration (ppm) | Average dose rate (Gy/min) | Maximum dose rate (Gy/min) | Minimum dose rate (Gy/min) | Average dose (Gy) | Maximu m dose (Gy) | Minimum dose (Gy) |
|---|---|---|---|---|---|---|---|
| 1# High dose | 22.48 | 0.224 | 0.227 | 0.218 | 1.500 | 1.524 | 1.460 |
| 2# Low dose | 15.06 | 0.183 | 0.185 | 0.177 | 1.224 | 1.243 | 1.190 |

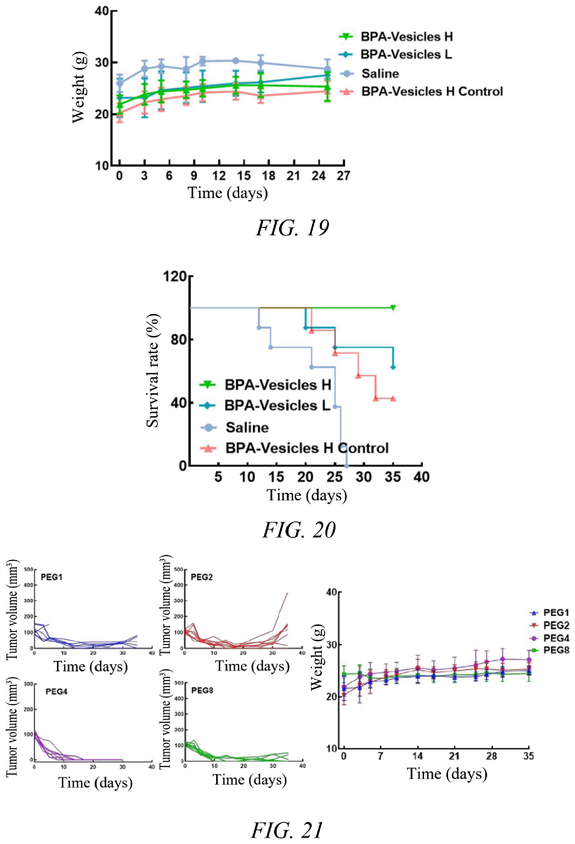

After neutron irradiation, the statistical trend of tumour volume growth in mice (as shown in FIG. 18) indicated that: the tumours of mice in the high-dose treatment group (BPA-Vesicles H) and the low-dose treatment group (BPA-Vesicles L) shrank or even disappeared within 5 days; The tumour volume in the high-dose control group (BPA-Vesicles H Control) showed a slow increasing trend over time; whereas the tumour volume of the animals in the physiological saline control group (Saline) showed a significant increasing trend over time. The experimental results demonstrate that the vesicle compound (BPA-Vesicles) of the present invention is an excellent BNCT candidate drug. The body weight statistical results of mice in each experimental group (as shown in FIG. 19) indicate that the body weights of animals in all experimental groups remained within the normal range during the experiment, demonstrating that the tumour treatment method of the present invention exhibits no significant biological toxic side effect. As shown in FIG. 20, the survival time statistical results indicate that the survival time of the physiological saline control group was within 30 days, the median survival time of the high-dose drug control group exceeded 25 days, and the median survival time of both high-dose/low-dose treatment groups was greater than 35 days. The experimental results indicate that the nano-drug of the present invention can effectively improve the survival time of tumour-bearing mice when used for BNCT therapy.

(2) Vesicle preparation: 2 mg of the corresponding compound was weighed and placed into a 5 mL sample vial, 1 mL of double-distilled water was added, an appropriate amount of 2.5 M NaOH aqueous solution was added to adjust the pH to approximately 10-11, mixed well, followed by an appropriate amount of 1 M hydrochloric acid was added to adjust the pH to approximately 7.0, thereby completing vesicle assembly.

The experimental groups were as follows: PEG1 vesicle group (including LA-PEG₁-¹⁰BPA vesicle test example and LA-PEG₁-3-¹⁰BPA vesicle test example), PEG2 vesicle group (including LA-PEG₂-¹⁰BPA vesicle test example and LA-PEG₂-3-¹⁰BPA vesicle test example), PEG4 vesicle group (including LA-PEG₄-¹⁰BPA vesicle test example and LA-PEG₄-3-¹⁰BPA vesicle test example), PEG8 vesicle group (including LA-PEG₈-¹⁰BPA vesicle test example and LA-PEG₈-3-¹⁰BPA vesicle test example), with approximately 8 mice per group, and the vesicle drug dose was 350 mg/kg for all groups.

Experimental method: Female Balb/c nude mice (weighing approximately 20 g) were ordered from GemPharmatech and housed in the animal facility for one week to acclimate to the environment. Prior to tumour cell inoculation, the mouse skin was disinfected by wiping with 75% ethanol, and approximately 1 million PANC-1 cells were subcutaneously inoculated per mouse. When the tumour volume reached approximately 150-200 mm³, BNCT treatment was initiated. At 6-7 h before neutron irradiation, drug injection was performed via tail vein administration at a dose of 350 mg/kg. Approximately 6 h after systemic circulation, sodium amobarbital was intraperitoneally injected for anesthesia. Upon achieving deep anesthesia, the mice were immobilized for neutron irradiation. The radiation dose of neutron irradiation was about 1.5 Gy.

Changes in tumour and body weight of mice after neutron irradiation in the four vesicle groups PEG1, PEG2, PEG4, and PEG8 are shown in FIG. 21. FIG. 21 shows the tumour volume and body weight changes in each group of mice in different drug groups after BNCT treatment. It can be seen that the tumour volume of mice in all four drug groups showed a reduction after irradiation therapy. Among them, the PEG2 group exhibited a trend of tumour recurrence in some mice. The body weights of tumour-bearing mice of the four groups after BNCT treatment showed a slow increase over 35 days.

### Test example 7

### ① In vitro BNCT efficacy evaluation

The experimental groups were as follows:
Control group: Physiological saline (i.e., B-10 concentration of 0), without neutron irradiation.
Neutron irradiation group: Physiological saline (i.e., B-10 concentration of 0), with neutron irradiation.
Vesicles control group: Vesicles with B-10 concentration of 1000 µg/mL, without neutron irradiation.
BNCT Group L: Vesicles with a B-10 concentration of 500 µg/mL, with neutron irradiation.
BNCT Group H: Vesicles with a B-10 concentration of 1000 µg/mL, with neutron irradiation.

The vesicle used in this test example was LA-PEG4-¹⁰BPA vesicle. Vesicle preparation: The corresponding lipoic acid derivative was weighed and placed into a sample vial, double-distilled water was added, an appropriate amount of 2.5 M NaOH aqueous solution was added to adjust the pH to approximately 10-11, mixed well, followed by an appropriate amount of 1 M hydrochloric acid was added to adjust the pH to approximately 7.0, thereby completing vesicle assembly.

Experimental method: 5 × 10⁶ PANC-1 cells were seeded in a 10 cm culture dish and cultured in a cell culture incubator for 24 h, treated with vesicles (LA-PEG₄-¹⁰BPA) at different concentrations (0, 500, 1000 µg/mL) for 6 h, washed three times with PBS to remove excess vesicles, digested with trypsin for 5 min to collect the cells, which were finally suspended in 150 µL culture medium and transferred to a 600 µL EP tube. Neutron irradiation was performed for approximately 10 min. Subsequently, the cells were subcultured for in vitro efficacy evaluation.

Evaluation of cell efficacy after BNCT treatment: Apoptotic cells were detected using the TUNEL method, and the apoptosis of cells was monitored by flow cytometry. Meanwhile, for the calcein-AM/PI (calcein-AM/PI) assay, the calcein-AM/PI stock solution was prepared proportionally to achieve final working concentrations of 2 µM calcein-AM and 4.5 µM PI, and cell apoptosis was observed via a confocal fluorescence microscope.

Monitoring of DNA double-strand break: Cells in each experimental group were washed three times with PBS, fixed, subjected to membrane perforation and blocking, treated with γ-H2AX antibody at 37°C for 2 h, washed three times with PBS, then incubated with fluorescent secondary antibody for 1 h, washed with PBS, and observed using a Zeiss 880 laser confocal microscope, and corresponding data was recorded.

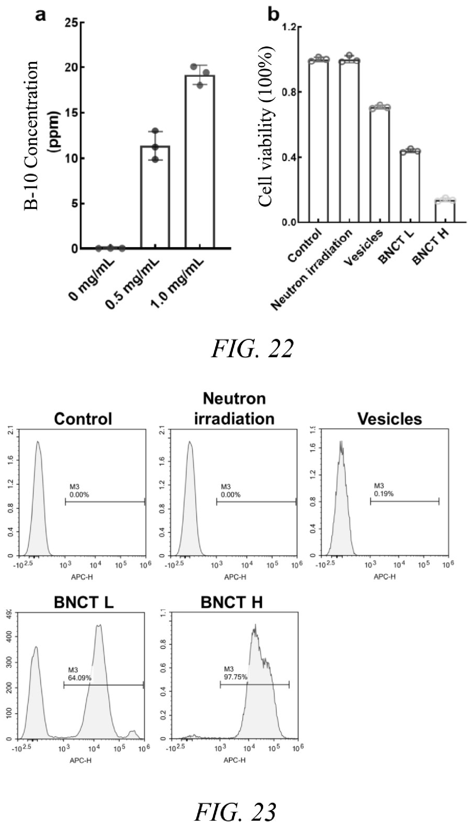

The experimental results were recorded to obtain FIGs. 22, 23, 24, and 25.

Panel a of FIG. 22 shows the intracellular B-10 concentration in the drug group detected by ICP-MS, where the intracellular B-10 concentration increased as a function of the administration concentration of vesicles. Panel b shows the cell viability of different experimental groups, indicating that the cell viability is negatively correlated with the vesicle concentration, and the tumour cells in the experimental group with the highest vesicle concentration (BNCT Group H) exhibited the lowest cell viability.

FIG. 23 shows the results of cell apoptosis detected by the TUNEL method in different experimental groups, clearly demonstrating that the BNCT Group H exhibited the most significant tumour cell apoptosis.

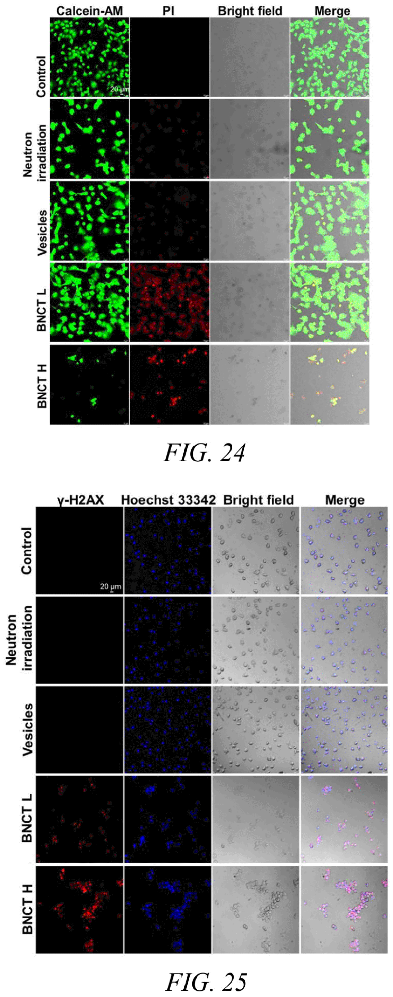

FIG. 24 shows the results of live/dead cell staining (calcein-AM/PI assay) imaging of cells in different experimental groups after corresponding treatments. Clearly, the BNCT Group L and BNCT Group H exhibited significantly increased dead cells, with dead cells (red fluorescence) outnumbering live cells (green fluorescence).

FIG. 25 shows the γ-H2AX staining imaging results of different experimental groups, indicating the results of DNA double-strand breaks in cells of different experimental groups. Among them, the cells in the experimental group with the highest administration dose, namely the BNCT Group H, exhibited the most γ-H2AX signals, indicating the highest level of DNA double-strand breaks, followed by the BNCT Experimental Group L. The cells in other three groups exhibited no significant DNA double-strand break phenomena. Similar to the results of cell viability shown in FIG. 22b, the tumour cells in the BNCT Group H exhibited the lowest cell viability.

In summary, based on FIGs. 22 to 25, the vesicle nano-drug of the present invention can serve as a BNCT candidate drug that effectively kills target tumour cells after neutron irradiation.

### Test example 8 in vivo distribution of vesicles

PANC-1 tumour-bearing mouse modelling: Female Balb/c nude mice (weighing approximately 20 g) were ordered from GemPharmatech and housed in the animal facility for one week to acclimate to the environment. Prior to tumour cell inoculation, the mouse skin was disinfected by wiping with 75% ethanol, and approximately 1 million PANC-1 cells were subcutaneously inoculated per mouse. The tumours were allowed to grow to a volume of approximately 150-200 mm³.

### ① Fluorescence distribution of vesicles in targeted tumour

Method: The tumour-bearing mice were randomly divided into two groups, with one group receiving tail vein injection of fluorescent probe-labelled vesicle nanoparticles (10 mg/kg) (n = 3/group), and the other group receiving tail vein injection of an equivalent amount of free fluorescent probe. This experiment employed fluorescent probe-labelled LA-PEG₄-BPA vesicles.

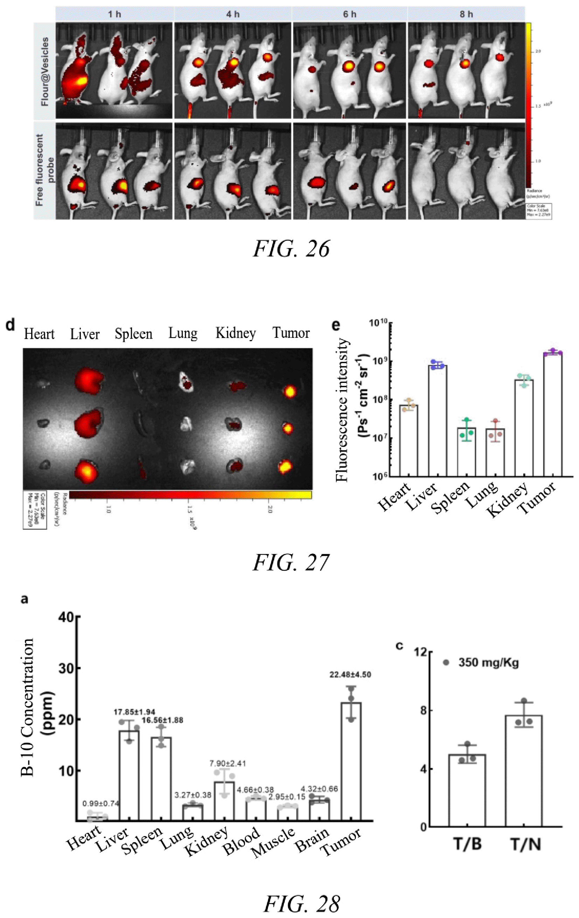

At 1, 4, 6, and 8 h post-injection, the mice were imaged using the IVIS Spectrum in Vivo imaging system. Refer to FIG. 26. Subsequently, the time point at which the fluorescent probe-labelled vesicles exhibited strong fluorescence distribution at the tumour site was determined.

Through experiments, it can be known that approximately 1 to 2 h after injection, the vesicles loaded with fluorescent probes had already begun to accumulate at the tumour site via blood circulation. Relatively high fluorescence signals were observed at the tumour site between 6-8 h over time. In contrast, the free probe group lacked tumour-targeting capability, with probes initially concentrating in the abdominal region and being almost completely metabolized and excreted from the body by around 8 h.

### ② Fluorescence distribution of vesicles in tissues

Experimental method: The tumour-bearing mouse was intravenously administered via the tail vein with fluorescent probe-loaded vesicle nanoparticles at a dose of 10 mg/kg. After approximately 6 h of systemic circulation, sodium amobarbital was intraperitoneally injected for anesthesia.

This experiment employed fluorescent probe-labelled LA-PEG₄-BPA vesicles. Vesicle preparation: An appropriate amount of the corresponding lipoic acid derivative was weighed and placed into a sample vial, double-distilled water was added, an appropriate amount of NaOH aqueous solution was added to adjust the pH to approximately 10-11, mixed well, and then an appropriate amount of hydrochloric acid was added to adjust the pH to approximately 7.0, thereby completing vesicle assembly.

After approximately 6-8 h of systemic circulation, the mice were euthanized, and major organs (heart, liver, spleen, lung, kidney) and tumours were harvested. Fluorescence images of the ex vivo organs were detected using IVIS Spectrum (refer to FIG. 27d), and fluorescence intensity was immediately measured (refer to FIG. 27e).

The fluorescence distribution of vesicles in tissues is shown in FIG. 27d and 27e. Panel d shows the imaging of major organs under the small animal in vivo imaging system, where the tumour exhibited the strongest fluorescence signal, followed by the liver; Panel e is a bar chart presenting the statistical analysis of fluorescence intensity from panel d.

### ③ Distribution of boron element in various sites

Experimental method: A dose of 350 mg/kg vesicle nanoparticles was injected into the tumour-bearing mouse via the tail vein. After approximately 6 h of systemic circulation, sodium amobarbital was administered by intraperitoneal injection for anesthesia. After the mice were deeply anesthetized, the heart, liver, spleen, lung, kidney, brain, blood, and tumour tissue were collected for ICP-MS detection of B-10 content.

This experiment utilized vesicles prepared from the LA-PEG₄-BPA small molecule, wherein the B element was the B-10 isotope. Vesicle preparation: An appropriate amount of the corresponding lipoic acid derivative was weighed and placed into a sample vial, double-distilled water was added, an appropriate amount of NaOH aqueous solution was added to adjust the pH to approximately 10-11, mixed well, and then an appropriate amount of hydrochloric acid was added to adjust the pH to approximately 7.0, thereby completing vesicle assembly.

Quantitative analysis of boron-10 content in organs and tissues such as the heart, liver, spleen, lung, kidney, brain, blood, and tumour was monitored by ICP-MS. For ICP-MS, the organs were digested with 5 mL of 60% nitric acid at 90°C for 12 h and diluted to 30 mL with deionized water. Refer to panels a and c in FIG. 28.

The distribution of boron element in various sites is shown in panels a and c of FIG. 28, where panels a and c illustrate the boron-10 distribution. Panel a shows the concentration of B-10 in heart, liver, spleen, lung, kidney, blood, muscle, brain, and tumour when the administration concentration was 350 mg/kg. Panel c shows that when the administration concentration was 350 mg/kg, the ratio of the drug concentration in tumour tissue to that in blood (T/B) was higher than 4, and the ratio of the drug concentration in tumour tissue to that in normal tissue (T/N) was higher than 6.

Detection results of B-10 distribution: The content distribution trend of B-10 was consistent with the in vivo distribution trend of fluorescent probe-labelled vesicles, with the greatest enrichment at the tumour site; the T/N ratio and T/B ratio of the drug group were both greater than 4.

Additionally, studies have found that the B-10 content at the tumour site increased as a function of the increasing administration dose, and the in vivo distribution of B-10 was essentially the same across different administration doses.

### Test example 9

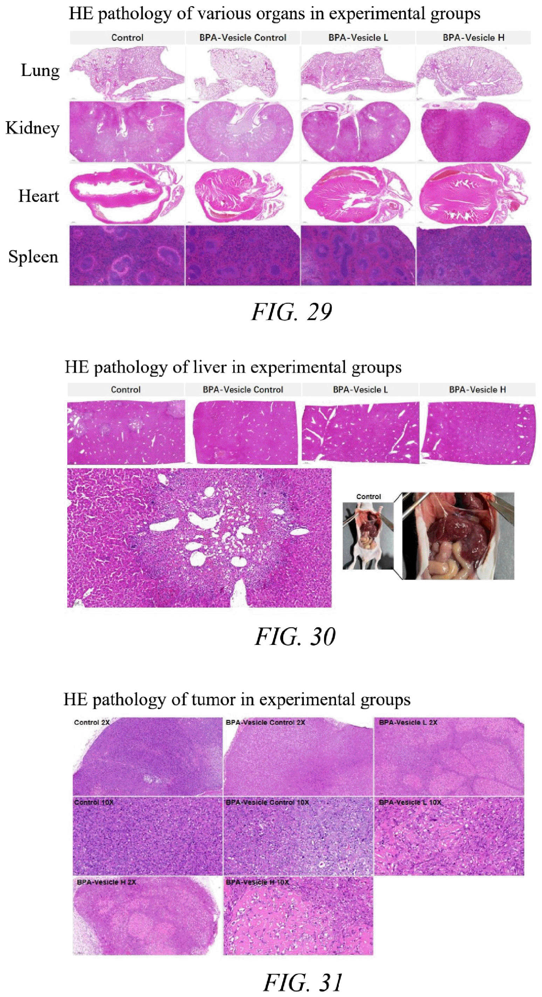

① In this test example, HE pathological studies were conducted on various organs of the mice after irradiation in 2.1) of (1) in test example 6, and the study results are shown in FIGs. 29 to 31. The Control, BPA-Vesicle Control, BPA-Vesicle L, and BPA-Vesicle H experimental groups in FIGs. 29 to 31 corresponded to the physiological saline group, high-dose control group, low-dose treatment group, and high-dose treatment group in 2.1) of test example 6, respectively.

HE pathological images of various organs in the experimental group are shown in FIG. 29: Two weeks after the end of treatment, the heart, lung, spleen, and kidney of mice were subjected to HE staining. After nanovesicle treatment, no significant macromolecular deposition was observed in the heart, lung, kidney, or spleen, and no obvious pathological changes were found in any of the tissues. The experimental results further verified that the nano-drug prepared in the present invention has low visceral toxicity and high biosafety in mice.

The liver HE pathological images of the experimental groups are shown in FIG. 30: Liver tissues from mice in different groups were embedded and subjected to paraffin sectioning for HE staining. The results showed that no significant pathological changes were observed in the liver of mice in the BPA-Vesicle Group H, BPA-Vesicle Group L, and the non-irradiated vesicle group (BPA-Vesicle Control). In contrast, diffuse tumour metastatic lesions were detected in the liver site of mice in the physiological saline control group (Control) (refer to the Control group and its magnified image below in the figure).

HE pathological images of tumours in the experimental groups are shown in FIG. 31. The tumour tissues of mice without neutron irradiation treatment exhibited dense cancer cells with robust growth, whereas the tumour tissue structure was disrupted after neutron irradiation treatment, and the cancer cells exhibited obvious apoptotic characteristics.

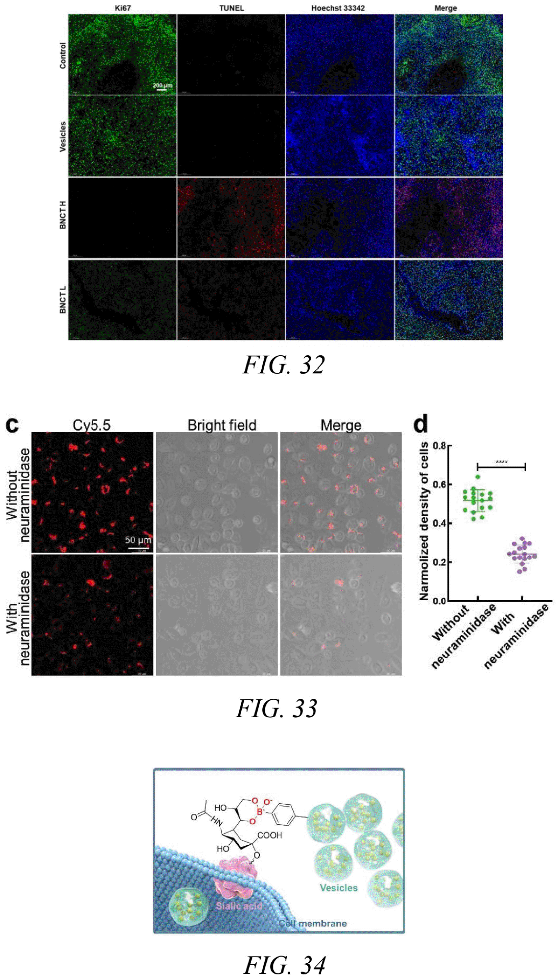

② This test example also performed cell apoptosis detection on the tumour tissues of each experimental group of mice in 2.1) of (1) in test example 6 after irradiation, Ki67 and TUNEL fluorescence labelling imaging methods were used to detect cell apoptosis. The detection results are shown in detail in FIG. 32. In FIG. 32 of this test example, Control, Vesicle, BNCT L, and BNCT H correspond to the physiological saline group, high-dose control group, low-dose treatment group, and high-dose treatment group in 2.1) of test example 6, respectively.

FIG. 32 shows the fluorescence imaging results of the Ki-67 proliferation assay and TUNEL apoptosis assay in each experimental group in this test example. FIG. 32 shows the results of Ki-67 proliferation assay and TUNEL apoptosis assay in different experimental groups. The green fluorescence represents the Ki-67 fluorescence signal of tumour tissue cancer cell proliferation, and the red fluorescence represents the TUNEL fluorescence signal of tumour tissue cancer cell apoptosis. The BNCT Group H exhibited the weakest green fluorescence signal and the strongest red fluorescence, indicating that most tumour cells in the tumour tissue had undergone apoptosis after treatment in the BNCT Group H. The BNCT L group showed the next best results. It can be seen that the BNCT Group L and BNCT Group H in this test example exhibited superior cell apoptosis effects.

### Test example 10 Sialic acid-targeting detection

To explore the sialic acid-targeting function of vesicles, this test example further evaluated the uptake of L^{p}B-3 (LA-PEG₄-BPA) vesicles in PANC-1 cells with and without neuraminidase treatment.

Experimental method for sialic acid-targeting detection: PANC-1 cells were seeded into 2.5 cm confocal culture dishes, and cell confluency reached 70-80% for subsequent experiments. The original culture medium was discarded, and cell culture medium containing 200 µg/mL Cy5.5@vesicles was added to the cells. Meanwhile, a control experiment was set up, where PANC-1 cells were treated with 0.5 U of neuraminidase for 2 h before adding 200 µg/mL Cy5.5@vesicles, the original culture medium was discarded, and then Cy5.5@vesicles were added to treat PANC-1 cells. Prior to imaging, the original culture medium was discarded, and ImageJ was used to statistically analyse the fluorescence intensity of the imaging results. Cy5.5@vesicles were Cy5.5-loaded vesicles, and according to test example 5, the Cy5.5-loaded vesicles could be uniformly distributed within the cell through cell uptake, and the vesicles could serve as nanocarriers to effectively deliver small molecule compounds into the cell.

Experimental results: As shown in panel c of FIG. 33, PANC-1 cells without neuraminidase treatment exhibited higher cell uptake of Cy5.5@vesicles, with significantly stronger fluorescence intensity; whereas cells with neuraminidase treatment exhibited significantly reduced cell uptake, with decreased intracellular fluorescence intensity. The fluorescence statistical results are shown in panel d of FIG. 33, where the intracellular fluorescence intensity without neuraminidase treatment was approximately 2.4 times that with neuraminidase treatment. Experimental results indicate that L^{p}B-3 vesicles could achieve effective intracellular enrichment by targeting sialic acid on the PANC-1 cell membrane surface. FIG. 34 shows the schematic diagram of the sialic acid-targeting mechanism of vesicles.

This test example further evaluated the uptake of L^{p}B-3 vesicles in cancer cells with high sialic acid expression.

Experimental method for cell uptake: PANC-1, Primary pancreatic acinar cells, MDA-MB-231, and MCF-10A were seeded in 2.5 cm confocal culture dishes, respectively, and cultured until reaching approximately 80% confluence for subsequent experiments. The original culture medium was discarded, and a cell culture medium containing 200 µg/mL Cy5.5@vesicles was added. After incubation for 4 h, confocal imaging was performed to observe the vesicle uptake within individual cells.

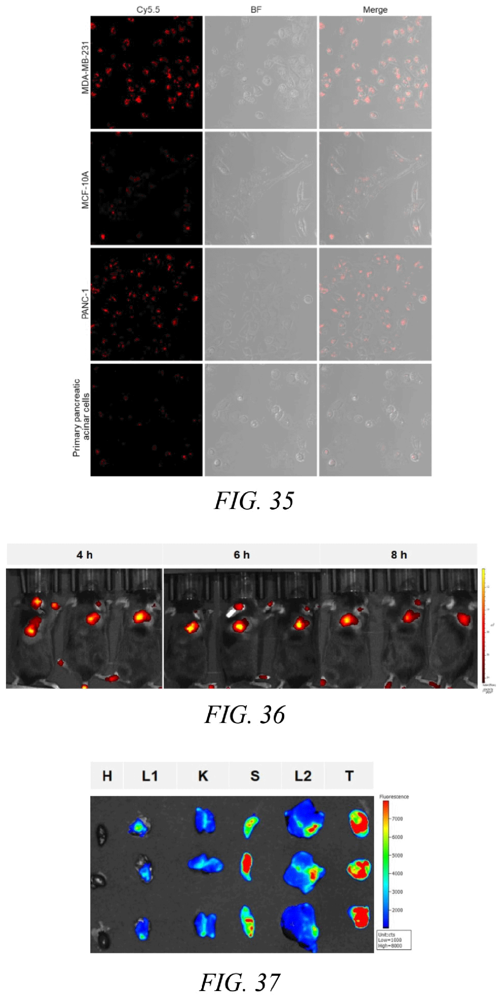

Experimental results: As shown in FIG. 35, the fluorescence intensity of L^{P}B-3 vesicles in cancer cells (PANC-1 and MDA-MB-231) was significantly higher than that in normal cells (Primary pancreatic acinar cells and MCF-10A), indicating that cancer cells with high expression of sialic acid exhibited greater uptake of the vesicles compared to normal cells.

### Test example 11

B16F10 tumour-bearing C57 mouse modelling: Female C57 mice (weighing approximately 20 g) were ordered from GemPharmatech and housed in the animal facility for one week to acclimate to the environment. Prior to tumour cell inoculation, the inoculation site was depilated and the mouse skin was disinfected by wiping with 75% ethanol. Approximately 500,000 B16F10 cells (melanoma cells) were subcutaneously inoculated per mouse. The tumours were allowed to grow to a volume of approximately 80-100 mm³.

### ① Fluorescence distribution of vesicles in targeted tumour

Method: Fluorescent probe-labelled vesicle nanoparticles (10 mg/Kg) were injected into B16F10 subcutaneous tumour-bearing mice via tail vein (n = 3/group). This experiment employed fluorescent probe-labelled LA-PEG₄-BPA vesicles.

At 4, 6, and 8 h post-injection, the mice were imaged using the IVIS Spectrum in Vivo imaging system. Refer to FIG. 36. Subsequently, the time point at which the fluorescent probe-labelled vesicles exhibited strong fluorescence distribution at the tumour site was determined.

As shown in FIG. 36: At 4 h after injection, the vesicles loaded with fluorescent probes had already begun to accumulate at the tumour site via blood circulation. Over time, relatively high fluorescence signals were observed at the tumour site at 6 h.

### ② Fluorescence distribution of vesicles in tissues

Experimental method: The tumour-bearing mouse was intravenously administered via the tail vein with fluorescent probe-loaded vesicle nanoparticles at a dose of 10 mg/kg. After approximately 6 h of systemic circulation, sodium amobarbital was intraperitoneally injected for anesthesia.

This experiment employed fluorescent probe-labelled LA-PEG₄-BPA vesicles. Vesicle preparation: An appropriate amount of the corresponding lipoic acid derivative was weighed and placed into a sample vial, double-distilled water was added, an appropriate amount of NaOH aqueous solution was added to adjust the pH to approximately 10-11, mixed well, and then an appropriate amount of hydrochloric acid was added to adjust the pH to approximately 7.0, thereby completing vesicle assembly.

After approximately 6 h of systemic circulation, the mice were euthanized, and major organs (heart (H), liver (L2), spleen (S), lung (L1), kidney (K)) and tumour (T) were harvested. The fluorescence images of the ex vivo organs were detected using IVIS Spectrum, and the fluorescence intensity was immediately measured. Refer to FIG. 37.

### ③ Distribution of boron element in various sites

Experimental method: A dose of 350 mg/kg vesicle nanoparticles was injected into the tumour-bearing mouse via the tail vein. After approximately 6 h of systemic circulation, sodium amobarbital was administered by intraperitoneal injection for anesthesia. After the mice were deeply anesthetized, the heart, liver, spleen, lung, kidney, brain, blood, and tumour tissue were collected for ICP-MS detection of B-10 content.

This experiment utilized vesicles prepared from the LA-PEG₄-BPA small molecule, wherein the B element was the B-10 isotope. Vesicle preparation: An appropriate amount of the corresponding lipoic acid derivative was weighed and placed into a sample vial, double-distilled water was added, an appropriate amount of NaOH aqueous solution was added to adjust the pH to approximately 10-11, mixed well, and then an appropriate amount of hydrochloric acid was added to adjust the pH to approximately 7.0, thereby completing vesicle assembly.

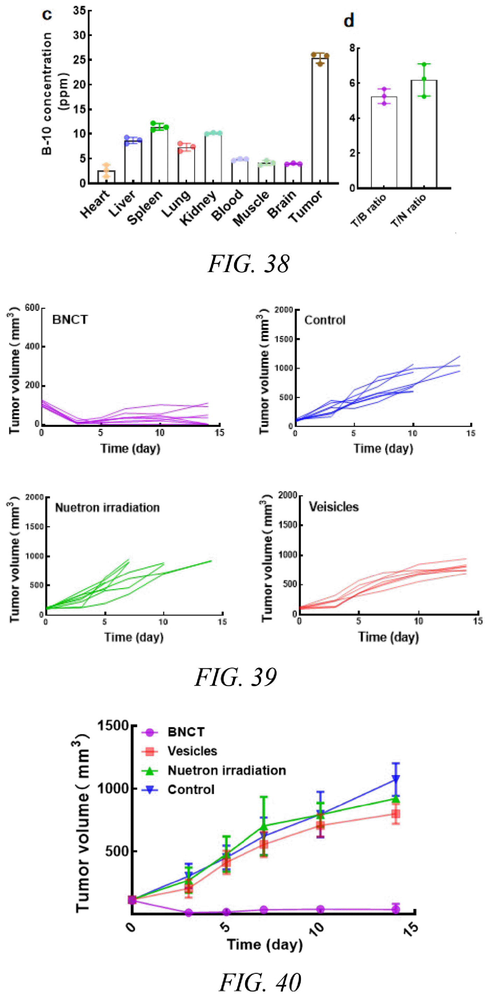

Quantitative analysis of boron-10 content in organs and tissues such as the heart, liver, spleen, lung, kidney, brain, blood, and tumour was monitored by ICP-MS. For ICP-MS, the organs and tissues were digested with 5 mL of 60% nitric acid at 90°C for 12 h and diluted to 30 mL with deionized water. Refer to panel c of FIG. 38.

The distribution of boron element in various sites is as shown in panel c of FIG. 38. Panel c shows the concentration of B-10 in heart, liver, spleen, lung, kidney, blood, muscle, brain, and tumour when the administration concentration was 350 mg/kg. Panel d of FIG. 38 shows that when the administration concentration was 350 mg/kg, the ratio of the drug concentration in tumour tissue to that in blood (T/B) was higher than 4, and the ratio of the drug concentration in tumour tissue to that in normal tissue (T/N) was higher than 6.

Detection results of B-10 distribution: The content distribution trend of B-10 was consistent with the in vivo distribution trend of fluorescent probe-labelled vesicles, with the greatest enrichment at the tumour site; the T/N ratio and T/B ratio of the drug group were both greater than 4.

### Test example 12

Using the same B16F10 tumour-bearing C57 mice as in example 11, this test example further evaluated the BNCT efficacy of the vesicle in B16F10 tumour-bearing C57 mice. The experimental groups were as follows.
Control: physiological saline control group, without neutron irradiation;
Neutrion irradiation: neutron irradiation group, physiological saline, with neutron irradiation;
Veisicles: vesicle control group, administered with 350 mg/kg of LA-PEG₄-¹⁰BPA vesicles without neutron irradiation;
BNCT: BNCT treatment group, administered with 350 mg/kg of LA-PEG₄-¹⁰BPA vesicles, with neutron irradiation;
The radiation dose of neutron irradiation was about 1.5 Gy.

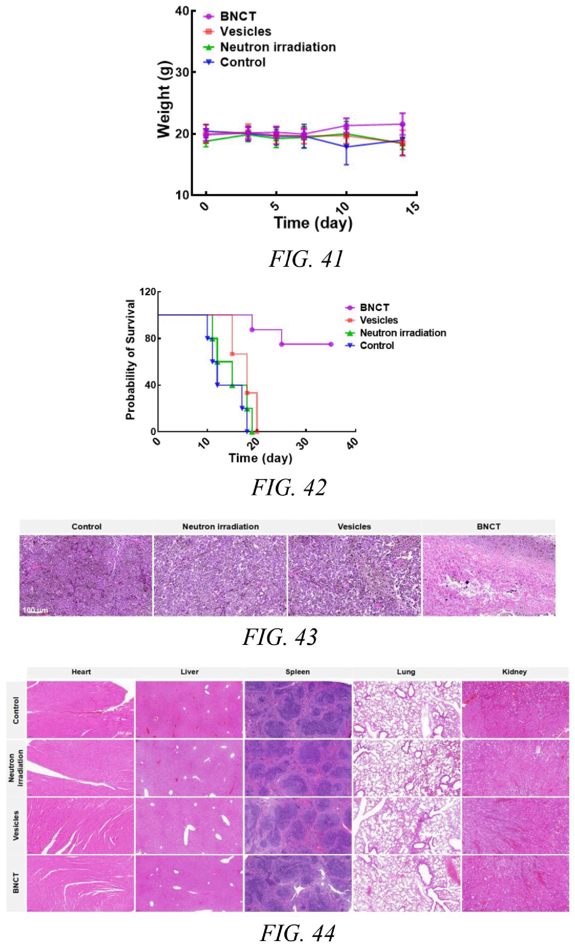

As shown in FIGs. 39-40, the tumour volume of mice in the BNCT experimental group of the treatment group was well inhibited, while the tumour volume of mice in the other three experimental groups increased significantly over time. During the treatment, no significant changes in body weight were observed in mice of any of the experimental groups (as shown in FIG. 41). The survival time statistics results are shown in FIG. 42, where the mice in BNCT treatment group exhibited significantly improved survival time, while the physiological saline control group, neutron irradiation group, and vesicle control group all had a total survival time of less than three weeks.

HE pathological images of tumours in the experimental groups are shown in FIG. 43. The tumour tissues of mice without neutron irradiation treatment exhibited dense cancer cells with robust growth, whereas the tumour tissue structure was disrupted after neutron irradiation treatment, and the cancer cells exhibited obvious apoptotic characteristics.

This test example conducted HE pathological studies on various organs of mice after irradiation in the test example, and the study results are shown in FIG. 44. In FIG. 44, the Control, Vesicle, Neutron irradiation, and BNCT experimental groups correspond to the physiological saline group, vesicle control group, neutron irradiation group, and BNCT treatment group in the test example, respectively.

HE pathological images of various organs in the experimental group are shown in FIG. 44: Approximately 1 week after the end of treatment, the heart, liver, lung, spleen, and kidney of mice were subjected to HE staining. After nanovesicle treatment, no significant macromolecular deposition was observed in the heart, liver, lung, kidney, or spleen, and no obvious pathological changes were found in any of the tissues. The experimental results further verified that the nano-drug prepared in the present invention has low visceral toxicity and high biosafety in mice.

In summary, the lipoic acid derivative and vesicle of the present invention are a BNCT drug with potential for clinical application, which exhibits excellent tumour-targeting characteristics, can target cancer cells with high sialic acid expression, and achieves a high T/N (tumour/normal tissue) ratio and T/B (tumour/blood) ratio through active targeting, thus facilitating effective enrichment and retention of B-10 in cancer cells and benefiting the implementation of BNCT.

The above are only the preferred embodiments of the present invention. It should be pointed out that for those of ordinary skill in the art, several improvements and modifications can be made without departing from the principle of the present invention and these improvements and modifications should also be regarded as the protection scope of the present invention.

## Claims

1. A lipoic acid derivative, or a stereoisomer or pharmaceutically acceptable salt thereof, **characterized in that** the lipoic acid derivative is represented by formula (1): 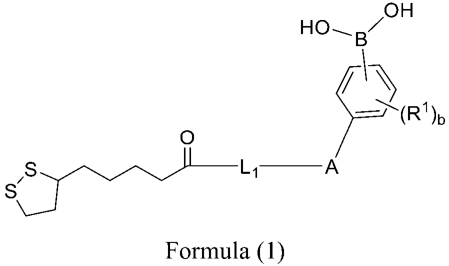
L₁ is selected from a bond, 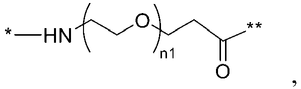 or 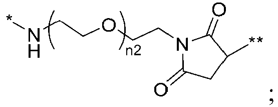
A is selected from 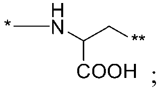
each R^{I} is independently selected from H, halogen, a hydrocarbon group having 1 to 4 carbon atoms, or a boronic acid group;
n1 is selected from 0, 1, 2, 3, 4, 5, 6, 7, 8, 9, 10, 11, or 12;
n2 is selected from 0, 1, 2, 3, 4, 5, 6, 7, 8, 9, 10, 11, or 12;
b is selected from 0, 1, 2, 3, or 4;
* and ** are linking sites.

2. The lipoic acid derivative, or the stereoisomer or pharmaceutically acceptable salt thereof according to claim 1, **characterized in that** in the structure of the lipoic acid derivative, at least one B is ¹⁰B; and/or
the lipoic acid derivative has a ¹⁰B abundance of greater than or equal to 90%; and/or
each R¹ is independently selected from H, F, Cl, Br, I or boronic acid group.

3. The lipoic acid derivative, or the stereoisomer or pharmaceutically acceptable salt thereof according to claim 1, **characterized in that** the structure of the lipoic acid derivative is represented by formula (1a):

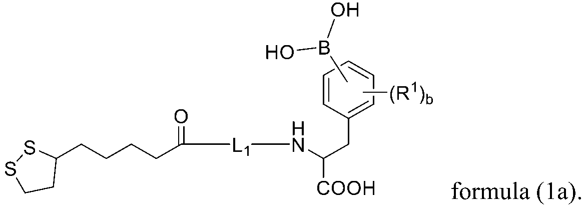

4. The lipoic acid derivative, or the stereoisomer or pharmaceutically acceptable salt thereof according to claim 3, **characterized in that** the lipoic acid derivative is selected from one of the following structures:

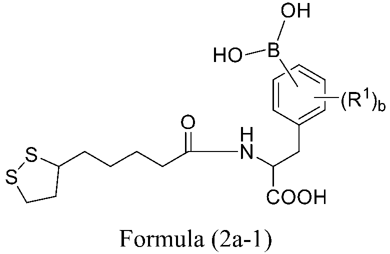

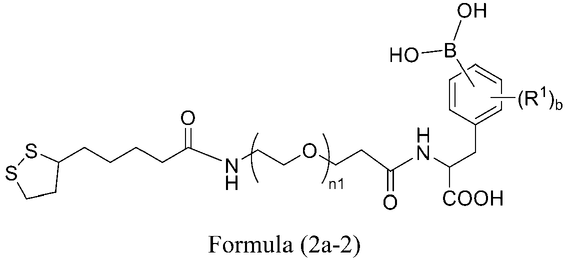

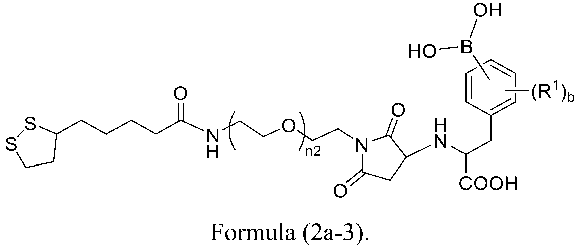

5. The lipoic acid derivative, or the stereoisomer or pharmaceutically acceptable salt thereof according to claim 3, **characterized in that** the structure of the lipoic acid derivative is selected from one of the following structures:

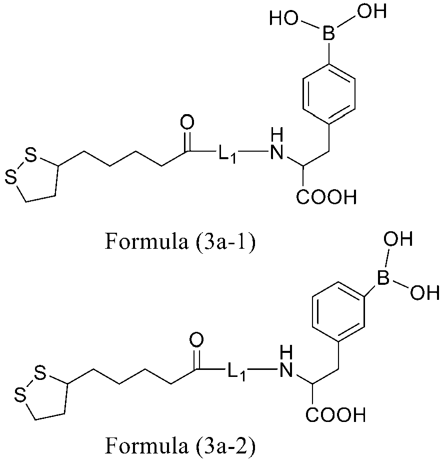

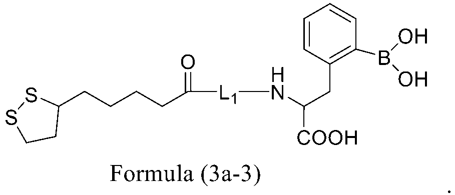

6. The lipoic acid derivative, or the stereoisomer or pharmaceutically acceptable salt thereof according to claim 3, **characterized in that** the lipoic acid derivative is selected from one of the following structures: 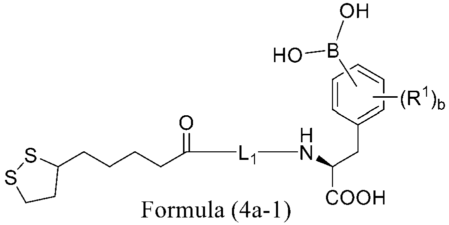 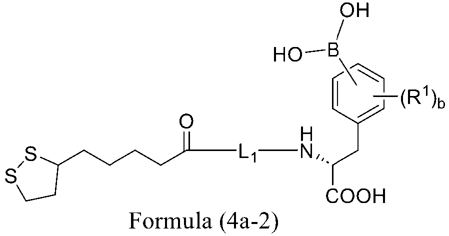 each R¹ is independently selected from H, halogen, a hydrocarbon group having 1 to 4 carbon atoms, or a boronic acid group; preferably, each R¹ is independently H.

7. The lipoic acid derivative, or the stereoisomer or pharmaceutically acceptable salt thereof according to claim 3, **characterized in that** the lipoic acid derivative is selected from one of the following structures:

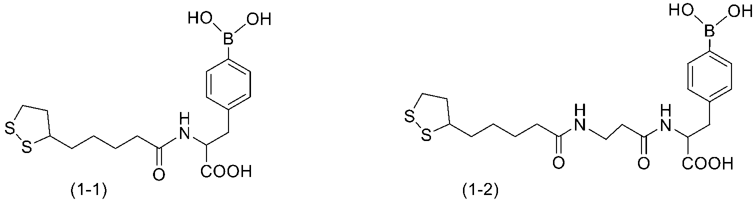

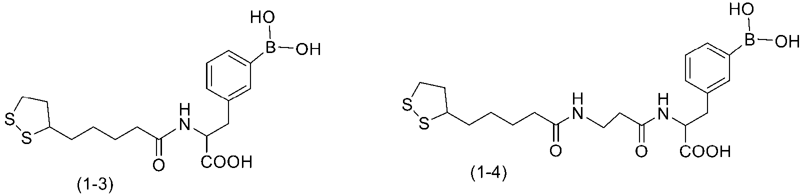

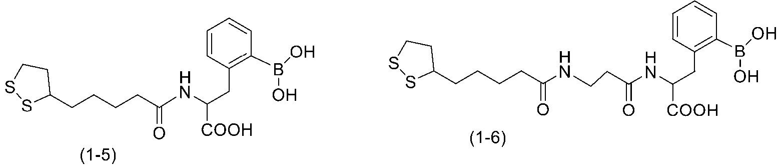

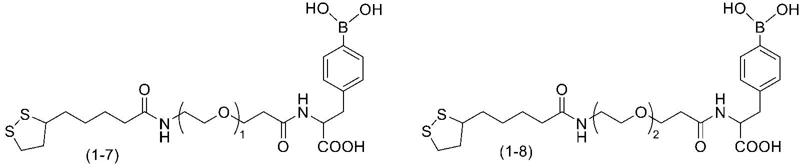

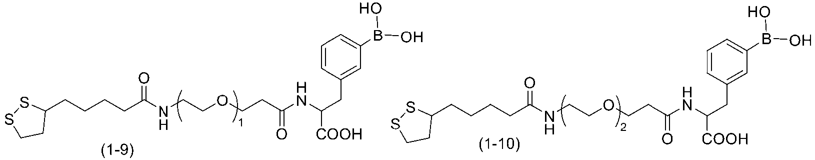

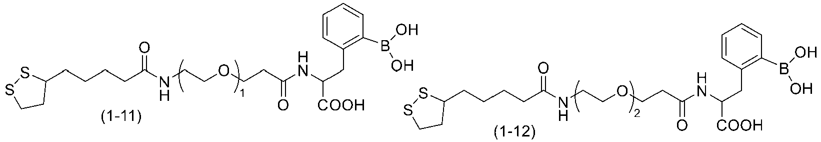

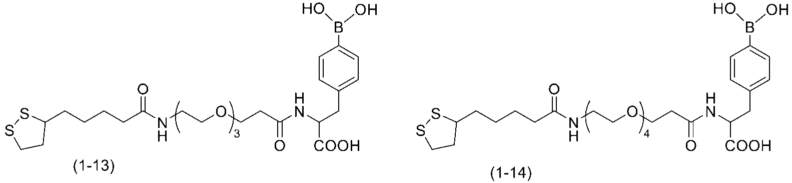

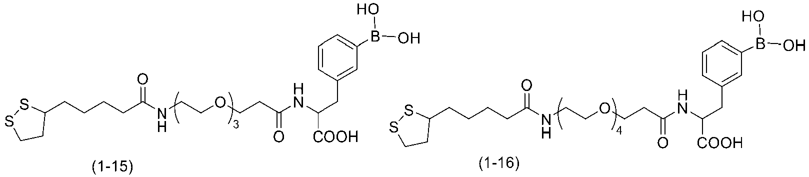

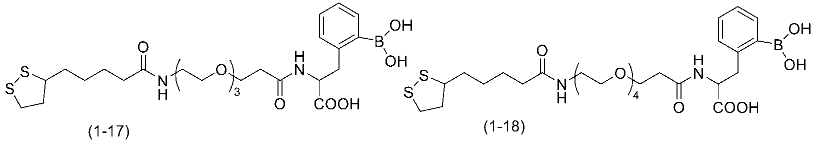

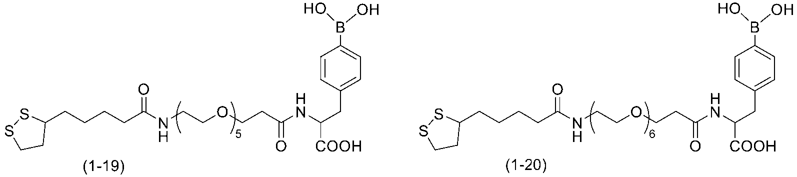

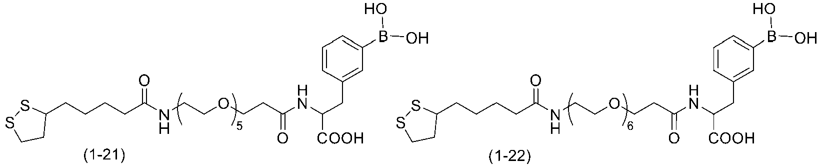

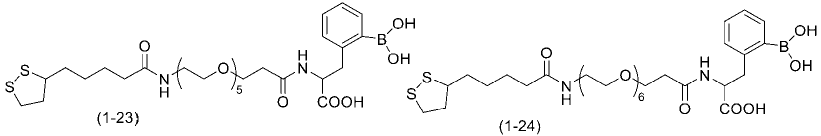

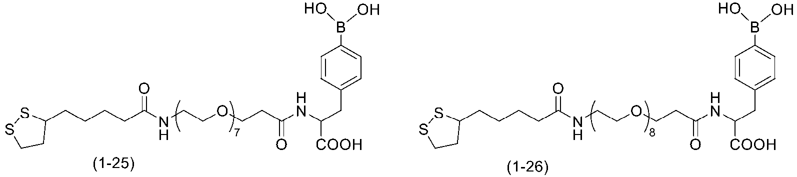

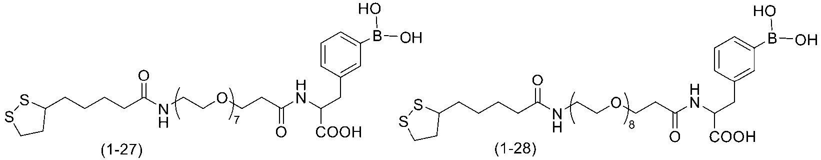

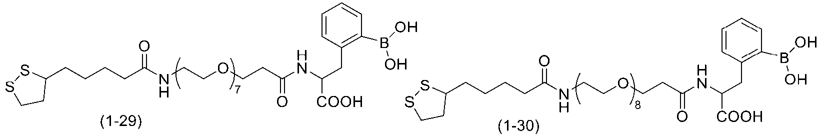

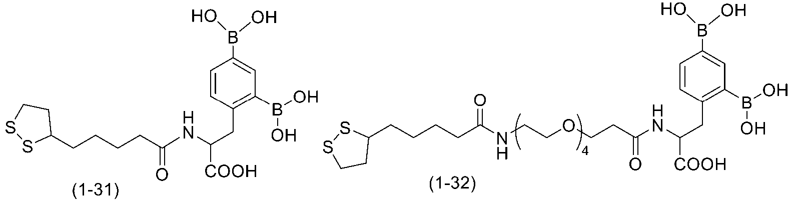

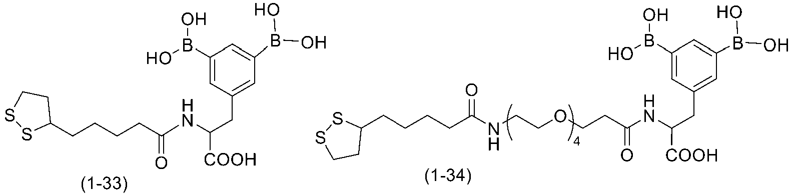

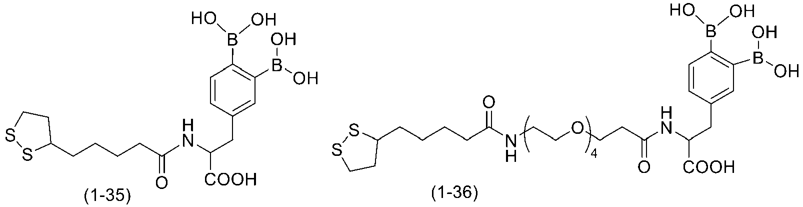

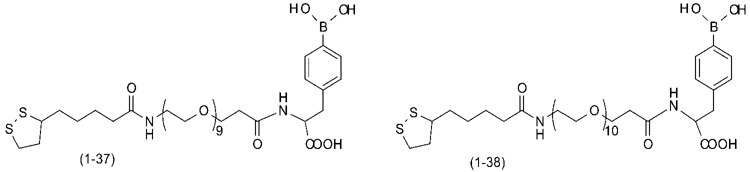

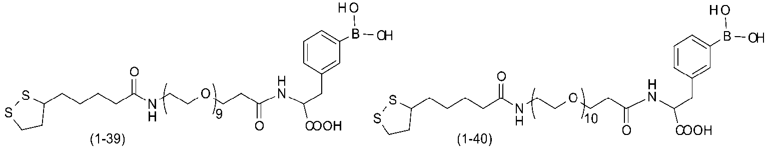

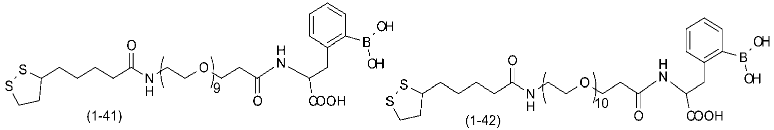

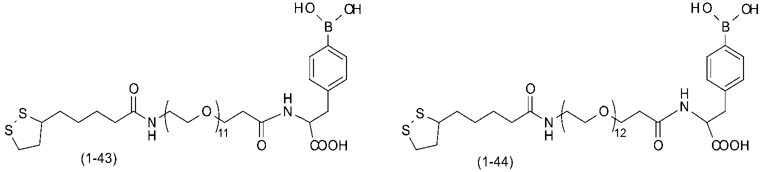

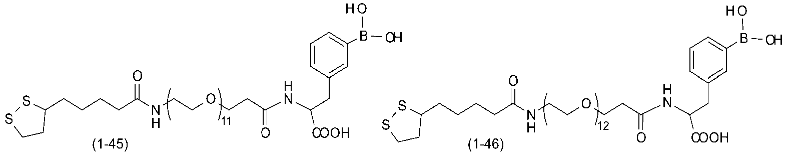

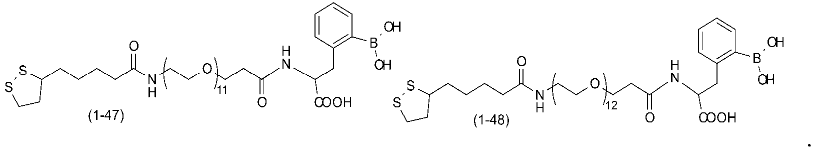

8. The lipoic acid derivative, or the stereoisomer or pharmaceutically acceptable salt thereof according to any one of claims 3 to 7, **characterized in that** in the structure of the lipoic acid derivative, at least one B is ¹⁰B.

9. The lipoic acid derivative, or the stereoisomer or pharmaceutically acceptable salt thereof according to any one of claims 3 to 7, **characterized in that** the lipoic acid derivative has a ¹⁰B abundance of greater than or equal to 90%.

10. A vesicle compound, **characterized in that** the vesicle compound is formed using the lipoic acid derivative, or the stereoisomer or pharmaceutically acceptable salt thereof according to any one of claims 1 to 9; the lipoic acid derivative forms the vesicle compound through disulfide bond ring-opening crosslinking.

11. The vesicle compound according to claim 10, **characterized in that** the vesicle compound is represented by formula (a): 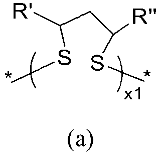
R' and R" are each independently selected from H or 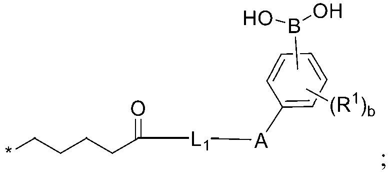 and, in each 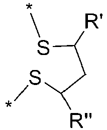 structure, one of R' and R" is 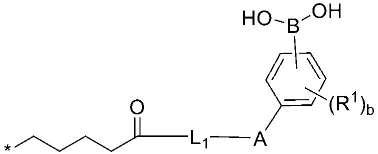 and the other is H;
each L₁ is independently selected from a bond, 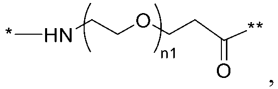 or 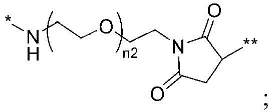
A is selected from 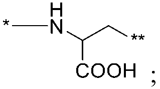
each R¹ is independently selected from H, halogen, a hydrocarbon group having 1 to 4 carbon atoms, or a boronic acid group;
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
x1 is selected from an integer from 1 to 500,000;
* and ** are linking sites.

12. The vesicle compound according to claim 10, **characterized in that** the vesicle compound is represented by formula (a): 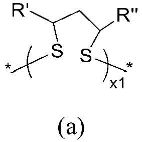
R' and R" are each independently selected from H or 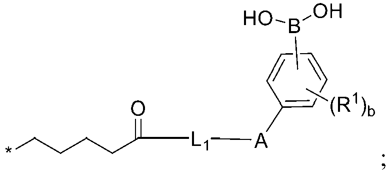 and, in each 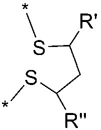 structure, one of R' and R" is 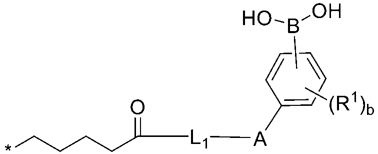 and the other is H;
each L₁ is independently selected from a bond, 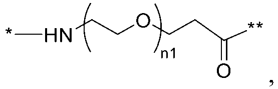 or 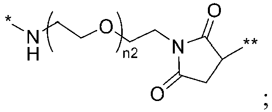
A is selected from 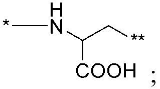
each R¹ is independently selected from H, halogen, a hydrocarbon group having 1 to 4 carbon atoms, or a boronic acid group;
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
x1 is selected from an integer from 1 to 10,003;
* and ** are linking sites.

13. The vesicle compound according to claim 10, **characterized in that** the vesicle compound has a structure represented by formula (2): 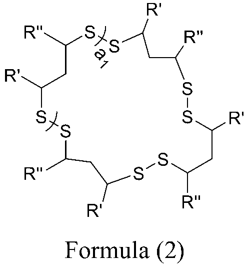
R' and R" are each independently selected from H or 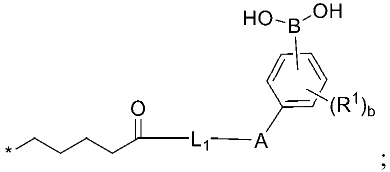 and, in each 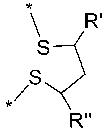 structure, one of R' and R" is 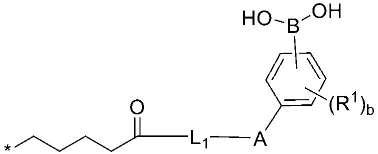 and the other is H;
each L₁ is independently selected from a bond, 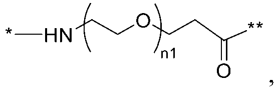 or 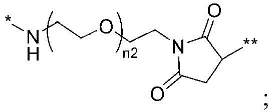
A is selected from 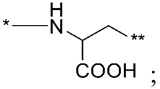
each R¹ is independently selected from H, halogen, a hydrocarbon group having 1 to 4 carbon atoms, or a boronic acid group;
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
a1 is selected from an integer from 0 to 500,000;
* and ** are linking sites.

14. The vesicle compound according to claim 10, **characterized in that** the vesicle compound has a structure represented by formula (2): 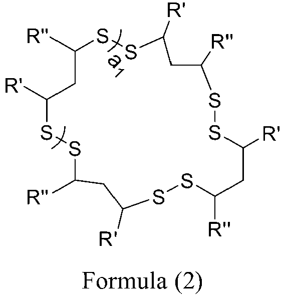
R' and R" are each independently selected from H or 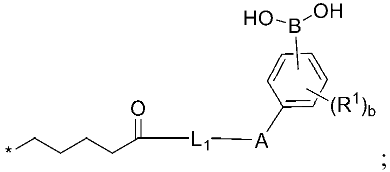 and, in each 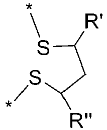 structure, one of R' and R" is 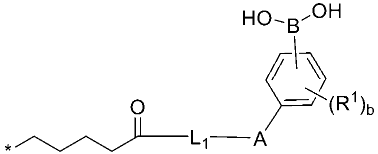 and the other is H;
each L₁ is independently selected from a bond, 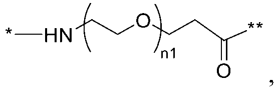 or 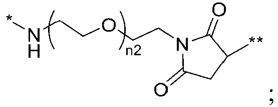
A is selected from 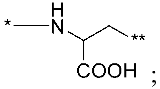
each R¹ is independently selected from H, halogen, a hydrocarbon group having 1 to 4 carbon atoms, or a boronic acid group;
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
a1 is selected from an integer from 0 to 10,000;
* and ** are linking sites.

15. A vesicle compound, or a stereoisomer or pharmaceutically acceptable salt thereof, **characterized in that** the vesicle compound is represented by formula (a): 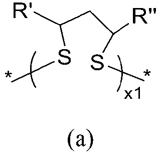
R' and R" are each independently selected from H or 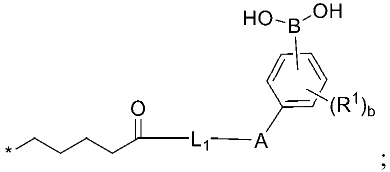 and, in each 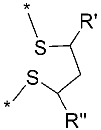 structure, one of R' and R" is 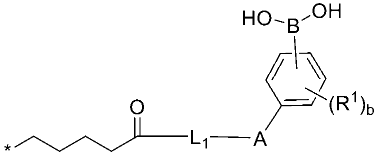 and the other is H;
each L₁ is independently selected from a bond, 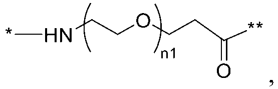 or 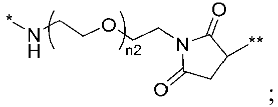
A is selected from 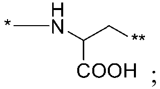
each R¹ is independently selected from H, halogen, a hydrocarbon group having 1 to 4 carbon atoms, or a boronic acid group;
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
x1 is selected from an integer from 1 to 500,000;
* and ** are linking sites.

16. A vesicle compound, or a stereoisomer or pharmaceutically acceptable salt thereof, **characterized in that** the vesicle compound is represented by formula (2): 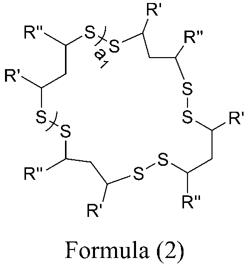
R' and R" are each independently selected from H or 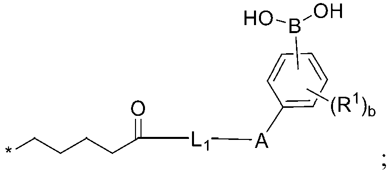 and, in each 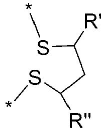 structure, one of R' and R" is 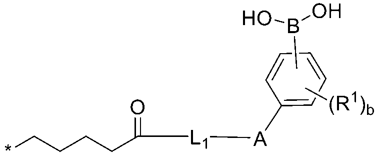 and the other is H;
each L₁ is independently selected from a bond, 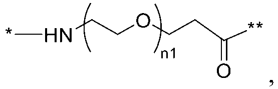 or 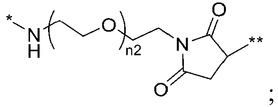
A is selected from 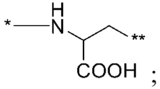
each R¹ is independently selected from H, halogen, a hydrocarbon group having 1 to 4 carbon atoms, or a boronic acid group;
each n1 is independently selected from 0, 1, 2, 3, 4, 5, 6, 7, 8, 9, 10, 11, or 12;
each n2 is independently selected from 0, 1, 2, 3, 4, 5, 6, 7, 8, 9, 10, 11, or 12;
each b is independently selected from 0, 1, 2, 3, or 4;
a1 is selected from an integer from 0 to 500,000;
* and ** are linking sites.

17. The vesicle compound according to any one of claims 11 to 16, **characterized in that** n1 and n2 are each independently selected from 1, 2, 3, 4, 5, 6, 7, or 8, respectively; and/or
each R¹ is independently selected from H, halogen or boronic acid group; and/or
a grain size of the vesicle compound is 50-300 nm; and/or
in the vesicle compound, at least one B is ¹⁰B.

18. The vesicle compound according to any one of claims 11 to 16, **characterized in that** the vesicle compound has a ¹⁰B abundance of greater than or equal to 90%; and/or
the grain size of the vesicle compound is 50-200 nm.

19. A pharmaceutical composition, **characterized in that** the pharmaceutical composition comprises the lipoic acid derivative according to any one of claims 1 to 9 and/or the vesicle compound according to any one of claims 10 to 18.

20. The lipoic acid derivative according to any one of claims 1 to 9, or the vesicle compound according to any one of claims 10 to 18, or the pharmaceutical composition according to claim 19 for use in the preparation of an antitumour drug.

21. The use according to claim 20, **characterized in that** the tumour is a tumour with high sialic acid expression.

22. The use according to claim 20, **characterized in that** the tumour is selected from prostate cancer, ovarian cancer, liver cancer, glioma, pancreatic cancer, or breast cancer.

23. The use according to claim 20, **characterized in that** the tumour is melanoma.

24. The use according to claim 20, **characterized in that** The tumour is selected from melanoma, glioblastoma, renal neuroblastoma, astrocytoma, brain tumour, head and neck cancer, sarcoma, rhabdomyosarcoma, mastocarcinoma, bladder cancer, colon cancer, ovarian cancer, colorectal cancer, prostate cancer, gastric cancer, pancreatic cancer, extrahepatic cholangiocarcinoma, cervical cancer, adenocarcinoma, liver cancer, lung cancer, breast cancer, cysteine cancer, renal cancer, childhood leukaemia, acute myelocytic leukaemia, chronic myeloid leukaemia, multiple myeloma, squamous cell carcinoma, cervical cancer, or epithelial cell carcinoma.

25. A preparation method for the lipoic acid derivative according to any one of claims 1 to 9, **characterized in that**
when A is selected from 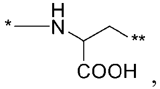 and L₁ is selected from a bond or 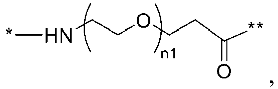
the method comprises preparing a lipoic acid derivative using a compound of formula (11) and a phenylboronic acid derivative of formula (12) as starting materials:

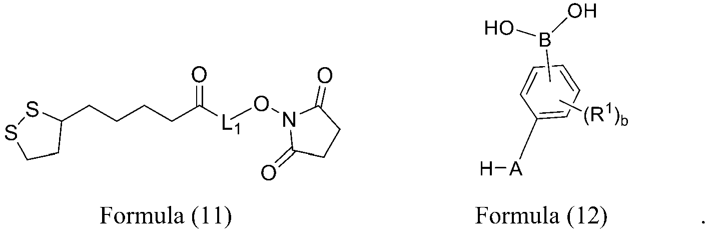

26. The preparation method according to claim 25, wherein the method further comprises preparing the compound of formula (11) using a lipoic acid compound of formula (13) as a starting material:

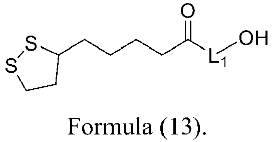

27. The preparation method according to claim 26, wherein
when L₁ is 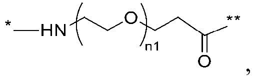 the method further comprises:
preparing the compound of formula (13) using a compound of formula (14) as a starting material: 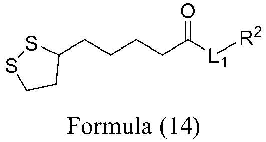
R² is a carboxyl protecting group.

28. The preparation method according to claim 27, wherein the method further comprises preparing the compound of formula (14) using a compound of formula (15) and a compound of formula (16) as starting materials: 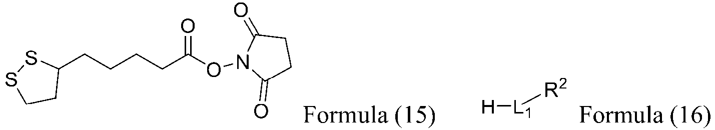 in the compound of Formula (16), R² is a carboxyl protecting group.

29. The preparation method according to claim 27 or 28, wherein R² is - OR³ or -NR⁴R⁵.

30. The preparation method according to claim 29, wherein R³ is selected from alkyl having 1 to 20 carbon atoms, and heteroalkyl having 1 to 20 carbon atoms, wherein the heteroalkyl contains one or more heteroatoms such as nitrogen, oxygen, or sulphur; preferably, R³ is selected from methyl, dimethyl, ethyl, tert-butyl, benzyl, diphenylmethyl, p-nitrobenzyl, p-methoxybenzyl, 4-pyridylmethyl, β,β,β-trichloroethyl, β-methylthioethyl, β-p-toluenesulfonylethyl or β-p-nitrophenylthioethyl; more preferably, R³ is tert-butyl.

31. The preparation method according to claim 29, wherein R⁴ is selected from hydrogen, alkyl having 1 to 20 carbon atoms, and heteroalkyl having 1 to 20 carbon atoms, wherein the heteroalkyl contains one or more heteroatoms such as nitrogen, oxygen, or sulphur; R⁵ is selected from alkyl having 1 to 20 carbon atoms, and heteroalkyl having 1 to 20 carbon atoms, wherein the heteroalkyl contains one or more heteroatoms such as nitrogen, oxygen, or sulphur.

32. The preparation method according to claim 26, wherein
when L₁ is 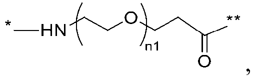 the method further comprises:
preparing the compound of formula (13) using a compound of formula (15) and a compound of formula (18) as starting materials,

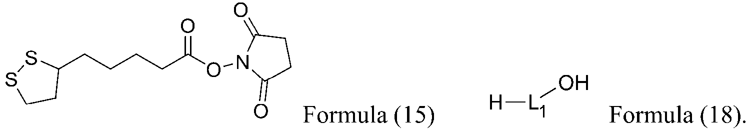

33. The preparation method according to claim 28 or 32, wherein the method further comprises preparing the compound of formula (15) using a lipoic acid of formula (17) and N-hydroxysuccinimide as starting materials:

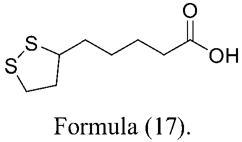

34. A preparation method for the vesicle compound according to any one of claims 10 to 18, **characterized in that** the preparation method comprises preparing the vesicle compound using the lipoic acid derivative according to any one of claims 1 to 9 or the lipoic acid derivative prepared by the method according to any one of claims 25 to 33 as a starting material.

35. The preparation method according to claim 34, **characterized in that** the lipoic acid derivative reacts under a condition of pH not greater than 7.2 to form the vesicle compound; preferably, the lipoic acid derivative reacts under a condition of pH 5 to 7.2 to form the vesicle compound.

36. The preparation method according to claim 35, **characterized in that** the method further comprises, after the reaction to form the vesicle compound, repeating the steps of adding a base to the reaction system for reaction and then adding an acid for reaction; wherein when adding the base, a pH value of the reaction system is adjusted to be not less than 8; when adding the acid, a pH value of the reaction system is adjusted to be not greater than 7.2.

37. The preparation method according to any one of claims 35 to 36, **characterized in that** a concentration of the lipoic acid derivative in the reaction system is greater than a critical aggregation concentration, wherein the critical aggregation concentration is 230 µM to 300 µM.
